# EUROPEAN PATENT SPECIFICATION

(11) **EP 4 162 034 B1**
(45) Date of publication and mention of the grant of the patent: **29.07.2026**
(21) Application number: 21729331.5
(22) Date of filing: 01.06.2021
(51) Int. Cl.: C12N 9/12, C12N 15/86, A61K 39/12

(54) **RNA REPLICON FOR VERSATILE AND EFFICIENT GENE EXPRESSION**
RNA-REPLIKON FÜR VIELSEITIGE UND EFFIZIENTE GENEXPRESSION
RÉPLICON D'ARN POUR OBTENIR UNE EXPRESSION GÉNIQUE VERSATILE ET EFFICACE

(30) Priority: 04.06.2020 WO PCT/EP2020/065491
(43) Date of publication of application: 12.04.2023
(73) Proprietor: BioNTech SE, 55131 Mainz (DE); TRON - Translationale Onkologie an der Universitätsmedizin der Johannes Gutenberg- Universität Mainz gemeinnützige GmbH, 55131 Mainz (DE)
(72) Inventor: PERKOVIC, Mario, 55131 Mainz (DE); WITZEL, Sonja, 55131 Mainz (DE); BEISSERT, Tim, 55131 Mainz (DE); SAHIN, Ugur, 55131 Mainz (DE)
(74) Representative: Schnappauf, Georg
(86) International application number: PCT/EP2021/064690
(87) International publication number: WO 2021/245090

(56) References cited:
- WO-A1-2017/162266
- WANG QING S. ET AL: "Switch from Cap- to Factorless IRES-Dependent 0 and +1 Frame Translation during Cellular Stress and Dicistrovirus Infection", PLOS ONE, vol. 9, no. 8, 4 August 2014 (2014-08-04), pages e103601, XP055847886, Retrieved from the Internet <URL:https://storage.googleapis.com/plos-corpus-prod/10.1371/journal.pone.0103601/1/pone.0103601.pdf?X-Goog-Algorithm=GOOG4-RSA-SHA256&X-Goog-Credential=wombat-sa@plos-prod.iam.gserviceaccount.com/20211005/auto/storage/goog4_request&X-Goog-Date=20211005T132848Z&X-Goog-Expires=86400&X-Goog-SignedHeaders=h> DOI: 10.1371/journal.pone.0103601
- ANNETTE B. VOGEL ET AL: "Self-Amplifying RNA Vaccines Give Equivalent Protection against Influenza to mRNA Vaccines but at Much Lower Doses", MOLECULAR THERAPY, vol. 26, no. 2, 1 February 2018 (2018-02-01), US, pages 446 - 455, XP055643534, ISSN: 1525-0016, DOI: 10.1016/j.ymthe.2017.11.017
- BEISSERT TIM ET AL: "A Trans-amplifying RNA Vaccine Strategy for Induction of Potent Protective Immunity", MOLECULAR THERAPY, vol. 28, no. 1, 1 January 2020 (2020-01-01), US, pages 119 - 128, XP055847688, ISSN: 1525-0016, Retrieved from the Internet <URL:https://www.ncbi.nlm.nih.gov/pmc/articles/PMC6953774/pdf/main.pdf> DOI: 10.1016/j.ymthe.2019.09.009
- HAE LI KO ET AL: "Development of an RNA Expression Platform Controlled by Viral Internal Ribosome Entry Sites", JOURNAL OF MICROBIOLOGY AND BIOTECHNOLOGY, vol. 29, no. 1, 28 November 2018 (2018-11-28), pages 127 - 140, XP055649150, ISSN: 1017-7825, DOI: 10.4014/jmb.1811.11019
- THOMPSON SUNNIE R.: "Tricks an IRES uses to enslave ribosomes", TRENDS IN MICROBIOLOGY, vol. 20, no. 11, 1 November 2012 (2012-11-01), GB, pages 558 - 566, XP055847892, ISSN: 0966-842X, DOI: 10.1016/j.tim.2012.08.002

## Description

### Technical Field

The present teaching embraces an RNA replicon (self-amplifying RNA vector (saRNA)) that can be replicated by a replicase of a self-replicating virus, e.g., a replicase of alphavirus origin. According to the teaching, translation of the replicase open reading frame is uncoupled from a 5'-terminal cap by placing translation of the replicase open reading frame under the translational control of an internal ribosome entry site (IRES). Thereby the initiation of translation depends on the molecular properties of the respective IRES, which compared to cap-dependent translation may require less or no cellular initiation factors to direct the ribosome to the translational start site. According to the teaching, IRES-controlled replicase translation may allow the use of uncapped synthetic saRNA. Furthermore, the use of an IRES provides for the option to insert additional transgenes upstream to the IRES.

### Background

Nucleic acid molecules comprising foreign genetic information encoding one or more polypeptides for prophylactic and therapeutic purposes have been studied in biomedical research for many years. Prior art approaches share the delivery of a nucleic acid molecule to a target cell or organism, but differ in the type of nucleic acid molecule and/or delivery system: influenced by safety concerns associated with the use of deoxyribonucleic acid (DNA) molecules, ribonucleic acid (RNA) molecules have received growing attention in recent years. Various approaches have been proposed, including administration of single stranded or double-stranded RNA, in the form of naked RNA, or in complexed or packaged form, e.g. in non-viral or viral delivery vehicles. In viruses and in viral delivery vehicles, the genetic information is typically encapsulated by proteins and/or lipids (virus particle). For example, engineered RNA virus particles derived from RNA viruses have been proposed as delivery vehicle for treating plants (WO 2000/053780 A2) or for vaccination of mammals (Tubulekas et al., 1997, Gene, vol. 190, pp. 191-195). In general, RNA viruses are a diverse group of infectious particles with an RNA genome. RNA viruses can be sub-grouped into single-stranded RNA (ssRNA) and double-stranded RNA (dsRNA) viruses, and the ssRNA viruses can be further generally divided into positive-stranded [(+) stranded] and/or negative-stranded [(-) stranded] viruses or retroviruses which reverse transcribe their RNA genome into DNA during their replications cycle. Positive-stranded RNA viruses are *prima facie* attractive as a delivery system in biomedicine because their RNA may serve directly as template for translation in the host cell.

Alphaviruses are typical representatives of positive-stranded RNA viruses. The hosts of alphaviruses include a wide range of organisms, comprising insects, fish and mammals, such as domesticated animals and humans. Alphaviruses replicate in the cytoplasm of infected cells (for review of the alphaviral life cycle see José et al., Future Microbiol., 2009, vol. 4, pp. 837-856). The total genome length of many alphaviruses typically ranges between 11,000 and 12,000 nucleotides, and the genomic RNA typically has a 5'-cap, and a 3' poly(A) tail. The genome of alphaviruses encodes non-structural proteins (involved in transcription, modification and replication of viral RNA and in protein modification) and structural proteins (forming the virus particle). There are typically two open reading frames (ORFs) in the genome. The four non-structural proteins (nsP1-nsP4) are typically encoded together by a first ORF beginning near the 5' terminus of the genome, while alphavirus structural proteins are encoded together by a second ORF which is found downstream of the first ORF and extends near the 3' terminus of the genome. Typically, the first ORF is larger than the second ORF, the ratio being roughly 2:1.

In cells infected by an alphavirus, only the non-structural proteins are translated from the genomic RNA, while the structural proteins are translatable from a subgenomic transcript, which is an RNA molecule that resembles eukaryotic messenger RNA (mRNA; Gould et al., 2010, Antiviral Res., vol. 87 pp. 111-124). Following infection, i.e. at early stages of the viral life cycle, the (+) stranded genomic RNA directly acts like a messenger RNA for the translation of the open reading frame encoding the non-structural poly-protein (nsP1234). In some alphaviruses, there is an opal stop codon between the coding sequences of nsP3 and nsP4: polyprotein P123, containing nsP1, nsP2, and nsP3, is produced when translation terminates at the opal stop codon, and polyprotein P1234, containing in addition nsP4, is produced upon readthrough of this opal codon (Strauss & Strauss, Microbiol. Rev., 1994, vol. 58, pp. 491-562; Rupp et al., 2015, J. Gen. Virology, vol. 96, pp. 2483-2500). nsP1234 is autoproteolytically cleaved into the fragments nsP123 and nsP4. The polypeptides nsP123 and nsP4 associate to form the (-) strand replicase complex that transcribes (-) stranded RNA, using the (+) stranded genomic RNA as template. Typically at later stages, the nsP123 fragment is completely cleaved into individual proteins nsP1, nsP2 and nsP3 (Shirako & Strauss, 1994, J. Virol., vol. 68, pp. 1874-1885). All four proteins assemble to form the (+) strand replicase complex that synthesizes new (+) stranded genomes, using the (-) stranded complement of genomic RNA as template (Kim et al., 2004, Virology, vol. 323, pp. 153-163, Vasiljeva et al., 2003, J. Biol. Chem. vol. 278, pp. 41636-41645).

In infected cells, subgenomic RNA as well as new genomic RNA is provided with a 5'-cap by nsP1 (Pettersson et al. 1980, Eur. J. Biochem. 105, 435-443; Rozanov et al., 1992, J. Gen. Virology, vol. 73, pp. 2129-2134), and provided with a poly-adenylate [poly(A)] tail by nsP4 (Rubach et al., Virology, 2009, vol. 384, pp. 201-208). Thus, both subgenomic RNA and genomic RNA resemble messenger RNA (mRNA).

Alphavirus structural proteins (core nucleocapsid protein C, envelope protein E2 and envelope protein E1, all constituents of the virus particle) are typically encoded by one single open reading frame under control of a subgenomic promoter (Strauss & Strauss, Microbiol. Rev., 1994, vol. 58, pp. 491-562). The subgenomic promoter is recognized by alphaviral non-structural proteins acting in *cis.* In particular, alphavirus replicase synthesizes a (+) stranded subgenomic transcript using the (-) stranded complement of genomic RNA as template. The (+) stranded subgenomic transcript encodes the alphavirus structural proteins (Kim et al., 2004, Virology, vol. 323, pp. 153-163, Vasiljeva et al., 2003, J. Biol. Chem. vol. 278, pp. 41636-41645). The subgenomic RNA transcript serves as template for translation of the open reading frame encoding the structural proteins as one poly-protein, and the poly-protein is cleaved to yield the structural proteins. At a late stage of alphavirus infection in a host cell, a packaging signal which is located within the coding sequence of nsP2 ensures selective packaging of genomic RNA into budding virions, packaged by structural proteins (White et al., 1998, J. Virol., vol. 72, pp. 4320-4326).

In infected cells, (-) strand RNA synthesis is typically observed only in the first 3-4 h post infection, and is undetectable at late stages, at which time the synthesis of only (+) strand RNA (both genomic and subgenomic) is observed. According to Frolov et al., 2001, RNA, vol. 7, pp. 1638-1651, the prevailing model for regulation of RNA synthesis suggests a dependence on the processing of the non-structural poly-protein: initial cleavage of the non-structural polyprotein nsP1234 yields nsP123 and nsP4; nsP4 acts as RNA-dependent RNA polymerase (RdRp) that is active for (-) strand synthesis, but inefficient for the generation of (+) strand RNAs. Further processing of the polyprotein nsP123, including cleavage at the nsP2/nsP3 junction, changes the template specificity of the replicase to increase synthesis of (+) strand RNA and to decrease or terminate synthesis of (-) strand RNA.

The synthesis of alphaviral RNA is also regulated by cis-acting RNA elements, including four conserved sequence elements (CSEs; Strauss & Strauss, Microbiol. Rev., 1994, vol. 58, pp. 491-562; and Frolov, 2001, RNA, vol. 7, pp. 1638-1651).

The alphavirus genome comprises four conserved sequence elements (CSEs) which are understood to be important for viral RNA replication in the host cell (Strauss & Strauss, Microbiol. Rev., 1994, vol. 58, pp. 491-562). CSE 1, found at or near the 5' end of the virus genome, is believed to function as a promoter for (+) strand synthesis from (-) strand templates. CSE 2, located downstream of CSE1 but still close to the 5' end of the genome within the coding sequence for nsP1 is thought to act as a promoter for initiation of (-) strand synthesis from a genomic RNA template (note that the subgenomic RNA transcript, which does not comprise CSE 2, does not function as a template for (-) strand synthesis). CSE 3 is located in the junction region between the coding sequence for the non-structural and structural proteins and acts as core promoter for the efficient transcription of the subgenomic transcript. Finally, CSE 4, which is located just upstream of the poly(A) sequence in the 3' untranslated region of the alphavirus genome, is understood to function as a core promoter for initiation of (-) strand synthesis (José et al., Future Microbiol., 2009, vol. 4, pp. 837-856). CSE 4 and the poly(A) tail of the alphavirus are understood to function together for efficient (-) strand synthesis (Hardy & Rice, J. Virol., 2005, vol. 79, pp. 4630-4639). In addition to alphavirus proteins, also host cell factors, presumably proteins, may bind to conserved sequence elements (Strauss & Strauss, Microbiol. Rev., 1994, vol. 58, pp. 491-562). The 5' replication recognition sequence of the alphavirus genome is not only involved in translation initiation, but also comprises two conserved sequence elements involved in synthesis of viral RNA, CSE 1 and CSE 2. For the function of CSE 1 and 2, the secondary structure is believed to be more important than the linear sequence (Strauss & Strauss, Microbiol. Rev., 1994, vol. 58, pp. 491-562).

Alphavirus-derived vectors have been proposed for delivery of foreign genetic information into target cells or target organisms. In simple approaches, the open reading frame encoding alphaviral structural proteins is replaced by an open reading frame encoding a protein of interest. Alphavirus-based trans-replication systems rely on alphavirus nucleotide sequence elements on two separate nucleic acid molecules: one nucleic acid molecule encodes a viral replicase (typically as poly-protein nsP1234), and the other nucleic acid molecule is capable of being replicated by said replicase *in trans* (hence the designation trans-replication system). trans-replication requires the presence of both these nucleic acid molecules in a given host cell. The nucleic acid molecule capable of being replicated by the replicase *in trans* must comprise certain alphaviral sequence elements to allow recognition and RNA synthesis by the alphaviral replicase.

Self-amplifying RNA (saRNA), also called replicon RNA, such as alphavirus-derived RNA vectors allow efficient expression of a gene of interest under control of a subgenomic promotor. However, versatility of such vectors is limited because the initially *in vitro* transcribed synthetic saRNA requires a cap. This increases costs and expenditure to provide such vectors. Furthermore, protein expression (replicase and gene of interest (GOI)) using conventional saRNA is cap-controlled and requires all cellular translation initiation factors that are enabling, regulating or restricting canonical translation.

The present teaching removes the constraints normally imposed by cap-dependent translation of the replicase by using an internal ribosomal entry site (IRES) to direct translation of the replicase. An additional GOI may be inserted upstream of the IRES and placed under the control of a 5'-terminal cap. Such GOI may be fused in frame to nsP1 sequences overlapping with the 5' replication recognition sequence (RRS) and translated from the original nsP1 start codon, or the AUG-codons are removed within nsP1 of the RRS such that the GOI is translated from its own AUG.

### Summary

The present invention is defined by the appended claims. The present disclosure provides teachings which in some respects go beyond the disclosure of the invention as such, which is defined exclusively by the appended claims. The teachings are provided to place the actual invention in a broader technical context and to illustrate possible related technical developments. Such additional technical information which does not fall within the scope of the appended claims is not part of the invention. In particular, the term "embodiment" is not to be construed as necessarily referring to an embodiment of the invention, unless the "embodiment" in question falls within the scope of the claims.

In one aspect, the present teaching provides an RNA replicon comprising an internal ribosome entry site (IRES) and an open reading frame encoding a functional non-structural protein from a self-replicating virus, wherein the IRES controls expression of the functional non-structural protein.

In one embodiment, the open reading frame encoding a functional non-structural protein from a self-replicating virus is located downstream from the IRES.

In one embodiment, the IRES is insensitive to cellular stress. In one embodiment, the IRES is insensitive to interferons, preferably type I interferons such as interferon-alpha and/or interferon-beta. In one embodiment, the IRES is a cellular or viral IRES, preferably a viral IRES. In one embodiment, the IRES is derived from viruses selected from the group consisting of picornaviruses, flaviviruses or dicistroviruses. In one embodiment, the IRES is derived from a picornavirus or a dicistrovirus, preferably a dicistrovirus. In one embodiment, the IRES is a type IV IRES. In one embodiment, expression controlled by the IRES is independent of IRES trans-acting factors. In one embodiment, expression controlled by the IRES is independent of cellular translation initiation factors. In one embodiment, expression controlled by the IRES is independent of phosphorylation of eukaryotic initiation factor 2 (eIF2).

In one embodiment, the RNA replicon does not comprise a 5' cap.

In one embodiment, the RNA replicon comprises a 5' replication recognition sequence. In one embodiment, the functional non-structural protein is heterologous to the 5' replication recognition sequence. In one embodiment, the 5' replication recognition sequence comprises a sequence homologous to an open reading frame of a non-structural protein or a fragment thereof from a self-replicating virus. In one embodiment, the 5' replication recognition sequence of the RNA replicon comprises sequences homologous to conserved sequence element 1 (CSE 1) and conserved sequence element 2 (CSE 2) of an alphavirus. In one embodiment, the the 5' replication recognition sequence comprises a sequence homologous to an open reading frame of a non-structural protein or a fragment thereof from a self-replicating virus, wherein the sequence homologous to an open reading frame of a non-structural protein or a fragment thereof from a self-replicating virus is characterized in that it comprises the removal of at least one initiation codon compared to the native viral sequence. In one embodiment, the sequence homologous to an open reading frame of a non-structural protein or a fragment thereof from a self-replicating virus is characterized in that it comprises the removal of at least the native start codon of the open reading frame of a non-structural protein from a self-replicating virus. In one embodiment, the sequence homologous to an open reading frame of a non-structural protein or a fragment thereof from a self-replicating virus is characterized in that it comprises the removal of at least one initiation codon other than the native start codon of the open reading frame of a non-structural protein from a self-replicating virus. In one embodiment, the sequence homologous to an open reading frame of a non-structural protein or a fragment thereof from a self-replicating virus is characterized in that it is free of initiation codons. In one embodiment, the 5' replication recognition sequence comprises one or more stem loops providing functionality of the 5' replication recognition sequence with respect to RNA replication. In one embodiment, one or more stem loops of the 5' replication recognition sequence are deleted or disrupted which deletion or disruption, however, does not affect functionality of the 5' replication recognition sequence. In a preferred embodiment, one or more stem loops of the 5' replication recognition sequence are not deleted or disrupted. More preferably, none of the stem loops of the 5' replication recognition sequence is deleted or disrupted. In one embodiment, the RNA replicon comprises at least one nucleotide change compensating for nucleotide pairing disruptions within at least one stem loop introduced by the removal of at least one initiation codon. In one embodiment, the RNA replicon does not comprise an open reading frame encoding a truncated non-structural protein from a self-replicating virus, in particular a fragment of nsP1. In one embodiment, the open reading frame encoding a functional non-structural protein from a self-replicating virus does not overlap with the 5' replication recognition sequence.

In one embodiment, the RNA replicon comprises an open reading frame encoding a protein of interest downstream from the 5' replication recognition sequence and upstream from the IRES. In one embodiment, the open reading frame encoding a protein of interest is not under control of a subgenomic promotor. In one embodiment, the open reading frame encoding a protein of interest is under control of a subgenomic promotor. In one embodiment, the open reading frame encoding a protein of interest is a transgene which is preferably not derived from a self-replicating virus. In one embodiment, the 5' replication recognition sequence and the open reading frame encoding a protein of interest do not overlap and preferably the 5' replication recognition sequence does not overlap with any open reading frame of the RNA replicon, e.g. the 5' replication recognition sequence does not contain a functional initiation codon and preferably does not contain any initiation codon. In one embodiment, the initiation codon of the open reading frame encoding a protein of interest is in the 5' → 3' direction of the RNA replicon the first functional initiation codon, preferably the first initiation codon. In one embodiment, the protein of interest can be expressed from the RNA replicon as a template. In one embodiment, the protein of interest can additionally be expressed from subgenomic RNA. In one embodiment, the protein of interest is expressed as a fusion protein with a protein or peptide encoded by the sequence homologous to an open reading frame of a non-structural protein or a fragment thereof from a self-replicating virus and expression of the fusion protein is initiated at an initiation codon of the sequence homologous to an open reading frame of a non-structural protein or a fragment thereof from a self-replicating virus. In one embodiment, the initiation codon of the sequence homologous to an open reading frame of a non-structural protein or a fragment thereof from a self-replicating virus is the native start codon of the open reading frame of a non-structural protein from a self-replicating virus. In one embodiment, expression of the protein of interest is initiated at an initiation codon of the open reading frame encoding a protein of interest.

In one embodiment, the RNA replicon comprises a subgenomic promotor controlling production of subgenomic RNA comprising an open reading frame encoding a protein of interest. In one embodiment, the subgenomic RNA is a transcription product of an RNA-dependent RNA polymerase derived from the functional non-structural protein from a self-replicating virus. In one embodiment, the protein of interest can be expressed from the subgenomic RNA as a template. In one embodiment, the RNA replicon comprises the open reading frame encoding a protein of interest controlled by the subgenomic promotor downstream from the open reading frame encoding a functional non-structural protein from a self-replicating virus. In one embodiment, the subgenomic promotor overlaps with the open reading frame encoding a functional non-structural protein from a self-replicating virus. In one embodiment, the subgenomic promotor does not overlap with the open reading frame encoding a functional non-structural protein from a self-replicating virus.

In one embodiment, the RNA replicon comprises a 3' replication recognition sequence. In one embodiment, the open reading frame encoding a functional non-structural protein from a self-replicating virus, the 5' and/or 3' replication recognition sequences and the subgenomic promotor are derived from a self-replicating virus, preferably the same self-replicating virus species.

In one embodiment, the RNA replicon can be replicated by an RNA-dependent RNA polymerase derived from the functional non-structural protein from a self-replicating virus.

In one embodiment, the self-replicating virus is an alphavirus.

In one embodiment, the alphavirus is selected from the group consisting of Venezuelan equine encephalitis complex viruses, Eastern equine encephalitis complex viruses, Western equine encephalitis complex viruses, Semliki Forest virus complex viruses, Barmah Forest virus, Middelburg virus and Ndumu virus. In one embodiment, the alphavirus is a Venezuelan equine encephalitis virus or Semliki Forest virus.

In a further aspect, the present teaching provides a non-viral particle comprising the RNA replicon described herein.

In a further aspect, the present teaching provides a DNA comprising a nucleic acid sequence encoding the RNA replicon described herein.

In a further aspect, the present teaching provides a composition comprising the RNA replicon described herein and a pharmaceutically acceptable carrier.

In a further aspect, the present teaching provides a composition described herein for use in therapy.

In a further aspect, the present teaching provides a method for producing a protein of interest in a cell comprising the steps of:
(a) obtaining the RNA replicon described herein, which comprises an open reading frame encoding the protein of interest, and
(b) inoculating the RNA replicon into the cell.

In a further aspect, the present teaching provides a method for producing a protein of interest in a subject comprising the steps of:
(a) obtaining the RNA replicon described herein, which comprises an open reading frame encoding the protein of interest, and
(b) administering the RNA replicon to the subject.

In one embodiment, the protein of interest is an antigenic peptide or protein.

In one embodiment, the method is a method for eliciting an immune response in the subject.

In a further aspect, the teaching provides a cell containing the replicon described herein. In one embodiment, the cell is inoculated according to the method described herein. In one embodiment, the cell is obtainable by the method described herein. In one embodiment, the cell is part of an organism.

### Brief description of the drawings

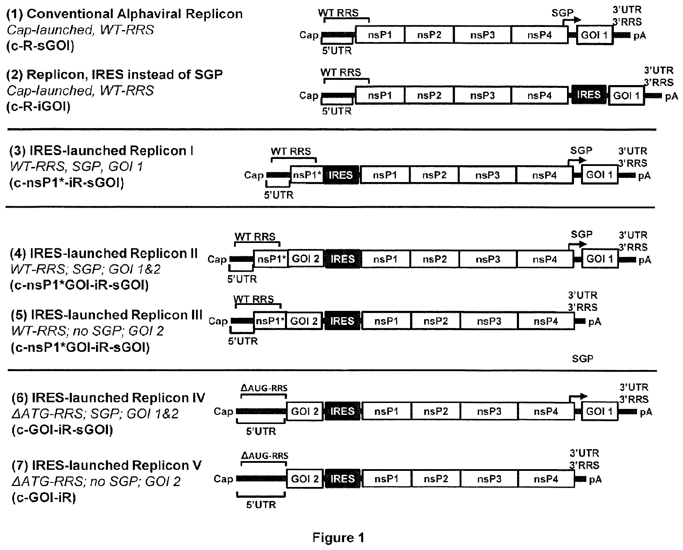

**Figure 1****: RNA constructs.** (1) In conventional alphaviral replicons, the non-structural proteins 1 to 4 (nsP1-4; =replicase enzyme complex) are translated in a cap-dependent manner from the 5'-end of the RNA. The subgenomic transcript that starts within the subgenomic promoter (SGP) and encodes the first gene of interest (GOI 1) is also capped. (2) When an internal ribosome entry site (IRES) substitutes the SGP, the GOI 1 is no longer encoded by a capped subgenomic transcript. Instead, it is encoded by the genomic RNA, and its translation initiates internally (downstream of the nsP1-4) independently from a cap. (3) An IRES upstream of nsP1-4 uncouples the translation of the nsP1-4 from the 5'cap. To conserve the replicative potential of the RNA it is required to keep the unaltered (wildtype; wt) 5' replication-recognition sequence (RRS) which overlaps with the 5'untranslated region (UTR) and the N-terminus of nsP1 (nsP1*). In this IRES-launched replicon the translation of nsP1-4 depends upon the characteristics of the selected IRES, while expression of GOI 1 remains under the control of the SGP. (4) A second GOI (GOI 2) can be fused in frame to the N-terminal part of nsP1* overlapping to the 5'RRS. (5) In a further variant, the SGP and the GOI 1 downstream of it may be removed. (6) To avoid the translation of the N-terminal nsP1* the original start-codon and further putative AUG-codon can be mutated (ΔAUG), thereby enabling cap-dependent translation to start at the AUG of GOI 2. The nsP1-4 remain downstream to the IRES, and GOI 1 downstream to the SGP. (7) A simplified construct having a 5'RRS lacking AUG, and lacking the SGP and GOI 1.
**Figure 2****: IRES-mediated expression is approximately 10 times weaker than cap-dependent translation.**
**Figure 3****: IRES-mediated replicase expression enables RNA replication.**
**Figure 4****: IRES-launched replicon show robust expression of both open reading frames.**
**Figure 5****: Factor independent Dicistrovirus intergenic region compared to EMCV IRES.**
**Figure 6****: IRES-launched replicons derived from the Semliki Forest Virus are replicating with and without cap.**
**Figure 7****: The enterovirus 71 IRES and hepatitis C virus IRES controlled replicase expression enables replication**
**Figure 8****: IRES-launched replicons derived from the VEEV vaccine strain TC-83 replicate and express more efficiently as WT VEEV.**
**Figure 9****: Uncapped conventioal replicon of WT and TC-83 VEEV strains replicate poorly.**
**Figure 10****: Interferon inhibitors expressed upstream of the IRES enhance the expression of IRES-launched replicons**

### Detailed description

Although the present teaching is described in detail below, it is to be understood that this teaching is not limited to the particular methodologies, protocols and reagents described herein as these may vary. It is also to be understood that the terminology used herein is for the purpose of describing particular embodiments only, and is not intended to limit the scope of the present teaching which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present teaching will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

In the following, the elements of the present teaching will be described. These elements are listed with specific embodiments, however, it should be understood that they may be combined in any manner and in any number to create additional embodiments. The variously described examples and preferred embodiments should not be construed to limit the present teaching to only the explicitly described embodiments. This description should be understood to disclose and encompass embodiments which combine the explicitly described embodiments with any number of the disclosed and/or preferred elements. Furthermore, any permutations and combinations of all described elements in this application should be considered disclosed by this description unless the context indicates otherwise.

The term "about" means approximately or nearly, and in the context of a numerical value or range set forth herein preferably means +/- 10 % of the numerical value or range recited or claimed.

The terms "a" and "an" and "the" and similar reference used in the context of describing the teaching (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the teaching and does not pose a limitation on the scope of the teaching otherwise claimed. No language in the specification should be construed as indicating any non-claimed element essential to the practice of the teaching.

Unless expressly specified otherwise, the term "comprising" is used in the context of the present document to indicate that further members may optionally be present in addition to the members of the list introduced by "comprising". It is, however, contemplated as a specific embodiment of the present teaching that the term "comprising" encompasses the possibility of no further members being present, i.e. for the purpose of this embodiment "comprising" is to be understood as having the meaning of "consisting of".

Indications of relative amounts of a component characterized by a generic term are meant to refer to the total amount of all specific variants or members covered by said generic term. If a certain component defined by a generic term is specified to be present in a certain relative amount, and if this component is further characterized to be a specific variant or member covered by the generic term, it is meant that no other variants or members covered by the generic term are additionally present such that the total relative amount of components covered by the generic term exceeds the specified relative amount; more preferably no other variants or members covered by the generic term are present at all.

Several documents are cited throughout the text of this specification. Nothing herein is to be construed as an admission that the present teaching was not entitled to antedate such disclosure.

Terms such as "reduce" or "inhibit" as used herein means the ability to cause an overall decrease, preferably of 5% or greater, 10% or greater, 20% or greater, more preferably of 50% or greater, and most preferably 75% or greater, in the level. The term "inhibit" or similar phrases includes a complete or essentially complete inhibition, i.e. a reduction to zero or essentially to zero.

Terms such as "increase" or "enhance" preferably relate to an increase or enhancement by about at least 10%, preferably at least 20%, preferably at least 30%, more preferably at least 40%, more preferably at least 50%, even more preferably at least 80%, and most preferably at least 100%.

The term "net charge" refers to the charge on a whole object, such as a compound or particle.

An ion having an overall net positive charge is a cation, while an ion having an overall net negative charge is an anion. Thus, according to the teaching, an anion is an ion with more electrons than protons, giving it a net negative charge; and a cation is an ion with fewer electrons than protons, giving it a net positive charge.

Terms as "charged", "net charge", "negatively charged" or "positively charged", with reference to a given compound or particle, refer to the electric net charge of the given compound or particle when dissolved or suspended in water at pH 7.0.

The term "nucleic acid" according to the teaching also comprises a chemical derivatization of a nucleic acid on a nucleotide base, on the sugar or on the phosphate, and nucleic acids containing non-natural nucleotides and nucleotide analogs. In some embodiments, the nucleic acid is a deoxyribonucleic acid (DNA) or a ribonucleic acid (RNA). In general, a nucleic acid molecule or a nucleic acid sequence refers to a nucleic acid which is preferably deoxyribonucleic acid (DNA) or ribonucleic acid (RNA). According to the teaching, nucleic acids comprise genomic DNA, cDNA, mRNA, viral RNA, recombinantly prepared and chemically synthesized molecules. According to the teaching, a nucleic acid may be in the form of a single-stranded or double-stranded and linear or covalently closed circular molecule.

According to the teaching "nucleic acid sequence" refers to the sequence of nucleotides in a nucleic acid, e.g. a ribonucleic acid (RNA) or a deoxyribonucleic acid (DNA). The term may refer to an entire nucleic acid molecule (such as to the single strand of an entire nucleic acid molecule) or to a part (e.g. a fragment) thereof.

According to the present teaching, the term "RNA" or "RNA molecule" relates to a molecule which comprises ribonucleotide residues and which is preferably entirely or substantially composed of ribonucleotide residues. The term "ribonucleotide" relates to a nucleotide with a hydroxyl group at the 2'-position of a β-D-ribofuranosyl group. The term "RNA" comprises double-stranded RNA, single stranded RNA, isolated RNA such as partially or completely purified RNA, essentially pure RNA, synthetic RNA, and recombinantly generated RNA such as modified RNA which differs from naturally occurring RNA by addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs, particularly analogs of naturally occurring RNAs.

According to the teaching, RNA may be single-stranded or double-stranded. In some embodiments of the present teaching, single-stranded RNA is preferred. The term "single-stranded RNA" generally refers to an RNA molecule to which no complementary nucleic acid molecule (typically no complementary RNA molecule) is associated. Single-stranded RNA may contain self-complementary sequences that allow parts of the RNA to fold back and to form secondary structure motifs including without limitation base pairs, stems, stem loops and bulges. Single-stranded RNA can exist as minus strand [(-) strand] or as plus strand [(+) strand]. The (+) strand is the strand that comprises or encodes genetic information. The genetic information may be for example a polynucleotide sequence encoding a protein. When the (+) strand RNA encodes a protein, the (+) strand may serve directly as template for translation (protein synthesis). The (-) strand is the complement of the (+) strand. In the case of double-stranded RNA, (+) strand and (-) strand are two separate RNA molecules, and both these RNA molecules associate with each other to form a double-stranded RNA ("duplex RNA").

The term "stability" of RNA relates to the "half-life" of RNA. "Half-life" relates to the period of time which is needed to eliminate half of the activity, amount, or number of molecules. In the context of the present teaching, the half-life of an RNA is indicative for the stability of said RNA. The half-life of RNA may influence the "duration of expression" of the RNA. It can be expected that RNA having a long half-life will be expressed for an extended time period.

The term "translation efficiency" relates to the amount of translation product provided by an RNA molecule within a particular period of time.

"Fragment", with reference to a nucleic acid sequence, relates to a part of a nucleic acid sequence, i.e. a sequence which represents the nucleic acid sequence shortened at the 5'- and/or 3'-end(s). Preferably, a fragment of a nucleic acid sequence comprises at least 80%, preferably at least 90%, 95%, 96%, 97%, 98%, or 99% of the nucleotide residues from said nucleic acid sequence. In the present teaching those fragments of RNA molecules are preferred which retain RNA stability and/or translational efficiency.

"Fragment", with reference to an amino acid sequence (peptide or protein), relates to a part of an amino acid sequence, i.e. a sequence which represents the amino acid sequence shortened at the N-terminus and/or C-terminus. A fragment shortened at the C-terminus (N-terminal fragment) is obtainable e.g. by translation of a truncated open reading frame that lacks the 3'-end of the open reading frame. A fragment shortened at the N-terminus (C-terminal fragment) is obtainable e.g. by translation of a truncated open reading frame that lacks the 5'-end of the open reading frame, as long as the truncated open reading frame comprises a start codon that serves to initiate translation. A fragment of an amino acid sequence comprises e.g. at least 1 %, at least 2 %, at least 3 %, at least 4 %, at least 5 %, at least 10 %, at least 20 %, at least 30 %, at least 40 %, at least 50 %, at least 60 %, at least 70 %, at least 80%, at least 90% of the amino acid residues from an amino acid sequence.

The term "variant" with respect to, for example, nucleic acid and amino acid sequences, according to the teaching includes any variants, in particular mutants, viral strain variants, splice variants, conformations, isoforms, allelic variants, species variants and species homologs, in particular those which are naturally present. An allelic variant relates to an alteration in the normal sequence of a gene, the significance of which is often unclear. Complete gene sequencing often identifies numerous allelic variants for a given gene. With respect to nucleic acid molecules, the term "variant" includes degenerate nucleic acid sequences, wherein a degenerate nucleic acid according to the teaching is a nucleic acid that differs from a reference nucleic acid in codon sequence due to the degeneracy of the genetic code. A species homolog is a nucleic acid or amino acid sequence with a different species of origin from that of a given nucleic acid or amino acid sequence. A virus homolog is a nucleic acid or amino acid sequence with a different virus of origin from that of a given nucleic acid or amino acid sequence.

According to the teaching, nucleic acid variants include single or multiple nucleotide deletions, additions, mutations, substitutions and/or insertions in comparison with the reference nucleic acid. Deletions include removal of one or more nucleotides from the reference nucleic acid. Addition variants comprise 5'- and/or 3'-terminal fusions of one or more nucleotides, such as 1, 2, 3, 5, 10, 20, 30, 50, or more nucleotides. In the case of substitutions, at least one nucleotide in the sequence is removed and at least one other nucleotide is inserted in its place (such as transversions and transitions). Mutations include abasic sites, crosslinked sites, and chemically altered or modified bases. Insertions include the addition of at least one nucleotide into the reference nucleic acid.

According to the teaching, "nucleotide change" can refer to single or multiple nucleotide deletions, additions, mutations, substitutions and/or insertions in comparison with the reference nucleic acid. In some embodiments, a "nucleotide change" is selected from the group consisting of a deletion of a single nucleotide, the addition of a single nucleotide, the mutation of a single nucleotide, the substitution of a single nucleotide and/or the insertion of a single nucleotide, in comparison with the reference nucleic acid. According to the teaching, a nucleic acid variant can comprise one or more nucleotide changes in comparison with the reference nucleic acid.

Variants of specific nucleic acid sequences preferably have at least one functional property of said specific sequences and preferably are functionally equivalent to said specific sequences, e.g. nucleic acid sequences exhibiting properties identical or similar to those of the specific nucleic acid sequences.

As described below, some embodiments of the present teaching are characterized *inter alia* by nucleic acid sequences that are homologous to other nucleic acid sequences. These homologous sequences are variants of other nucleic acid sequences.

Preferably the degree of identity between a given nucleic acid sequence and a nucleic acid sequence which is a variant of said given nucleic acid sequence will be at least 70%, preferably at least 75%, preferably at least 80%, more preferably at least 85%, even more preferably at least 90% or most preferably at least 95%, 96%, 97%, 98% or 99%. The degree of identity is preferably given for a region of at least about 30, at least about 50, at least about 70, at least about 90, at least about 100, at least about 150, at least about 200, at least about 250, at least about 300, or at least about 400 nucleotides. In preferred embodiments, the degree of identity is given for the entire length of the reference nucleic acid sequence.

"Sequence similarity" indicates the percentage of amino acids that either are identical or that represent conservative amino acid substitutions. "Sequence identity" between two polypeptide or nucleic acid sequences indicates the percentage of amino acids or nucleotides that are identical between the sequences.

The term "% identical" is intended to refer, in particular, to a percentage of nucleotides which are identical in an optimal alignment between two sequences to be compared, with said percentage being purely statistical, and the differences between the two sequences may be randomly distributed over the entire length of the sequence and the sequence to be compared may comprise additions or deletions in comparison with the reference sequence, in order to obtain optimal alignment between two sequences. Comparisons of two sequences are usually carried out by comparing said sequences, after optimal alignment, with respect to a segment or "window of comparison", in order to identify local regions of corresponding sequences. The optimal alignment for a comparison may be carried out manually or with the aid of the local homology algorithm by Smith and Waterman, 1981, Ads App. Math. 2, 482, with the aid of the local homology algorithm by Needleman and Wunsch, 1970, J. Mol. Biol. 48, 443, and with the aid of the similarity search algorithm by Pearson and Lipman, 1988, Proc. Natl Acad. Sci. USA 85, 2444 or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.).

Percentage identity is obtained by determining the number of identical positions in which the sequences to be compared correspond, dividing this number by the number of positions compared and multiplying this result by 100.

For example, the BLAST program "BLAST 2 sequences" which is available on the website http://www.ncbi.nlm.nih.gov/blast/bl2seq/wblast2.cgi may be used.

A nucleic acid is "capable of hybridizing" or "hybridizes" to another nucleic acid if the two sequences are complementary with one another. A nucleic acid is "complementary" to another nucleic acid if the two sequences are capable of forming a stable duplex with one another. According to the teaching, hybridization is preferably carried out under conditions which allow specific hybridization between polynucleotides (stringent conditions). Stringent conditions are described, for example, in Molecular Cloning: A Laboratory Manual, J. Sambrook et al., Editors, 2nd Edition, Cold Spring Harbor Laboratory press, Cold Spring Harbor, New York, 1989 or Current Protocols in Molecular Biology, F.M. Ausubel et al., Editors, John Wiley & Sons, Inc., New York and refer, for example, to hybridization at 65°C in hybridization buffer (3.5 x SSC, 0.02% Ficoll, 0.02% polyvinylpyrrolidone, 0.02% bovine serum albumin, 2.5 mM NaH₂PO₄ (pH 7), 0.5% SDS, 2 mM EDTA). SSC is 0.15 M sodium chloride/0.15 M sodium citrate, pH 7. After hybridization, the membrane to which the DNA has been transferred is washed, for example, in 2 × SSC at room temperature and then in 0.1-0.5 × SSC/0.1 × SDS at temperatures of up to 68°C.

A percent complementarity indicates the percentage of contiguous residues in a nucleic acid molecule that can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" or "fully complementary" means that all the contiguous residues of a nucleic acid sequence will hydrogen bond with the same number of contiguous residues in a second nucleic acid sequence. Preferably, the degree of complementarity according to the teaching is at least 70%, preferably at least 75%, preferably at least 80%, more preferably at least 85%, even more preferably at least 90% or most preferably at least 95%, 96%, 97%, 98% or 99%. Most preferably, the degree of complementarity according to the teaching is 100%.

The term "derivative" comprises any chemical derivatization of a nucleic acid on a nucleotide base, on the sugar or on the phosphate. The term "derivative" also comprises nucleic acids which contain nucleotides and nucleotide analogs not occurring naturally. Preferably, a derivatization of a nucleic acid increases its stability.

According to the teaching, a "nucleic acid sequence which is derived from a nucleic acid sequence" refers to a nucleic acid which is a variant of the nucleic acid from which it is derived. Preferably, a sequence which is a variant with respect to a specific sequence, when it replaces the specific sequence in an RNA molecule retains RNA stability and/or translational efficiency.

"nt" is an abbreviation for nucleotide; or for nucleotides, preferably consecutive nucleotides in a nucleic acid molecule.

According to the teaching, the term "codon" refers to a base triplet in a coding nucleic acid that specifies which amino acid will be added next during protein synthesis at the ribosome.

The terms "transcription" and "transcribing" relate to a process during which a nucleic acid molecule with a particular nucleic acid sequence (the "nucleic acid template") is read by an RNA polymerase so that the RNA polymerase produces a single-stranded RNA molecule. During transcription, the genetic information in a nucleic acid template is transcribed. The nucleic acid template may be DNA; however, e.g. in the case of transcription from an alphaviral nucleic acid template, the template is typically RNA. Subsequently, the transcribed RNA may be translated into protein. According to the present teaching, the term "transcription" comprises *"in vitro* transcription", wherein the term *"in vitro* transcription" relates to a process wherein RNA, in particular mRNA, is *in vitro* synthesized in a cell-free system. Preferably, cloning vectors are applied for the generation of transcripts. These cloning vectors are generally designated as transcription vectors and are according to the present teaching encompassed by the term "vector". The cloning vectors are preferably plasmids. According to the present teaching, RNA preferably is *in vitro* transcribed RNA (IVT-RNA) and may be obtained by *in vitro* transcription of an appropriate DNA template. The promoter for controlling transcription can be any promoter for any RNA polymerase. A DNA template for *in vitro* transcription may be obtained by cloning of a nucleic acid, in particular cDNA, and introducing it into an appropriate vector for *in vitro* transcription. The cDNA may be obtained by reverse transcription of RNA.

The single-stranded nucleic acid molecule produced during transcription typically has a nucleic acid sequence that is the complementary sequence of the template.

According to the teaching, the terms "template" or "nucleic acid template" or "template nucleic acid" generally refer to a nucleic acid sequence that may be replicated or transcribed.

"Nucleic acid sequence transcribed from a nucleic acid sequence" and similar terms refer to a nucleic acid sequence, where appropriate as part of a complete RNA molecule, which is a transcription product of a template nucleic acid sequence. Typically, the transcribed nucleic acid sequence is a single-stranded RNA molecule.

"3' end of a nucleic acid" refers according to the teaching to that end which has a free hydroxy group. In a diagrammatic representation of double-stranded nucleic acids, in particular DNA, the 3' end is always on the right-hand side. "5' end of a nucleic acid" refers according to the teaching to that end which has a free phosphate group. In a diagrammatic representation of double-strand nucleic acids, in particular DNA, the 5' end is always on the left-hand side.

| | | |
|---|---|---|
| 5' end | 5'--P-NNNNNNN-OH-3' | 3' end |
| | 3'-HO-NNNNNNN-P--5' | |

"Upstream" describes the relative positioning of a first element of a nucleic acid molecule with respect to a second element of that nucleic acid molecule, wherein both elements are comprised in the same nucleic acid molecule, and wherein the first element is located nearer to the 5' end of the nucleic acid molecule than the second element of that nucleic acid molecule. The second element is then said to be "downstream" of the first element of that nucleic acid molecule. An element that is located "upstream" of a second element can be synonymously referred to as being located "5'" of that second element. For a double-stranded nucleic acid molecule, indications like "upstream" and "downstream" are given with respect to the (+) strand.

According to the teaching, "functional linkage" or "functionally linked" relates to a connection within a functional relationship. A nucleic acid is "functionally linked" if it is functionally related to another nucleic acid sequence. For example, a promoter is functionally linked to a coding sequence if it influences transcription of said coding sequence. Functionally linked nucleic acids are typically adjacent to one another, where appropriate separated by further nucleic acid sequences, and, in particular embodiments, are transcribed by RNA polymerase to give a single RNA molecule (common transcript).

In particular embodiments, a nucleic acid is functionally linked according to the teaching to expression control sequences which may be homologous or heterologous with respect to the nucleic acid.

The term "expression control sequence" comprises according to the teaching promoters, ribosome-binding sequences and other control elements which control transcription of a gene or translation of the derived RNA. In particular embodiments of the teaching, the expression control sequences can be regulated. The precise structure of expression control sequences may vary depending on the species or cell type but usually includes 5'-untranscribed and 5'- and 3'-untranslated sequences involved in initiating transcription and translation, respectively. More specifically, 5'-untranscribed expression control sequences include a promoter region which encompasses a promoter sequence for transcription control of the functionally linked gene. Expression control sequences may also include enhancer sequences or upstream activator sequences. An expression control sequence of a DNA molecule usually includes 5'-untranscribed and 5'- and 3'-untranslated sequences such as TATA box, capping sequence, CAAT sequence and the like. An expression control sequence of alphaviral RNA may include a subgenomic promoter and/or one or more conserved sequence element(s). A specific expression control sequence according to the present teaching is a subgenomic promoter of an alphavirus, as described herein.

The nucleic acid sequences specified herein, in particular transcribable and coding nucleic acid sequences, may be combined with any expression control sequences, in particular promoters, which may be homologous or heterologous to said nucleic acid sequences, with the term "homologous" referring to the fact that a nucleic acid sequence is also functionally linked naturally to the expression control sequence, and the term "heterologous" referring to the fact that a nucleic acid sequence is not naturally functionally linked to the expression control sequence.

A transcribable nucleic acid sequence, in particular a nucleic acid sequence coding for a peptide or protein, and an expression control sequence are "functionally" linked to one another, if they are covalently linked to one another in such a way that transcription or expression of the transcribable and in particular coding nucleic acid sequence is under the control or under the influence of the expression control sequence. If the nucleic acid sequence is to be translated into a functional peptide or protein, induction of an expression control sequence functionally linked to the coding sequence results in transcription of said coding sequence, without causing a frame shift in the coding sequence or the coding sequence being unable to be translated into the desired peptide or protein.

The term "promoter" or "promoter region" refers to a nucleic acid sequence which controls synthesis of a transcript, e.g. a transcript comprising a coding sequence, by providing a recognition and binding site for RNA polymerase. The promoter region may include further recognition or binding sites for further factors involved in regulating transcription of said gene. A promoter may control transcription of a prokaryotic or eukaryotic gene. A promoter may be "inducible" and initiate transcription in response to an inducer, or may be "constitutive" if transcription is not controlled by an inducer. An inducible promoter is expressed only to a very small extent or not at all, if an inducer is absent. In the presence of the inducer, the gene is "switched on" or the level of transcription is increased. This is usually mediated by binding of a specific transcription factor. A specific promoter according to the present teaching is a subgenomic promoter, e.g., of an alphavirus, as described herein. Other specific promoters are genomic plus-strand or negative-strand promoters, e.g., of an alphavirus.

The term "core promoter" refers to a nucleic acid sequence that is comprised by the promoter. The core promoter is typically the minimal portion of the promoter required to properly initiate transcription. The core promoter typically includes the transcription start site and a binding site for RNA polymerase.

A "polymerase" generally refers to a molecular entity capable of catalyzing the synthesis of a polymeric molecule from monomeric building blocks. A "RNA polymerase" is a molecular entity capable of catalyzing the synthesis of a RNA molecule from ribonucleotide building blocks. A "DNA polymerase" is a molecular entity capable of catalyzing the synthesis of a DNA molecule from deoxy ribonucleotide building blocks. For the case of DNA polymerases and RNA polymerases, the molecular entity is typically a protein or an assembly or complex of multiple proteins. Typically, a DNA polymerase synthesizes a DNA molecule based on a template nucleic acid, which is typically a DNA molecule. Typically, a RNA polymerase synthesizes a RNA molecule based on a template nucleic acid, which is either a DNA molecule (in that case the RNA polymerase is a DNA-dependent RNA polymerase, DdRP), or is a RNA molecule (in that case the RNA polymerase is a RNA-dependent RNA polymerase, RdRP).

A "RNA-dependent RNA polymerase" or "RdRP", is an enzyme that catalyzes the transcription of RNA from an RNA template. In the case of alphaviral RNA-dependent RNA polymerase, sequential synthesis of (-) strand complement of genomic RNA and of (+) strand genomic RNA leads to RNA replication. RNA-dependent RNA polymerase is thus synonymously referred to as "RNA replicase". In nature, RNA-dependent RNA polymerases are typically encoded by all RNA viruses except retroviruses. Typical representatives of viruses encoding a RNA-dependent RNA polymerase are alphaviruses.

According to the present teaching, "RNA replication" generally refers to an RNA molecule synthesized based on the nucleotide sequence of a given RNA molecule (template RNA molecule). The RNA molecule that is synthesized may be e.g. identical or complementary to the template RNA molecule. In general, RNA replication may occur via synthesis of a DNA intermediate, or may occur directly by RNA-dependent RNA replication mediated by a RNA-dependent RNA polymerase (RdRP). In the case of alphaviruses, RNA replication does not occur via a DNA intermediate, but is mediated by a RNA-dependent RNA polymerase (RdRP): a template RNA strand (first RNA strand) - or a part thereof - serves as template for the synthesis of a second RNA strand that is complementary to the first RNA strand or to a part thereof. The second RNA strand - or a part thereof - may in turn optionally serve as a template for synthesis of a third RNA strand that is complementary to the second RNA strand or to a part thereof. Thereby, the third RNA strand is identical to the first RNA strand or to a part thereof. Thus, RNA-dependent RNA polymerase is capable of directly synthesizing a complementary RNA strand of a template, and of indirectly synthesizing an identical RNA strand (via a complementary intermediate strand).

According to the teaching, the term "template RNA" refers to RNA that can be transcribed or replicated by an RNA-dependent RNA polymerase.

According to the teaching, the term "gene" refers to a particular nucleic acid sequence which is responsible for producing one or more cellular products and/or for achieving one or more intercellular or intracellular functions. More specifically, said term relates to a nucleic acid section (typically DNA; but RNA in the case of RNA viruses) which comprises a nucleic acid coding for a specific protein or a functional or structural RNA molecule.

An "isolated molecule" as used herein, is intended to refer to a molecule which is substantially free of other molecules such as other cellular material. The term "isolated nucleic acid" means according to the teaching that the nucleic acid has been (i) amplified *in vitro,* for example by polymerase chain reaction (PCR), (ii) recombinantly produced by cloning, (iii) purified, for example by cleavage and gel-electrophoretic fractionation, or (iv) synthesized, for example by chemical synthesis. An isolated nucleic acid is a nucleic acid available to manipulation by recombinant techniques.

The term "vector" is used here in its most general meaning and comprises any intermediate vehicles for a nucleic acid which, for example, enable said nucleic acid to be introduced into prokaryotic and/or eukaryotic host cells and, where appropriate, to be integrated into a genome. Such vectors are preferably replicated and/or expressed in the cell. Vectors comprise plasmids, phagemids, virus genomes, and fractions thereof.

The term "recombinant" in the context of the present teaching means "made through genetic engineering". Preferably, a "recombinant object" such as a recombinant cell in the context of the present teaching is not occurring naturally.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring. The term "found in nature" means "present in nature" and includes known objects as well as objects that have not yet been discovered and/or isolated from nature, but that may be discovered and/or isolated in the future from a natural source.

According to the teaching, the term "expression" is used in its most general meaning and comprises production of RNA and/or protein. It also comprises partial expression of nucleic acids. Furthermore, expression may be transient or stable. With respect to RNA, the term "expression" or "translation" relates to the process in the ribosomes of a cell by which a strand of coding RNA (e.g. messenger RNA) directs the assembly of a sequence of amino acids to make a peptide or protein.

According to the teaching, the term "mRNA" means "messenger-RNA" and relates to a transcript which is typically generated by using a DNA template and encodes a peptide or protein. Typically, mRNA comprises a 5'-UTR, a protein coding region, a 3'-UTR, and a poly(A) sequence. mRNA may be generated by *in vitro* transcription from a DNA template. The *in vitro* transcription methodology is known to the skilled person. For example, there is a variety of *in vitro* transcription kits commercially available. According to the teaching, mRNA may be modified by stabilizing modifications and capping.

According to the teaching, the terms "poly(A) sequence" or "poly(A) tail" refer to an uninterrupted or interrupted sequence of adenylate residues which is typically located at the 3' end of an RNA molecule. An uninterrupted sequence is characterized by consecutive adenylate residues. In nature, an uninterrupted poly(A) sequence is typical. While a poly(A) sequence is normally not encoded in eukaryotic DNA, but is attached during eukaryotic transcription in the cell nucleus to the free 3' end of the RNA by a template-independent RNA polymerase after transcription, the present teaching encompasses poly(A) sequences encoded by DNA.

According to the teaching, the term "primary structure", with reference to a nucleic acid molecule, refers to the linear sequence of nucleotide monomers.

According to the teaching, the term "secondary structure", with reference to a nucleic acid molecule, refers to a two-dimensional representation of a nucleic acid molecule that reflects base pairings; e.g. in the case of a single-stranded RNA molecule particularly intramolecular base pairings. Although each RNA molecule has only a single polynucleotide chain, the molecule is typically characterized by regions of (intramolecular) base pairs. According to the teaching, the term "secondary structure" comprises structural motifs including without limitation base pairs, stems, stem loops, bulges, loops such as interior loops and multi-branch loops. The secondary structure of a nucleic acid molecule can be represented by a two-dimensional drawing (planar graph), showing base pairings (for further details on secondary structure of RNA molecules, see Auber et al., J. Graph Algorithms Appl., 2006, vol. 10, pp. 329-351). As described herein, the secondary structure of certain RNA molecules is relevant in the context of the present teaching.

According to the teaching, secondary structure of a nucleic acid molecule, particularly of a single-stranded RNA molecule, is determined by prediction using the web server for RNA secondary structure prediction (http://rna.urmc.rochester.edu/RNAstructureWeb/Servers/Predict1/Predict1.html). Preferably, according to the teaching, "secondary structure", with reference to a nucleic acid molecule, specifically refers to the secondary structure determined by said prediction. The prediction may also be performed or confirmed using MFOLD structure prediction (http://unafold.rna.albany.edu/?q=mfold).

According to the teaching, a "base pair" is a structural motif of a secondary structure wherein two nucleotide bases associate with each other through hydrogen bonds between donor and acceptor sites on the bases. The complementary bases, A:U and G:C, form stable base pairs through hydrogen bonds between donor and acceptor sites on the bases; the A:U and G:C base pairs are called Watson-Crick base pairs. A weaker base pair (called Wobble base pair) is formed by the bases G and U (G:U). The base pairs A:U and G:C are called canonical base pairs. Other base pairs like G:U (which occurs fairly often in RNA) and other rare base-pairs (e.g. A:C; U:U) are called non-canonical base pairs.

According to the teaching, "nucleotide pairing" refers to two nucleotides that associate with each other so that their bases form a base pair (canonical or non-canonical base pair, preferably canonical base pair, most preferably Watson-Crick base pair).

According to the teaching, the terms "stem loop" or "hairpin" or "hairpin loop", with reference to a nucleic acid molecule, all interchangeably refer to a particular secondary structure of a nucleic acid molecule, typically a single-stranded nucleic acid molecule, such as single-stranded RNA. The particular secondary structure represented by the stem loop consists of a consecutive nucleic acid sequence comprising a stem and a (terminal) loop, also called hairpin loop, wherein the stem is formed by two neighbored entirely or partially complementary sequence elements; which are separated by a short sequence (e.g. 3-10 nucleotides), which forms the loop of the stem-loop structure. The two neighbored entirely or partially complementary sequences may be defined as e.g. stem loop elements stem 1 and stem 2. The stem loop is formed when these two neighbored entirely or partially reverse complementary sequences, e.g. stem loop elements stem 1 and stem 2, form base-pairs with each other, leading to a double stranded nucleic acid sequence comprising an unpaired loop at its terminal ending formed by the short sequence located between stem loop elements stem 1 and stem 2. Thus, a stem loop comprises two stems (stem 1 and stem 2), which - at the level of secondary structure of the nucleic acid molecule - form base pairs with each other, and which - at the level of the primary structure of the nucleic acid molecule - are separated by a short sequence that is not part of stem 1 or stem 2. For illustration, a two-dimensional representation of the stem loop resembles a lollipop-shaped structure. The formation of a stem-loop structure requires the presence of a sequence that can fold back on itself to form a paired double strand; the paired double strand is formed by stem 1 and stem 2. The stability of paired stem loop elements is typically determined by the length, the number of nucleotides of stem 1 that are capable of forming base pairs (preferably canonical base pairs, more preferably Watson-Crick base pairs) with nucleotides of stem 2, versus the number of nucleotides of stem 1 that are not capable of forming such base pairs with nucleotides of stem 2 (mismatches or bulges). According to the present teaching, the optimal loop length is 3-10 nucleotides, more preferably 4 to 7, nucleotides, such as 4 nucleotides, 5 nucleotides, 6 nucleotides or 7 nucleotides. If a given nucleic acid sequence is characterized by a stem loop, the respective complementary nucleic acid sequence is typically also characterized by a stem loop. A stem loop is typically formed by single-stranded RNA molecules. For example, several stem loops are present in the 5' replication recognition sequence of alphaviral genomic RNA.

According to the teaching, "disruption" or "disrupt", with reference to a specific secondary structure of a nucleic acid molecule (e.g. a stem loop) means that the specific secondary structure is absent or altered. Typically, a secondary structure may be disrupted as a consequence of a change of at least one nucleotide that is part of the secondary structure. For example, a stem loop may be disrupted by change of one or more nucleotides that form the stem, so that nucleotide pairing is not possible.

According to the teaching, "compensates for secondary structure disruption" or "compensating for secondary structure disruption" refers to one or more nucleotide changes in a nucleic acid sequence; more typically it refers to one or more second nucleotide changes in a nucleic acid sequence, which nucleic acid sequence also comprises one or more first nucleotide changes, characterized as follows: while the one or more first nucleotide changes, in the absence of the one or more second nucleotide changes, cause a disruption of the secondary structure of the nucleic acid sequence, the co-occurrence of the one or more first nucleotide changes and the one or more second nucleotide changes does not cause the secondary structure of the nucleic acid to be disrupted. Co-occurrence means presence of both the one or more first nucleotide changes and of the one or more second nucleotide changes. Typically, the one or more first nucleotide changes and the one or more second nucleotide changes are present together in the same nucleic acid molecule. In a specific embodiment, one or more nucleotide changes that compensate for secondary structure disruption is/are one or more nucleotide changes that compensate for one or more nucleotide pairing disruptions. Thus, in one embodiment, "compensating for secondary structure disruption" means "compensating for nucleotide pairing disruptions", i.e. one or more nucleotide pairing disruptions, for example one or more nucleotide pairing disruptions within one or more stem loops. The one or more one or more nucleotide pairing disruptions may have been introduced by the removal of at least one initiation codon. Each of the one or more nucleotide changes that compensates for secondary structure disruption is a nucleotide change, which can each be independently selected from a deletion, an addition, a substitution and/or an insertion of one or more nucleotides. In an illustrative example, when the nucleotide pairing A:U has been disrupted by substitution of A to C (C and U are not typically suitable to form a nucleotide pair); then a nucleotide change that compensates for nucleotide pairing disruption may be substitution of U by G, thereby enabling formation of the C:G nucleotide pairing. The substitution of U by G thus compensates for the nucleotide pairing disruption. In an alternative example, when the nucleotide pairing A:U has been disrupted by substitution of A to C; then a nucleotide change that compensates for nucleotide pairing disruption may be substitution of C by A, thereby restoring formation of the original A:U nucleotide pairing. In general, in the present teaching, those nucleotide changes compensating for secondary structure disruption are preferred which do neither restore the original nucleic acid sequence nor create novel AUG triplets. In the above set of examples, the U to G substitution is preferred over the C to A substitution.

According to the teaching, the term "tertiary structure", with reference to a nucleic acid molecule, refers to the three dimensional structure of a nucleic acid molecule, as defined by the atomic coordinates.

According to the teaching, a nucleic acid such as RNA, e.g. mRNA, may encode a peptide or protein. Accordingly, a transcribable nucleic acid sequence or a transcript thereof may contain an open reading frame (ORF) encoding a peptide or protein.

According to the teaching, the term "nucleic acid encoding a peptide or protein" means that the nucleic acid, if present in the appropriate environment, preferably within a cell, can direct the assembly of amino acids to produce the peptide or protein during the process of translation. Preferably, coding RNA according to the teaching is able to interact with the cellular translation machinery allowing translation of the coding RNA to yield a peptide or protein.

According to the teaching, the term "peptide" comprises oligo- and polypeptides and refers to substances which comprise two or more, preferably 3 or more, preferably 4 or more, preferably 6 or more, preferably 8 or more, preferably 10 or more, preferably 13 or more, preferably 16 or more, preferably 20 or more, and up to preferably 50, preferably 100 or preferably 150, consecutive amino acids linked to one another via peptide bonds. The term "protein" refers to large peptides, preferably peptides having at least 151 amino acids, but the terms "peptide" and "protein" are used herein usually as synonyms.

The terms "peptide" and "protein" comprise, according to the teaching, substances which contain not only amino acid components but also non-amino acid components such as sugars and phosphate structures, and also comprise substances containing bonds such as ester, thioether or disulfide bonds.

According to the teaching, the terms "initiation codon" and "start codon" synonymously refer to a codon (base triplet) of a RNA molecule that is potentially the first codon that is translated by a ribosome. Such codon typically encodes the amino acid methionine in eukaryotes and a modified methionine in prokaryotes. The most common initiation codon in eukaryotes and prokaryotes is AUG. Unless specifically stated herein that an initiation codon other than AUG is meant, the terms "initiation codon" and "start codon", with reference to an RNA molecule, refer to the codon AUG. According to the teaching, the terms "initiation codon" and "start codon" are also used to refer to a corresponding base triplet of a deoxyribonucleic acid, namely the base triplet encoding the initiation codon of a RNA. If the initiation codon of messenger RNA is AUG, the base triplet encoding the AUG is ATG. According to the teaching, the terms "initiation codon" and "start codon" preferably refer to a functional initiation codon or start codon, i.e. to an initiation codon or start codon that is used or would be used as a codon by a ribosome to start translation. There may be AUG codons in an RNA molecule that are not used as codons by a ribosome to start translation, e.g. due to a short distance of the codons to the cap. These codons are not encompassed by the term functional initiation codon or start codon.

According to the teaching, the terms "start codon of the open reading frame" or "initiation codon of the open reading frame" refer to the base triplet that serves as initiation codon for protein synthesis in a coding sequence, e.g. in the coding sequence of a nucleic acid molecule found in nature. In an RNA molecule, the start codon of the open reading frame is often preceded by a 5' untranslated region (5'-UTR), although this is not strictly required.

According to the teaching, the terms "native start codon of the open reading frame" or "native initiation codon of the open reading frame" refer to the base triplet that serves as initiation codon for protein synthesis in a native coding sequence. A native coding sequence may be e.g. the coding sequence of a nucleic acid molecule found in nature. In some embodiments, the present teaching provides variants of nucleic acid molecules found in nature, which are characterized in that the native start codon (which is present in the native coding sequence) has been removed (so that it is not present in the variant nucleic acid molecule).

According to the teaching, "first AUG" means the most upstream AUG base triplet of a messenger RNA molecule, preferably the most upstream AUG base triplet of a messenger RNA molecule that is used or would be used as a codon by a ribosome to start translation. Accordingly, "first ATG" refers to the ATG base triplet of a coding DNA sequence that encodes the first AUG. In some instances, the first AUG of a mRNA molecule is the start codon of an open reading frame, i.e. the codon that is used as start codon during ribosomal protein synthesis.

According to the teaching, the terms "comprises the removal" or "characterized by the removal" and similar terms, with reference to a certain element of a nucleic acid variant, mean that said certain element is not functional or not present in the nucleic acid variant, compared to a reference nucleic acid molecule. Without limitation, a removal can consist of deletion of all or part of the certain element, of substitution of all or part of the certain element, or of alteration of the functional or structural properties of the certain element. The removal of a functional element of a nucleic acid sequence requires that the function is not exhibited at the position of the nucleic acid variant comprising the removal. For example, a RNA variant characterized by the removal of a certain initiation codon requires that ribosomal protein synthesis is not initiated at the position of the RNA variant characterized by the removal. The removal of a structural element of a nucleic acid sequence requires that the structural element is not present at the position of the nucleic acid variant comprising the removal. For example, a RNA variant characterized by the removal of a certain AUG base triplet, i.e. of a AUG base triplet at a certain position, may be characterized, e.g. by deletion of part or all of the certain AUG base triplet (e.g. ΔAUG), or by substitution of one or more nucleotides (A, U, G) of the certain AUG base triplet by any one or more different nucleotides, so that the resulting nucleotide sequence of the variant does not comprise said AUG base triplet. Suitable substitutions of one nucleotide are those that convert the AUG base triplet into a GUG, CUG or UUG base triplet, or into a AAG, ACG or AGG base triplet, or into a AUA, AUC or AUU base triplet. Suitable substitutions of more nucleotides can be selected accordingly.

According to the teaching, the term "self-replicating virus" includes RNA viruses capable of replicating autonomously in a host cell. Self-replicating viruses may have a single-stranded RNA (ssRNA) genome and include alphaviruses, flaviviruses, measles viruses (MVs) and rhabdoviruses. Alphaviruses and flaviviruses possess a genome of positive polarity, whereas the genome of measles viruses (MVs) and rhabdoviruses is negative strand ssRNA. Typically, a self-replicating virus is a virus with a (+) stranded RNA genome which can be directly translated after infection of a cell, and this translation provides a RNA-dependent RNA polymerase which then produces both antisense and sense transcripts from the infected RNA. In the following, the teaching is illustrated by referring to alphavirus-derived vectors as an example of self-replicating virus-derived vectors. However, it is to be understood that the present teaching is not limited to alphavirus-derived vectors.

According to the teaching, the term "alphavirus" is to be understood broadly and includes any virus particle that has characteristics of alphaviruses. Characteristics of alphavirus include the presence of a (+) stranded RNA which encodes genetic information suitable for replication in a host cell, including RNA polymerase activity. Further characteristics of many alphaviruses are described e.g. in Strauss & Strauss, Microbiol. Rev., 1994, vol. 58, pp. 491-562. The term "alphavirus" includes alphavirus found in nature, as well as any variant or derivative thereof. In some embodiments, a variant or derivative is not found in nature.

In one embodiment, the alphavirus is an alphavirus found in nature. Typically, an alphavirus found in nature is infectious to any one or more eukaryotic organisms, such as an animal (including a vertebrate such as a human, and an arthropod such as an insect).

An alphavirus found in nature is preferably selected from the group consisting of the following: Barmah Forest virus complex (comprising Barmah Forest virus); Eastern equine encephalitis complex (comprising seven antigenic types of Eastern equine encephalitis virus); Middelburg virus complex (comprising Middelburg virus); Ndumu virus complex (comprising Ndumu virus); Semliki Forest virus complex (comprising Bebaru virus, Chikungunya virus, Mayaro virus and its subtype Una virus, O'Nyong Nyong virus, and its subtype Igbo-Ora virus, Ross River virus and its subtypes Bebaru virus, Getah virus, Sagiyama virus, Semliki Forest virus and its subtype Me Tri virus); Venezuelan equine encephalitis complex (comprising Cabassou virus, Everglades virus, Mosso das Pedras virus, Mucambo virus, Paramana virus, Pixuna virus, Rio Negro virus, Trocara virus and its subtype Bijou Bridge virus, Venezuelan equine encephalitis virus); Western equine encephalitis complex (comprising Aura virus, Babanki virus, Kyzylagach virus, Sindbis virus, Ockelbo virus, Whataroa virus, Buggy Creek virus, Fort Morgan virus, Highlands J virus, Western equine encephalitis virus); and some unclassified viruses including Salmon pancreatic disease virus; Sleeping Disease virus; Southern elephant seal virus; Tonate virus. More preferably, the alphavirus is selected from the group consisting of Semliki Forest virus complex (comprising the virus types as indicated above, including Semliki Forest virus), Western equine encephalitis complex (comprising the virus types as indicated above, including Sindbis virus), Eastern equine encephalitis virus (comprising the virus types as indicated above), Venezuelan equine encephalitis complex (comprising the virus types as indicated above, including Venezuelan equine encephalitis virus).

In a further preferred embodiment, the alphavirus is Semliki Forest virus. In an alternative further preferred embodiment, the alphavirus is Sindbis virus. In an alternative further preferred embodiment, the alphavirus is Venezuelan equine encephalitis virus.

In some embodiments of the present teaching, the alphavirus is not an alphavirus found in nature. Typically, an alphavirus not found in nature is a variant or derivative of an alphavirus found in nature, that is distinguished from an alphavirus found in nature by at least one mutation in the nucleotide sequence, i.e. the genomic RNA. The mutation in the nucleotide sequence may be selected from an insertion, a substitution or a deletion of one or more nucleotides, compared to an alphavirus found in nature. A mutation in the nucleotide sequence may or may not be associated with a mutation in a polypeptide or protein encoded by the nucleotide sequence. For example, an alphavirus not found in nature may be an attenuated alphavirus. An attenuated alphavirus not found in nature is an alphavirus that typically has at least one mutation in its nucleotide sequence by which it is distinguished from an alphavirus found in nature, and that is either not infectious at all, or that is infectious but has a lower disease-producing ability or no disease-producing ability at all. As an illustrative example, TC83 is an attenuated alphavirus that is distinguished from the Venezuelan equine encephalitis virus (VEEV) found in nature (McKinney et al., 1963, Am. J. Trop. Med. Hyg., 1963, vol. 12; pp. 597-603).

Members of the alphavirus genus may also be classified based on their relative clinical features in humans: alphaviruses associated primarily with encephalitis, and alphaviruses associated primarily with fever, rash, and polyarthritis.

The term "alphaviral" means found in an alphavirus, or originating from an alphavirus or derived from an alphavirus, e.g. by genetic engineering.

According to the teaching, "SFV" stands for Semliki Forest virus. According to the teaching, "SIN" or "SINV" stands for Sindbis virus. According to the teaching, "VEE" or "VEEV" stands for Venezuelan equine encephalitis virus.

According to the teaching, the term "of an alphavirus" refers to an entity of origin from an alphavirus. For illustration, a protein of an alphavirus may refer to a protein that is found in alphavirus and/or to a protein that is encoded by alphavirus; and a nucleic acid sequence of an alphavirus may refer to a nucleic acid sequence that is found in alphavirus and/or to a nucleic acid sequence that is encoded by alphavirus. Preferably, a nucleic acid sequence "of an alphavirus" refers to a nucleic acid sequence "of the genome of an alphavirus" and/or "of genomic RNA of an alphavirus".

According to the teaching, the term "alphaviral RNA" refers to any one or more of alphaviral genomic RNA (i.e. (+) strand), complement of alphaviral genomic RNA (i.e. (-) strand), and the subgenomic transcript (i.e. (+) strand), or a fragment of any thereof.

According to the teaching, "alphavirus genome" refers to genomic (+) strand RNA of an alphavirus.

According to the teaching, the term "native alphavirus sequence" and similar terms typically refer to a (e.g. nucleic acid) sequence of a naturally occurring alphavirus (alphavirus found in nature). In some embodiments, the term "native alphavirus sequence" also includes a sequence of an attenuated alphavirus.

According to the teaching, the term "5' replication recognition sequence" preferably refers to a continuous nucleic acid sequence, preferably a ribonucleic acid sequence, that is identical or homologous to a 5' fragment of a genome of a self-replicating virus, such as an alphavirus genome. The "5' replication recognition sequence" is a nucleic acid sequence that can be recognized by a replicase such as an alphaviral replicase. The term 5' replication recognition sequence includes native 5' replication recognition sequences as well as functional equivalents thereof, such as, e.g., functional variants of a 5' replication recognition sequence of a self-replicating virus found in nature, e.g., alphavirus found in nature. According to the teaching, functional equivalents include derivatives of 5' replication recognition sequences characterized by the removal of at least one initiation codon as described herein. The 5' replication recognition sequence is required for synthesis of the (-) strand complement of alphavirus genomic RNA, and is required for synthesis of (+) strand viral genomic RNA based on a (-) strand template. A native 5' replication recognition sequence typically encodes at least the N-terminal fragment of nsP1; but does not comprise the entire open reading frame encoding nsP1234. In view of the fact that a native 5' replication recognition sequence typically encodes at least the N-terminal fragment of nsP1, a native 5' replication recognition sequence typically comprises at least one initiation codon, typically AUG. In one embodiment, the 5' replication recognition sequence comprises conserved sequence element 1 of an alphavirus genome (CSE 1) or a variant thereof and conserved sequence element 2 of an alphavirus genome (CSE 2) or a variant thereof. The 5' replication recognition sequence is typically capable of forming four stem loops (SL), i.e. SL1, SL2, SL3, SL4. The numbering of these stem loops begins at the 5' end of the 5' replication recognition sequence.

The term "conserved sequence element" or "CSE" refers to a nucleotide sequence found in alphavirus RNA. These sequence elements are termed "conserved" because orthologs are present in the genome of different alphaviruses, and orthologous CSEs of different alphaviruses preferably share a high percentage of sequence identity and/or a similar secondary or tertiary structure. The term CSE includes CSE 1, CSE 2, CSE 3 and CSE 4.

According to the teaching, the terms "CSE 1" or "44-nt CSE" synonymously refer to a nucleotide sequence that is required for (+) strand synthesis from a (-) strand template. The term "CSE 1" refers to a sequence on the (+) strand; and the complementary sequence of CSE 1 (on the (-) strand) functions as a promoter for (+) strand synthesis. Preferably, the term CSE 1 includes the most 5' nucleotide of the alphavirus genome. CSE 1 typically forms a conserved stem-loop structure. Without wishing to be bound to a particular theory, it is believed that, for CSE 1, the secondary structure is more important than the primary structure, i.e. the linear sequence. In genomic RNA of the model alphavirus Sindbis virus, CSE 1 consists of a consecutive sequence of 44 nucleotides, which is formed by the most 5' 44 nucleotides of the genomic RNA (Strauss & Strauss, Microbiol. Rev., 1994, vol. 58, pp. 491-562).

According to the teaching, the terms "CSE 2" or "51-nt CSE" synonymously refer to a nucleotide sequence that is required for (-) strand synthesis from a (+) strand template. The (+) strand template is typically alphavirus genomic RNA or an RNA replicon (note that the subgenomic RNA transcript, which does not comprise CSE 2, does not function as a template for (-) strand synthesis). In alphavirus genomic RNA, CSE 2 is typically localized within the coding sequence for nsP1. In genomic RNA of the model alphavirus Sindbis virus, the 51-nt CSE is located at nucleotide positions 155-205 of genomic RNA (Frolov et al., 2001, RNA, vol. 7, pp. 1638-1651). CSE 2 forms typically two conserved stem loop structures. These stem loop structures are designated as stem loop 3 (SL3) and stem loop 4 (SL4) because they are the third and fourth conserved stem loop, respectively, of alphavirus genomic RNA, counted from the 5' end of alphavirus genomic RNA. Without wishing to be bound to a particular theory, it is believed that, for CSE 2, the secondary structure is more important than the primary structure, i.e. the linear sequence.

According to the teaching, the terms "CSE 3" or "junction sequence" synonymously refer to a nucleotide sequence that is derived from alphaviral genomic RNA and that comprises the start site of the subgenomic RNA. The complement of this sequence in the (-) strand acts to promote subgenomic RNA transcription. In alphavirus genomic RNA, CSE 3 typically overlaps with the region encoding the C-terminal fragment of nsP4 and extends to a short non-coding region located upstream of the open reading frame encoding the structural proteins.

According to the teaching, the terms "CSE 4" or "19-nt conserved sequence" or "19-nt CSE" synonymously refer to a nucleotide sequence from alphaviral genomic RNA, immediately upstream of the poly(A) sequence in the 3' untranslated region of the alphavirus genome. CSE 4 typically consists of 19 consecutive nucleotides. Without wishing to be bound to a particular theory, CSE 4 is understood to function as a core promoter for initiation of (-) strand synthesis (José et al., Future Microbiol., 2009, vol. 4, pp. 837-856); and/or CSE 4 and the poly(A) tail of the alphavirus genomic RNA are understood to function together for efficient (-) strand synthesis (Hardy & Rice, J. Virol., 2005, vol. 79, pp. 4630-4639).

According to the teaching, the term "subgenomic promoter" or "SGP" refers to a nucleic acid sequence upstream (5') of a nucleic acid sequence (e.g. coding sequence), which controls transcription of said nucleic acid sequence by providing a recognition and binding site for RNA polymerase, typically RNA-dependent RNA polymerase, in particular functional alphavirus non-structural protein. The SGP may include further recognition or binding sites for further factors. A subgenomic promoter is typically a genetic element of a positive strand RNA virus, such as an alphavirus. A subgenomic promoter of alphavirus is a nucleic acid sequence comprised in the viral genomic RNA. The subgenomic promoter is generally characterized in that it allows initiation of the transcription (RNA synthesis) in the presence of an RNA-dependent RNA polymerase, e.g. functional alphavirus non-structural protein. A RNA (-) strand, i.e. the complement of alphaviral genomic RNA, serves as a template for synthesis of a (+) strand subgenomic transcript, and synthesis of the (+) strand subgenomic transcript is typically initiated at or near the subgenomic promoter. The term "subgenomic promoter" as used herein, is not confined to any particular localization in a nucleic acid comprising such subgenomic promoter. In some embodiments, the SGP is identical to CSE 3 or overlaps with CSE 3 or comprises CSE 3.

The terms "subgenomic transcript" or "subgenomic RNA" synonymously refer to a RNA molecule that is obtainable as a result of transcription using a RNA molecule as template ("template RNA"), wherein the template RNA comprises a subgenomic promoter that controls transcription of the subgenomic transcript. The subgenomic transcript is obtainable in the presence of an RNA-dependent RNA polymerase, in particular functional alphavirus non-structural protein. For instance, the term "subgenomic transcript" may refer to the RNA transcript that is prepared in a cell infected by an alphavirus, using the (-) strand complement of alphavirus genomic RNA as template. However, the term "subgenomic transcript", as used herein, is not limited thereto and also includes transcripts obtainable by using heterologous RNA as template. For example, subgenomic transcripts are also obtainable by using the (-) strand complement of SGP-containing replicons according to the present teaching as template. Thus, the term "subgenomic transcript" may refer to a RNA molecule that is obtainable by transcribing a fragment of alphavirus genomic RNA, as well as to a RNA molecule that is obtainable by transcribing a fragment of a replicon according to the present teaching.

The term "autologous" is used to describe anything that is derived from the same subject. For example, "autologous cell" refers to a cell derived from the same subject. Introduction of autologous cells into a subject is advantageous because these cells overcome the immunological barrier which otherwise results in rejection.

The term "allogeneic" is used to describe anything that is derived from different individuals of the same species. Two or more individuals are said to be allogeneic to one another when the genes at one or more loci are not identical.

The term "syngeneic" is used to describe anything that is derived from individuals or tissues having identical genotypes, i.e., identical twins or animals of the same inbred strain, or their tissues or cells.

The term "heterologous" is used to describe something consisting of multiple different elements. As an example, the introduction of one individual's cell into a different individual constitutes a heterologous transplant. A heterologous gene is a gene derived from a source other than the subject.

The following provides specific and/or preferred variants of the individual features of the teaching. The present teaching also contemplates as particularly preferred embodiments those embodiments, which are generated by combining two or more of the specific and/or preferred variants described for two or more of the features of the present teaching.

### RNA replicon

### Internal ribosome entry site (IRES)-dependent translation of the replicase open reading frame

The present teaching provides an RNA replicon comprising an internal ribosome entry site (IRES) and an open reading frame encoding a functional non-structural protein from a self-replicating virus, wherein the IRES controls expression of the functional non-structural protein. Preferably, the RNA replicon contains sequence elements allowing replication by the functional non-structural protein. In one embodiment, the self-replicating virus is an alphavirus and the sequence elements allowing replication by the functional non-structural protein are derived from an alphavirus.

Alphavirus replicases have a capping enzyme function, and, typically, genomic as well as subgenomic (+) stranded RNAs are capped. The 5'-cap serves to protect mRNA from degradation, and to direct the ribosomal subunits as well as cellular factors to the mRNA in order to form a ribonucleoprotein complex on the mRNA that then can start translation from a nearby start codon. This complex process is extensively described in the literature (for Review: Jackson et al 2010, Nat Rev Mol Biol; Vol 10;pp 113-127).

Despite the very elaborated and efficient mechanism of cap dependent translation, cells have means to initiate translation fully or partially independently from the 5'cap (Thompson 2012; Trends in Microbiology, Vol. 20, No. 11; pp 558-566). Thereby, in situations of cellular stress that lead to a global down regulation of cap-dependent translation, the cells may still express selected genes preferentially, often with the help of an IRES.

Viruses also evolved different means to exploit the cells machinery for translation of the viral genes. Since a viral infection is often sensed by the cell which leads to cellular antiviral response (interferon response; stress response), many viruses also make use of cap-independent translation, especially RNA viruses. Cap independent translation ensure an advantage for the viral RNA translation upon cellular stress response giving the viruses the opportunity to fulfil their life cycle and be released from infected cells.

Internal ribosomal entry sites (IRESs) are RNA sequences forming appropriate secondary structures that attract the pre-initiation complex near to a translational start codon, AUG or others. Four classes of IRESs are described in literature that share common features. Prototypic IRESs are the poliovirus IRES (Type I), the encephalomyocarditis virus (EMCV) IRES (Type II), the hepathitis C virus (HCV) IRES (Type III) and the IRES found in the intergenic regions of dicistroviruses (Type IV)( Thompson 2012; Trends in Microbiology, Vol. 20, No. 11; pp 558-566; Lozano et al. 2018; Open Biology, Vol. 8:180155).

Type I to III IRESs have in common that they initiate translation at AUG start codons, whereas type IV IRES initiate at non-AUG codons (e.g. GCU). Thereby Type I to III require the initiator tRNA that delivers methionin by the help of eIF2/GTP (eIF2/GTP/Met-tRNAiMet). Activation of elF2 kinases under stress phosphorylates the alpha subunit of elF2 which inhibits translation that initiates at AUG. Thereby translation directed by type IV IRESs are not inhibited by elF2 phosphorylation.

According to the teaching, the term "internal ribosome entry site", abbreviated "IRES", relates to an RNA element that recruits ribosomes to the internal region of mRNAs to initiate translation in a cap-independent manner. IRESs are commonly located in the 5'-UTR of RNA viruses. However, mRNAs of viruses from dicistroviridae family possess two open reading frames (ORFs), and translation of each is directed by two distinct IRESs. It has also been suggested that some mammalian cellular mRNAs also have IRESs. These cellular IRES elements are thought to be located in eukaryotic mRNAs encoding genes involved in stress survival, and other processes critical to survival. The location for IRES elements is often in the 5'-UTR, but can also occur elsewhere in mRNAs.

According to the teaching, the term "internal ribosome entry site", abbreviated "IRES" includes IRESs that are present in the viruses of the *Picornaviridae* family such as poliovirus (PV) and encephalomyocarditis virus and pathogenic viruses, including human immunodeficiency virus, hepatitis C virus (HCV), and foot and mouth disease **virus. Although** these viral IRESs contain diverse sequences, many of them have similar secondary structures and initiate translation through similar mechanisms. In addition, the activities of IRESs often require assistance from other factors known as IRES-transacting factors (ITAFs). Based on the structures and the requirement of translation initiation factors (IFs) and ITAFs, the viral IRESs are classified into four types as described herein. Any of these IRES types is useful according to the teaching, with Type IV IRESs being particularly preferred.

Two groups of viral IRESs, Type I and Type II, cannot bind to the 40S small ribosomal subunit directly. Instead, they recruit the 40S small ribosomal subunit through different ITAFs and require canonical IFs in the cap-dependent translation (i.e. eIF2, elF3, elF4A, elF4B, and elF4G). The major difference between Type I and Type II IRESs is the requirement of 40S ribosome scanning, with 40S ribosome scanning being unnecessary for Type II IRES. Examples of Type I IRESs include IRESs found in poliovirus (PV) and rhinovirus. Examples of Type II IRESs include IRESs found in encephalomyocarditis virus (EMCV), foot-and-mouth disease virus (FMDV) and Theiler's murine encephalomyelitis viruses (TMEV).

Type III IRESs can directly interact with 40S small ribosomal subunit with specialized RNA structure, but their activities usually require assistance of several IFs including eIF2 and elF3 and initiator Met-tRNAi. Examples include IRESs found in hepatitis C-virus (HCV), classical swine fever virus (CSFV) and porcine teschovirus (PTV). Type IV viral IRESs generally have strong activities and can initiate translation from a non-AUG start codon without additional ITAFs or even eIF2/Met-tRNAi/GTP ternary complex. These IRESs are folded to a compact structure that directly interacts with the 40S small ribosomal subunit. Examples include IRESs found in dicistroviruses such as cricket paralysis virus (CrPV), plautia stali intestine virus (PSIV), and Taura-Syndrom-Virus (TSV).

According to the teaching, the term "internal ribosome entry site", abbreviated "IRES" also includes IRESs found in cellular mRNAs, many of which encode proteins required in stress response, e.g. in conditions of apoptosis, mitosis, hypoxia, and nutrient limitation. The cellular IRESs can be roughly classified into two types based on the mechanisms of ribosome recruitment: Type I IRESs interact with ribosomes through **ITAFs** that bound on the cis-elements, e.g. RNA binding motifs and N-6-methyladenosine (m6A) modification, whereas Type II IRESs contain a short cis-element that pairs with 18S rRNA to recruit ribosomes.

### Functional non-structural protein

The RNA replicon described herein comprises an open reading frame encoding a functional non-structural protein from a self-replicating virus the expression of which is under the control of an internal ribosome entry site (IRES).

The term "non-structural protein" relates to a protein encoded by a virus but that is not part of the viral particle. This term typically includes the various enzymes and transcription factors the virus uses to replicate itself, such as RNA replicase or other template-directed polymerases. The term "non-structural protein" includes each and every co- or post-translationally modified form, including carbohydrate-modified (such as glycosylated) and lipid-modified forms of a non-structural protein and preferably relates to an "alphavirus non-structural protein".

In some embodiments, the term "alphavirus non-structural protein" refers to any one or more of individual non-structural proteins of alphavirus origin (nsP1, nsP2, nsP3, nsP4), or to a poly-protein comprising the polypeptide sequence of more than one non-structural protein of alphavirus origin. In some embodiments, "alphavirus non-structural protein" refers to nsP123 and/or to nsP4. In other embodiments, "alphavirus non-structural protein" refers to nsP1234. In one embodiment, the protein of interest encoded by an open reading frame consists of all of nsP1, nsP2, nsP3 and nsP4 as one single, optionally cleavable poly-protein: nsP1234. In one embodiment, the protein of interest encoded by an open reading frame consists of nsP1, nsP2 and nsP3 as one single, optionally cleavable polyprotein: nsP123. In that embodiment, nsP4 may be a further protein of interest and may be encoded by a further open reading frame.

In some embodiments, non-structural protein is capable of forming a complex or association, e.g. in a host cell. In some embodiments, "alphavirus non-structural protein" refers to a complex or association of nsP123 (synonymously P123) and nsP4. In some embodiments, "alphavirus non-structural protein" refers to a complex or association of nsP1, nsP2, and nsP3. In some embodiments, "alphavirus non-structural protein" refers to a complex or association of nsP1, nsP2, nsP3 and nsP4.

In some embodiments, "alphavirus non-structural protein" refers to a complex or association of any one or more selected from the group consisting of nsP1, nsP2, nsP3 and nsP4. In some embodiments, the alphavirus non-structural protein comprises at least nsP4.

The terms "complex" or "association" refer to two or more same or different protein molecules that are in spatial proximity. Proteins of a complex are preferably in direct or indirect physical or physicochemical contact with each other. A complex or association can consist of multiple different proteins (heteromultimer) and/or of multiple copies of one particular protein (homomultimer). In the context of alphavirus non-structural protein, the term "complex or association" describes a multitude of at least two protein molecules, of which at least one is an alphavirus non-structural protein. The complex or association can consist of multiple copies of one particular protein (homomultimer) and/or of multiple different proteins (heteromultimer). In the context of a multimer, "multi" means more than one, such as two, three, four, five, six, seven, eight, nine, ten, or more than ten.

The term "functional non-structural protein" includes non-structural protein that has replicase function. Thus, "functional non-structural protein" includes alphavirus replicase. "Replicase function" comprises the function of an RNA-dependent RNA polymerase (RdRP), i.e. an enzyme which is capable to catalyze the synthesis of (-) strand RNA based on a (+) strand RNA template, and/or which is capable to catalyze the synthesis of (+) strand RNA based on a (-) strand RNA template. Thus, the term "functional non-structural protein" can refer to a protein or complex that synthesizes (-) stranded RNA, using the (+) stranded (e.g. genomic) RNA as template, to a protein or complex that synthesizes new (+) stranded RNA, using the (-) stranded complement of genomic RNA as template, and/or to a protein or complex that synthesizes a subgenomic transcript, using a fragment of the (-) stranded complement of genomic RNA as template. The functional non-structural protein may additionally have one or more additional functions, such as e.g. a protease (for auto-cleavage), helicase, terminal adenylyltransferase (for poly(A) tail addition), methyltransferase and guanylyltransferase (for providing a nucleic acid with a 5'-cap), nuclear localization sites, triphosphatase (Gould et al., 2010, Antiviral Res., vol. 87 pp. 111-124; Rupp et al., 2015, J. Gen. Virol., vol. 96, pp. 2483-500).

According to the teaching, the term "replicase" includes RNA-dependent RNA polymerase. According to the teaching, the term "replicase" includes "alphavirus replicase", including a RNA-dependent RNA polymerase from a naturally occurring alphavirus (alphavirus found in nature) and a RNA-dependent RNA polymerase from a variant or derivative of an alphavirus, such as from an attenuated alphavirus.

The term "replicase" comprises all variants, in particular post-translationally modified variants, conformations, isoforms and homologs of alphavirus replicase, which are expressed by alphavirus-infected cells or which are expressed by cells that have been transfected with a nucleic acid that codes for alphavirus replicase. Moreover, the term "replicase" comprises all forms of replicase that have been produced and can be produced by recombinant methods. For example, a replicase comprising a tag that facilitates detection and/or purification of the replicase in the laboratory, e.g. a myc-tag, a HA-tag or an oligohistidine tag (His-tag) may be produced by recombinant methods.

Optionally, the alphavirus replicase is additionally functionally defined by the capacity of binding to any one or more of alphavirus conserved sequence element 1 (CSE 1) or complementary sequence thereof, conserved sequence element 2 (CSE 2) or complementary sequence thereof, conserved sequence element 3 (CSE 3) or complementary sequence thereof, conserved sequence element 4 (CSE 4) or complementary sequence thereof. Preferably, the replicase is capable of binding to CSE 2 [i.e. to the (+) strand] and/or to CSE 4 [i.e. to the (+) strand], or of binding to the complement of CSE 1 [i.e. to the (-) strand] and/or to the complement of CSE 3 [i.e. to the (-) strand].

The origin of the alphavirus replicase is not limited to any particular alphavirus. In a preferred embodiment, the alphavirus replicase comprises non-structural protein from Semliki Forest virus, including a naturally occurring Semliki Forest virus and a variant or derivative of Semliki Forest virus, such as an attenuated Semliki Forest virus. In an alternative preferred embodiment, the alphavirus replicase comprises non-structural protein from Sindbis virus, including a naturally occurring Sindbis virus and a variant or derivative of Sindbis virus, such as an attenuated Sindbis virus. In an alternative preferred embodiment, the alphavirus replicase comprises non-structural protein from Venezuelan equine encephalitis virus (VEEV), including a naturally occurring VEEV and a variant or derivative of VEEV, such as an attenuated VEEV. In an alternative preferred embodiment, the alphavirus replicase comprises non-structural protein from chikungunya virus (CHIKV), including a naturally occurring CHIKV and a variant or derivative of CHIKV, such as an attenuated CHIKV.

A replicase can also comprise non-structural proteins from more than one virus, e.g., from more than one alphavirus. Thus, heterologous complexes or associations comprising alphavirus non-structural protein and having replicase function are equally comprised by the present teaching. Merely for illustrative purposes, replicase may comprise one or more non-structural proteins (e.g. nsP1, nsP2) from a first alphavirus, and one or more non-structural proteins (nsP3, nsP4) from a second alphavirus. Non-structural proteins from more than one different alphavirus may be encoded by separate open reading frames, or may be encoded by a single open reading frame as poly-protein, e.g. nsP1234.

In some embodiments, functional non-structural protein is capable of forming membranous replication complexes and/or vacuoles in cells in which the functional non-structural protein is expressed.

If functional non-structural protein, i.e. non-structural protein with replicase function, is encoded by a nucleic acid molecule according to the present teaching, it is preferable that the subgenomic promoter of the replicon, if present, is compatible with said replicase. Compatible in this context means that the replicase is capable of recognizing the subgenomic promoter, if present. In one embodiment, this is achieved when the subgenomic promoter is native to the virus from which the replicase is derived, i.e. the natural origin of these sequences is the same virus. In an alternative embodiment, the subgenomic promoter is not native to the virus from which the virus replicase is derived, provided that the virus replicase is capable of recognizing the subgenomic promoter. In other words, the replicase is compatible with the subgenomic promoter (cross-virus compatibility). Examples of cross-virus compatibility concerning subgenomic promoter and replicase originating from different alphaviruses are known in the art. Any combination of subgenomic promoter and replicase is possible as long as cross-virus compatibility exists. Cross-virus compatibility can readily be tested by the skilled person working the present teaching by incubating a replicase to be tested together with an RNA, wherein the RNA has a subgenomic promoter to be tested, at conditions suitable for RNA synthesis from the a subgenomic promoter. If a subgenomic transcript is prepared, the subgenomic promoter and the replicase are determined to be compatible. Various examples of cross-virus compatibility are known (reviewed by Strauss & Strauss, Microbiol. Rev., 1994, vol. 58, pp. 491-562).

The replicon can preferably be replicated by the functional non-structural protein. In particular, the RNA replicon that encodes functional non-structural protein can be replicated by the functional non-structural protein encoded by the replicon. In a preferred embodiment, the RNA replicon comprises an open reading frame encoding functional alphavirus non-structural protein. In one embodiment, the replicon comprises a further open reading frame encoding a protein of interest. This embodiment is particularly suitable in some methods for producing a protein of interest according to the present teaching. In one embodiment, the further open reading frame encoding a protein of interest is located downstream from the 5' replication recognition sequence and upstream from the IRES (and upstream from the open reading frame encoding a functional non-structural protein from a self-replicating virus) and/or downstream from the open reading frame encoding a functional non-structural protein from a self-replicating virus. The further open reading frame encoding a protein of interest located downstream from the 5' replication recognition sequence and upstream from the IRES (and upstream from the open reading frame encoding a functional non-structural protein from a self-replicating virus) may be expressed as a fusion protein with sequences encoded by the 5' replication recognition sequence. The further open reading frame encoding a protein of interest located downstream from the 5' replication recognition sequence and upstream from the IRES (and upstream from the open reading frame encoding a functional non-structural protein from a self-replicating virus) may be or may not be controlled by a subgenomic promoter. The one or more further open reading frames encoding one or more proteins of interest located downstream from the open reading frame encoding a functional non-structural protein from a self-replicating virus are generally controlled by (a) subgenomic promoter(s).

It is preferable that the open reading frame encoding functional non-structural protein does not overlap with the 5' replication recognition sequence. In one embodiment, the open reading frame encoding functional non-structural protein does not overlap with the subgenomic promoter, if present. Embodiments thereof are disclosed in WO 2017/162460.

### Uncoupling of sequence elements required for replication by the replicase and protein-coding regions

Versatile alphavirus-derived vectors are difficult to develop because the open reading frame encoding nsP1234 overlaps with the 5' replication recognition sequence of the alphavirus genome (coding sequence for nsP1) and typically also with the subgenomic promoter comprising CSE 3 (coding sequence for nsP4).

The RNA replicon described herein generally comprises sequence elements required for replication by the replicase, in particular a 5' replication recognition sequence. According to the teaching, the coding sequence for non-structural protein is under the control of an IRES and thus an IRES is located upstream of the coding sequence for non-structural protein. Thus, in one embodiment, the 5' replication recognition sequence which normally overlaps with the coding sequence for the N-terminal fragment of the alphavirus non-structural protein, is located upstream of the IRES and does not overlap with the coding sequence for non-structural protein.

In one embodiment, coding sequences of the 5' replication recognition sequence such as nsP1 coding sequences are fused in frame to a gene of interest which is located upstream from the IRES.

In one embodiment, the 5' replication recognition sequence does not encode any protein or fragment thereof, such as an alphavirus non-structural protein or fragment thereof. Thus, in the RNA replicon according to the teaching, the sequence elements required for replication by the replicase and protein-coding regions may be uncoupled. The uncoupling may be achieved by the removal of at least one initiation codon in the 5' replication recognition sequence compared to a native virus genomic RNA, e.g., native alphavirus genomic RNA.

Thus, the replicon may comprise a 5' replication recognition sequence, wherein the 5' replication recognition sequence is characterized in that it comprises the removal of at least one initiation codon compared to a native virus 5' replication recognition sequence, e.g., native alphavirus 5' replication recognition sequence.

The 5' replication recognition sequence that is characterized in that it comprises the removal of at least one initiation codon compared to a native virus 5' replication recognition sequence, according to the present teaching, can be referred to herein as "modified 5' replication recognition sequence" or "5' replication recognition sequence according to the teaching". As described herein below, the 5' replication recognition sequence according to the teaching may optionally be characterized by the presence of one or more additional nucleotide changes.

A nucleic acid construct that is capable of being replicated by a replicase, preferably an alphaviral replicase, is termed replicon. According to the teaching, the term "replicon" defines a RNA molecule that can be replicated by RNA-dependent RNA polymerase, yielding - without DNA intermediate - one or multiple identical or essentially identical copies of the RNA replicon. "Without DNA intermediate" means that no deoxyribonucleic acid (DNA) copy or complement of the replicon is formed in the process of forming the copies of the RNA replicon, and/or that no deoxyribonucleic acid (DNA) molecule is used as a template in the process of forming the copies of the RNA replicon, or complement thereof. The replicase function is typically provided by functional non-structural protein, e.g., functional alphavirus non-structural protein.

According to the teaching, the terms "can be replicated" and "capable of being replicated" generally describe that one or more identical or essentially identical copies of a nucleic acid can be prepared. When used together with the term "replicase", such as in "capable of being replicated by a replicase", the terms "can be replicated" and "capable of being replicated" describe functional characteristics of a nucleic acid molecule, e.g. a RNA replicon, with respect to a replicase. These functional characteristics comprise at least one of (i) the replicase is capable of recognizing the replicon and (ii) the replicase is capable of acting as RNA-dependent RNA polymerase (RdRP). Preferably, the replicase is capable of both (i) recognizing the replicon and (ii) acting as RNA-dependent RNA polymerase.

The expression "capable of recognizing" describes that the replicase is capable of physically associating with the replicon, and preferably, that the replicase is capable of binding to the replicon, typically non-covalently. The term "binding" can mean that the replicase has the capacity of binding to any one or more of a conserved sequence element 1 (CSE 1) or complementary sequence thereof (if comprised by the replicon), conserved sequence element 2 (CSE 2) or complementary sequence thereof (if comprised by the replicon), conserved sequence element 3 (CSE 3) or complementary sequence thereof (if comprised by the replicon), conserved sequence element 4 (CSE 4) or complementary sequence thereof (if comprised by the replicon). Preferably, the replicase is capable of binding to CSE 2 [i.e. to the (+) strand] and/or to CSE 4 [i.e. to the (+) strand], or of binding to the complement of CSE 1 [i.e. to the (-) strand] and/or to the complement of CSE 3 [i.e. to the (-) strand].

In one embodiment, the expression "capable of acting as RdRP" means that the replicase is capable to catalyze the synthesis of the (-) strand complement of viral genomic (+) strand RNA, wherein the (+) strand RNA has template function, and/or that the replicase is capable to catalyze the synthesis of (+) strand viral genomic RNA, wherein the (-) strand RNA has template function. In general, the expression "capable of acting as RdRP" can also include that the replicase is capable to catalyze the synthesis of a (+) strand subgenomic transcript wherein a (-) strand RNA has template function, and wherein synthesis of the (+) strand subgenomic transcript is typically initiated at a subgenomic promoter. In one embodiment, the virus is an alphavirus.

The expressions "capable of binding" and "capable of acting as RdRP" refer to the capability at normal physiological conditions. In particular, they refer to the conditions inside a cell, which expresses functional non-structural protein or which has been transfected with a nucleic acid that codes for functional non-structural protein. The cell is preferably a eukaryotic cell. The capability of binding and/or the capability of acting as RdRP can be experimentally tested, e.g. in a cell-free *in vitro* system or in a eukaryotic cell. Optionally, said eukaryotic cell is a cell from a species to which the particular virus that represents the origin of the replicase is infectious. For example, when the virus replicase from a particular virus is used that is infectious to humans, the normal physiological conditions are conditions in a human cell. More preferably, the eukaryotic cell (in one example human cell) is from the same tissue or organ to which the particular virus that represents the origin of the replicase is infectious.

According to the teaching, "compared to a native alphavirus sequence" and similar terms refer to a sequence that is a variant of a native alphavirus sequence. The variant is typically not itself a native alphavirus sequence.

In one embodiment, the RNA replicon comprises a 3' replication recognition sequence. A 3' replication recognition sequence is a nucleic acid sequence that can be recognized by functional non-structural protein. In other words, functional non-structural protein is capable of recognizing the 3' replication recognition sequence. Preferably, the 3' replication recognition sequence is located at the 3' end of the replicon (if the replicon does not comprise a poly(A) tail), or immediately upstream of the poly(A) tail (if the replicon comprises a poly(A) tail). In one embodiment, the 3' replication recognition sequence consists of or comprises CSE 4.

In one embodiment, the 5' replication recognition sequence and the 3' replication recognition sequence are capable of directing replication of the RNA replicon according to the present teaching in the presence of functional non-structural protein. Thus, when present alone or preferably together, these recognition sequences direct replication of the RNA replicon in the presence of functional non-structural protein.

It is preferable that a functional non-structural protein is provided that is capable of recognizing both the 5' replication recognition sequence and the 3' replication recognition sequence of the replicon. In one embodiment, this is achieved when the 3' replication recognition sequence is native to the alphavirus from which the functional alphavirus non-structural protein is derived, and when the 5' replication recognition sequence is native to the alphavirus from which the functional alphavirus non-structural protein is derived or is a variant of the 5' replication recognition sequence that is native to the alphavirus from which the functional alphavirus non-structural protein is derived. Native means that the natural origin of these sequences is the same alphavirus. In an alternative embodiment, the 5' replication recognition sequence and/or the 3' replication recognition sequence are not native to the alphavirus from which the functional alphavirus non-structural protein is derived, provided that the functional alphavirus non-structural protein is capable of recognizing both the 5' replication recognition sequence and the 3' replication recognition sequence of the replicon. In other words, the functional alphavirus non-structural protein is compatible to the 5' replication recognition sequence and the 3' replication recognition sequence. When a non-native functional alphavirus non-structural protein is capable of recognizing a respective sequence or sequence element, the functional alphavirus non-structural protein is said to be compatible (cross-virus compatibility). Any combination of (3'/5') replication recognition sequences and CSEs, respectively, with functional alphavirus non-structural protein is possible as long as cross-virus compatibility exists. Cross-virus compatibility can readily be tested by the skilled person working the present teaching by incubating a functional alphavirus non-structural protein to be tested together with an RNA, wherein the RNA has 3'- and 5' replication recognition sequences to be tested, at conditions suitable for RNA replication, e.g. in a suitable host cell. If replication occurs, the (3'/5') replication recognition sequences and the functional alphavirus non-structural protein are determined to be compatible.

The removal of at least one initiation codon within the 5' replication recognition sequence provides several advantages. Absence of an initiation codon in the nucleic acid sequence encoding nsP1* (N-terminal fragment of nsP1) will typically cause that nsP1* is not translated. Further, since nsP1* is not translated, the open reading frame encoding the protein of interest ("GOI 2") is the most upstream open reading frame accessible to the ribosome; thus when the replicon is present in a cell, translation is initiated at the first AUG of the open reading frame (RNA) encoding the gene of interest.

The removal of at least one initiation codon according to the present teaching can be achieved by any suitable method known in the art. For example, a suitable DNA molecule encoding the replicon according to the teaching, i.e. characterized by the removal of an initiation codon, can be designed *in silico,* and subsequently synthesized *in vitro* (gene synthesis); alternatively, a suitable DNA molecule may be obtained by site-directed mutagenesis of a DNA sequence encoding a replicon. In any case, the respective DNA molecule may serve as template for *in* vitro transcription, thereby providing the replicon according to the teaching.

The removal of at least one initiation codon compared to a native 5' replication recognition sequence is not particularly limited and may be selected from any nucleotide modification, including substitution of one or more nucleotides (including, on DNA level, a substitution of A and/or T and/or G of the initiation codon); deletion of one or more nucleotides (including, on DNA level, a deletion of A and/or T and/or G of the initiation codon), and insertion of one or more nucleotides (including, on DNA level, an insertion of one or more nucleotides between A and T and/or between T and G of the initiation codon). Irrespective of whether the nucleotide modification is a substitution, an insertion or a deletion, the nucleotide modification must not result in the formation of a new initiation codon (as an illustrative example: an insertion, at DNA level, must not be an insertion of an ATG).

The 5' replication recognition sequence of the RNA replicon that is characterized by the removal of at least one initiation codon (i.e. the modified 5' replication recognition sequence according to the present teaching) is preferably a variant of a 5' replication recognition sequence of the genome of an alphavirus found in nature. In one embodiment, the modified 5' replication recognition sequence according to the present teaching is preferably characterized by a degree of sequence identity of 80 % or more, preferably 85 % or more, more preferably 90 % or more, even more preferably 95 % or more, to the 5' replication recognition sequence of the genome of at least one alphavirus found in nature.

In one embodiment, the 5' replication recognition sequence of the RNA replicon that may be characterized by the removal of at least one initiation codon comprises a sequence homologous to about 250 nucleotides at the 5' end of an alphavirus, i.e. at the 5' end of the alphaviral genome. In a preferred embodiment, it comprises a sequence homologous to about 250 to 500, preferably about 300 to 500 nucleotides at the 5' end of an alphavirus, i.e. at the 5' end of the alphaviral genome. "At the 5' end of the alphaviral genome" means a nucleic acid sequence beginning at, and including, the most upstream nucleotide of the alphaviral genome. In other words, the most upstream nucleotide of the alphaviral genome is designated nucleotide no. 1, and e.g. "250 nucleotides at the 5' end of the alphaviral genome" means nucleotides 1 to 250 of the alphaviral genome. In one embodiment, the 5' replication recognition sequence of the RNA replicon is characterized by a degree of sequence identity of 80 % or more, preferably 85 % or more, more preferably 90 % or more, even more preferably 95 % or more, to at least 250 nucleotides at the 5' end of the genome of at least one alphavirus found in nature. At least 250 nucleotides includes e.g. 250 nucleotides, 300 nucleotides, 400 nucleotides, 500 nucleotides.

The 5' replication recognition sequence of an alphavirus found in nature is typically characterized by at least one initiation codon and/or by conserved secondary structural motifs. For example, the native 5' replication recognition sequence of Semliki Forest virus (SFV) comprises five specific AUG base triplets. According to Frolov et al. (2001, RNA, vol. 7, pp. 1638-1651) analysis by MFOLD revealed that the native 5' replication recognition sequence of Semliki Forest virus is predicted to form four stem loops (SL), termed stem loops 1 to 4 (SL1, SL2, SL3, SL4). According to Frolov et al., analysis by MFOLD revealed that also the native 5' replication recognition sequence of a different alphavirus, Sindbis virus, is predicted to form four stem loops: SL1, SL2, SL3, SL4.

It is known that the 5' end of the alphaviral genome comprises sequence elements that enable replication of the alphaviral genome by functional alphavirus non-structural protein. In one embodiment of the present teaching, the 5' replication recognition sequence of the RNA replicon comprises a sequence homologous to conserved sequence element 1 (CSE 1) and/or a sequence homologous to conserved sequence element 2 (CSE 2) of an alphavirus.

Conserved sequence element 2 (CSE 2) of alphavirus genomic RNA typically is represented by SL3 and SL4 which is preceded by SL2 comprising at least the native initiation codon that encodes the first amino acid residue of alphavirus non-structural protein nsP1. In this description, however, in some embodiments, the conserved sequence element 2 (CSE 2) of alphavirus genomic RNA refers to a region spanning from SL2 to SL4 and comprising the native initiation codon that encodes the first amino acid residue of alphavirus non-structural protein nsP1. In a preferred embodiment, the RNA replicon comprises CSE 2 or a sequence homologous to CSE 2. In one embodiment, the RNA replicon comprises a sequence homologous to CSE 2 that is preferably characterized by a degree of sequence identity of 80 % or more, preferably 85 % or more, more preferably 90 % or more, even more preferably 95 % or more, to the sequence of CSE 2 of at least one alphavirus found in nature.

In a preferred embodiment, the 5' replication recognition sequence comprises a sequence that is homologous to CSE 2 of an alphavirus. The CSE 2 of an alphavirus may comprise a fragment of an open reading frame of a non-structural protein from an alphavirus.

Thus, in a preferred embodiment, the RNA replicon is characterized in that it comprises a sequence homologous to an open reading frame of a non-structural protein or a fragment thereof from an alphavirus. The sequence homologous to an open reading frame of a non-structural protein or a fragment thereof is typically a variant of an open reading frame of a non-structural protein or a fragment thereof of an alphavirus found in nature. In one embodiment, the sequence homologous to an open reading frame of a non-structural protein or a fragment thereof is preferably characterized by a degree of sequence identity of 80 % or more, preferably 85 % or more, more preferably 90 % or more, even more preferably 95 % or more, to an open reading frame of a non-structural protein or a fragment thereof of at least one alphavirus found in nature.

In a more preferred embodiment, the sequence homologous to an open reading frame of a non-structural protein that is comprised by the replicon of the present teaching does not comprise the native initiation codon of a non-structural protein, and more preferably does not comprise any initiation codon of a non-structural protein. In a preferred embodiment, the sequence homologous to CSE 2 is characterized by the removal of all initiation codons compared to a native alphavirus CSE 2 sequence. Thus, the sequence homologous to CSE 2 does preferably not comprise any initiation codon.

When the sequence homologous to an open reading frame does not comprise any initiation codon, the sequence homologous to an open reading frame is not itself an open reading frame since it does not serve as a template for translation.

In one embodiment, the 5' replication recognition sequence comprises a sequence homologous to an open reading frame of a non-structural protein or a fragment thereof from an alphavirus, wherein the sequence homologous to an open reading frame of a non-structural protein or a fragment thereof from an alphavirus is characterized in that it comprises the removal of at least one initiation codon compared to the native alphavirus sequence.

In a preferred embodiment, the sequence homologous to an open reading frame of a non-structural protein or a fragment thereof from an alphavirus is characterized in that it comprises the removal of at least the native start codon of the open reading frame of a non-structural protein. Preferably, it is characterized in that it comprises the removal of at least the native start codon of the open reading frame encoding nsP1.

The native start codon is the AUG base triplet at which translation on ribosomes in a host cell begins when an RNA is present in a host cell. In other words, the native start codon is the first base triplet that is translated during ribosomal protein synthesis, e.g. in a host cell that has been inoculated with RNA comprising the native start codon. In one embodiment, the host cell is a cell from a eukaryotic species that is a natural host of the specific alphavirus that comprises the native alphavirus 5' replication recognition sequence. In a preferred embodiment, the host cell is a BHK21 cell from the cell line "BHK21 [C13] (ATCC^{®} CCL10^{™})", available from American Type Culture Collection, Manassas, Virginia, USA.

The genomes of many alphaviruses have been fully sequenced and are publically accessible, and the sequences of non-structural proteins encoded by these genomes are publically accessible as well. Such sequence information allows to determine the native start codon *in silico.*

In a preferred embodiment, the sequence homologous to an open reading frame of a non-structural protein or a fragment thereof from an alphavirus is characterized in that it comprises the removal of one or more initiation codons other than the native start codon of the open reading frame of a non-structural protein. In a more preferred embodiment, said nucleic acid sequence is additionally characterized by the removal of the native start codon. For example, in addition to the removal of the native start codon, any one or two or three or four or more than four (e.g. five) initiation codons may be removed.

If the replicon is characterized by the removal of the native start codon, and optionally by the removal of one or more initiation codons other than the native start codon, of the open reading frame of a non-structural protein, the sequence homologous to an open reading frame is not itself an open reading frame since it does not serve as a template for translation.

The one or more initiation codon other than the native start codon that is removed, preferably in addition to removal of the native start codon, is preferably selected from an AUG base triplet that has the potential to initiate translation. An AUG base triplet that has the potential to initiate translation may be referred to as "potential initiation codon". Whether a given AUG base triplet has the potential to initiate translation can be determined *in silico* or in a cell-based *in vitro* assay.

In one embodiment, it is determined *in silico* whether a given AUG base triplet has the potential to initiate translation: in that embodiment, the nucleotide sequence is examined, and an AUG base triplet is determined to have the potential to initiate translation if it is part of an AUGG sequence, preferably part of a Kozak sequence.

In one embodiment, it is determined in a cell-based *in vitro* assay whether a given AUG base triplet has the potential to initiate translation: a RNA replicon characterized by the removal of the native start codon and comprising the given AUG base triplet downstream of the position of the removal of the native start codon is introduced into a host cell. In one embodiment, the host cell is a cell from a eukaryotic species that is a natural host of the specific alphavirus that comprises the native alphavirus 5' replication recognition sequence. In a preferred embodiment, the host cell is a BHK21 cell from the cell line "BHK21 [C13] (ATCC^{®} CCL10^{™})", available from American Type Culture Collection, Manassas, Virginia, USA. It is preferable that no further AUG base triplet is present between the position of the removal of the native start codon and the given AUG base triplet. If, following transfer of the RNA replicon - characterized by the removal of the native start codon and comprising the given AUG base triplet - into the host cell, translation is initiated at the given AUG base triplet, the given AUG base triplet is determined to have the potential to initiate translation. Whether translation is initiated can be determined by any suitable method known in the art. For example, the replicon may encode, downstream of the given AUG base triplet and in-frame with the given AUG base triplet, a tag that facilitates detection of the translation product (if any), e.g. a myc-tag or a HA-tag; whether or not an expression product having the encoded tag is present may be determined e.g. by Western Blot. In this embodiment, it is preferable that no further AUG base triplet is present between the given AUG base triplet and the nucleic acid sequence encoding the tag. The cell-based *in vitro* assay can be performed individually for more than one given AUG base triplet: in each case, it is preferable that no further AUG base triplet is present between the position of the removal of the native start codon and the given AUG base triplet. This can be achieved by removing all AUG base triplets (if any) between the position of the removal of the native start codon and the given AUG base triplet. Thereby, the given AUG base triplet is the first AUG base triplet downstream of the position of the removal of the native start codon.

Preferably, the 5' replication recognition sequence of the RNA replicon according to the present teaching is characterized by the removal of all potential initiation codons. Thus, according to the teaching, the 5' replication recognition sequence preferably does not comprise an open reading frame that can be translated to protein.

In a preferred embodiment, the 5' replication recognition sequence of the RNA replicon according to the teaching is characterized by a secondary structure that is equivalent to the (predicted) secondary structure of the 5' replication recognition sequence of viral genomic RNA. To this end, the RNA replicon may comprise one or more nucleotide changes compensating for nucleotide pairing disruptions within one or more stem loops introduced by the removal of at least one initiation codon.

In a preferred embodiment, the 5' replication recognition sequence of the RNA replicon according to the teaching is characterized by a secondary structure that is equivalent to the secondary structure of the 5' replication recognition sequence of alphaviral genomic RNA. In a preferred embodiment, the 5' replication recognition sequence of the RNA replicon according to the teaching is characterized by a predicted secondary structure that is equivalent to the predicted secondary structure of the 5' replication recognition sequence of alphaviral genomic RNA. According to the present teaching, the secondary structure of an RNA molecule is preferably predicted by the web server for RNA secondary structure prediction http://rna.urmc.rochester.edu/RNAstructureWeb/Servers/Predict1/Predict1.html.

By comparing the secondary structure or predicted secondary structure of a 5' replication recognition sequence of an RNA replicon characterized by the removal of at least one initiation codon compared to the native alphavirus 5' replication recognition sequence, the presence or absence of a nucleotide pairing disruption can be identified. For example, at least one base pair may be absent at a given position, compared to a native alphavirus 5' replication recognition sequence, e.g. a base pair within a stem loop, in particular the stem of the stem loop.

In a preferred embodiment, one or more stem loops of the 5' replication recognition sequence are not deleted or disrupted. More preferably, stem loops 3 and 4 are not deleted or disrupted. More preferably, none of the stem loops of the 5' replication recognition sequence is deleted or disrupted.

In one embodiment, the removal of at least one initiation codon does not disrupt the secondary structure of the 5' replication recognition sequence. In an alternative embodiment, the removal of at least one initiation codon does disrupt the secondary structure of the 5' replication recognition sequence. In this embodiment, the removal of at least one initiation codon may be causative for the absence of at least one base pair at a given position, e.g. a base pair within a stem loop, compared to a native 5' replication recognition sequence. If a base pair is absent within a stem loop, compared to a native 5' replication recognition sequence, the removal of at least one initiation codon is determined to introduce a nucleotide pairing disruption within the stem loop. A base pair within a stem loop is typically a base pair in the stem of the stem loop.

In a preferred embodiment, the RNA replicon comprises one or more nucleotide changes compensating for nucleotide pairing disruptions within one or more stem loops introduced by the removal of at least one initiation codon.

If the removal of at least one initiation codon introduces a nucleotide pairing disruption within a stem loop, compared to a native 5' replication recognition sequence, one or more nucleotide changes may be introduced which are expected to compensate for the nucleotide pairing disruption, and the secondary structure or predicted secondary structure obtained thereby may be compared to a native 5' replication recognition sequence.

Based on the common general knowledge and on the disclosure herein, certain nucleotide changes can be expected by the skilled person to compensate for nucleotide pairing disruptions. For example, if a base pair is disrupted at a given position of the secondary structure or predicted secondary structure of a given 5' replication recognition sequence of an RNA replicon characterized by the removal of at least one initiation codon, compared to the native 5' replication recognition sequence, a nucleotide change that restores a base pair at that position, preferably without re-introducing an initiation codon, is expected to compensate for the nucleotide pairing disruption.

In a preferred embodiment, the 5' replication recognition sequence of the replicon does not overlap with, or does not comprise, a translatable nucleic acid sequence, i.e. translatable into a peptide or protein, in particular a nsP, in particular nsP1, or a fragment of any thereof. For a nucleotide sequence to be "translatable", it requires the presence of an initiation codon; the initiation codon encodes the most N-terminal amino acid residue of the peptide or protein. In one embodiment, the 5' replication recognition sequence of the replicon does not overlap with, or does not comprise, a translatable nucleic acid sequence encoding an N-terminal fragment of nsP1.

In some scenarios, which are described in detail below, the RNA replicon comprises at least one subgenomic promoter. In a preferred embodiment, the subgenomic promoter of the replicon does not overlap with, or does not comprise, a translatable nucleic acid sequence, i.e. translatable into a peptide or protein, in particular a nsP, in particular nsP4, or a fragment of any thereof. In one embodiment, the subgenomic promoter of the replicon does not overlap with, or does not comprise, a translatable nucleic acid sequence that encodes a C-terminal fragment of nsP4. A RNA replicon having a subgenomic promoter that does not overlap with, or does not comprise, a translatable nucleic acid sequence, e.g. translatable into the C-terminal fragment of nsP4, may be generated by deleting part of the coding sequence for nsP4 (typically the part encoding the N-terminal part of nsP4), and/or by removing AUG base triplets in the part of the coding sequence for nsP4 that has not been deleted. If AUG base triplets in the coding sequence for nsP4 or a part thereof are removed, the AUG base triplets that are removed are preferably potential initiation codons. Alternatively, if the subgenomic promoter does not overlap with a nucleic acid sequence that encodes nsP4, the entire nucleic acid sequence encoding nsP4 may be deleted.

In one embodiment, the RNA replicon does not comprise an open reading frame encoding a truncated non-structural protein, e.g., a truncated alphavirus non-structural protein. In the context of this embodiment, it is particularly preferable that the RNA replicon does not comprise an open reading frame encoding the N-terminal fragment of nsP1, and optionally does not comprise an open reading frame encoding the C-terminal fragment of nsP4. The N-terminal fragment of nsP1 is a truncated alphavirus protein; the C-terminal fragment of nsP4 is also a truncated alphavirus protein.

In some embodiments the replicon according to the present teaching does not comprise stem loop 2 (SL2) of the 5' terminus of the genome of an alphavirus. According to Frolov et al., *supra,* stem loop 2 is a conserved secondary structure found at the 5' terminus of the genome of an alphavirus, upstream of CSE 2, but is dispensible for replication.

The RNA replicon according to the present teaching is preferably a single stranded RNA molecule. The RNA replicon according to the present teaching is typically a (+) stranded RNA molecule. In one embodiment, the RNA replicon of the present teaching is an isolated nucleic acid molecule.

### At least one open reading frame comprised by the replicon

In one embodiment, the RNA replicon according to the present teaching comprises at least one open reading frame encoding a peptide of interest or a protein of interest. Preferably, the protein of interest is encoded by a heterologous nucleic acid sequence. The gene encoding the peptide or protein of interest is synonymously termed "gene of interest" or "transgene". In various embodiments, the peptide or protein of interest is encoded by a heterologous nucleic acid sequence. According to the present teaching, the term "heterologous" refers to the fact that a nucleic acid sequence is not naturally functionally or structurally linked to a virus nucleic acid sequence, e.g., an alphavirus nucleic acid sequence.

The replicon according to the present teaching may encode a single polypeptide or multiple polypeptides. Multiple polypeptides can be encoded as a single polypeptide (fusion polypeptide) or as separate polypeptides. In some embodiments, the replicon according to the present teaching may comprise more than one open reading frames, each of which may independently be selected to be under the control of a subgenomic promoter or not. Alternatively, a poly-protein or fusion polypeptide comprises individual polypeptides separated by a 2A self-cleaving peptides (e.g. from foot-and-mouth disease virus 2A protein), or protease cleavage site or an intein.

Proteins of interest may e.g. be selected from the group consisting of reporter proteins, pharmaceutically active peptides or proteins, inhibitors of intracellular interferon (IFN) signaling. According to the teaching, a protein of interest preferably does not include functional non-structural protein from a self-replicating virus, e.g., functional alphavirus non-structural protein.

### Reporter protein

In one embodiment, an open reading frame encodes a reporter protein. In that embodiment, the open reading frame comprises a reporter gene. Certain genes may be chosen as reporters because the characteristics they confer on cells or organisms expressing them may be readily identified and measured, or because they are selectable markers. Reporter genes are often used as an indication of whether a certain gene has been taken up by or expressed in the cell or organism population. Preferably, the expression product of the reporter gene is visually detectable. Common visually detectable reporter proteins typically possess fluorescent or luminescent proteins. Examples of specific reporter genes include the gene that encodes jellyfish green fluorescent protein (GFP), which causes cells that express it to glow green under blue light, the enzyme luciferase, which catalyzes a reaction with luciferin to produce light, and the red fluorescent protein (RFP). Variants of any of these specific reporter genes are possible, as long as the variants possess visually detectable properties. For example, eGFP is a point mutant variant of GFP. The reporter protein embodiment is particularly suitable for testing expression.

### Pharmaceutically active peptide or protein

According to the teaching, in one embodiment, RNA of the replicon comprises or consists of pharmaceutically active RNA. A "pharmaceutically active RNA" may be RNA that encodes a pharmaceutically active peptide or protein. Preferably, the RNA replicon according to the present teaching encodes a pharmaceutically active peptide or protein. Preferably, an open reading frame encodes a pharmaceutically active peptide or protein. Preferably, the RNA replicon comprises an open reading frame that encodes a pharmaceutically active peptide or protein, optionally under control of the subgenomic promoter.

A "pharmaceutically active peptide or protein" has a positive or advantageous effect on the condition or disease state of a subject when administered to the subject in a therapeutically effective amount. Preferably, a pharmaceutically active peptide or protein has curative or palliative properties and may be administered to ameliorate, relieve, alleviate, reverse, delay onset of or lessen the severity of one or more symptoms of a disease or disorder. A pharmaceutically active peptide or protein may have prophylactic properties and may be used to delay the onset of a disease or to lessen the severity of such disease or pathological condition. The term "pharmaceutically active peptide or protein" includes entire proteins or polypeptides, and can also refer to pharmaceutically active fragments thereof. It can also include pharmaceutically active analogs of a peptide or protein. The term "pharmaceutically active peptide or protein" includes peptides and proteins that are antigens, i.e., the peptide or protein elicits an immune response in a subject which may be therapeutic or partially or fully protective.

In one embodiment, the pharmaceutically active peptide or protein is or comprises an immunologically active compound or an antigen or an epitope.

According to the teaching, the term "immunologically active compound" relates to any compound altering an immune response, preferably by inducing and/or suppressing maturation of immune cells, inducing and/or suppressing cytokine biosynthesis, and/or altering humoral immunity by stimulating antibody production by B cells. In one embodiment, the immune response involves stimulation of an antibody response (usually including immunoglobulin G (IgG)). Immunologically active compounds possess potent immunostimulating activity including, but not limited to, antiviral and antitumor activity, and can also down-regulate other aspects of the immune response, for example shifting the immune response away from a TH₂ immune response, which is useful for treating a wide range of TH₂ mediated diseases.

According to the teaching, the term "antigen" or "immunogen" covers any substance that will elicit an immune response. In particular, an "antigen" relates to any substance that reacts specifically with antibodies or T-lymphocytes (T-cells). According to the present teaching, the term "antigen" comprises any molecule which comprises at least one epitope. Preferably, an antigen in the context of the present teaching is a molecule which, optionally after processing, induces an immune reaction, which is preferably specific for the antigen. According to the present teaching, any suitable antigen may be used, which is a candidate for an immune reaction, wherein the immune reaction may be both a humoral as well as a cellular immune reaction. In the context of the embodiments of the present teaching, the antigen is preferably presented by a cell, preferably by an antigen presenting cell, in the context of MHC molecules, which results in an immune reaction against the antigen. An antigen is preferably a product which corresponds to or is derived from a naturally occurring antigen. Such naturally occurring antigens may include or may be derived from allergens, viruses, bacteria, fungi, parasites and other infectious agents and pathogens or an antigen may also be a tumor antigen. According to the present teaching, an antigen may correspond to a naturally occurring product, for example, a viral protein, or a part thereof. In preferred embodiments, the antigen is a surface polypeptide, i.e. a polypeptide naturally displayed on the surface of a cell, a pathogen, a bacterium, a virus, a fungus, a parasite, an allergen, or a tumor. The antigen may elicit an immune response against a cell, a pathogen, a bacterium, a virus, a fungus, a parasite, an allergen, or a tumor.

The term "pathogen" refers to pathogenic biological material capable of causing disease in an organism, preferably a vertebrate organism. Pathogens include microorganisms such as bacteria, unicellular eukaryotic organisms (protozoa), fungi, as well as viruses.

The terms "epitope", "antigen peptide", "antigen epitope", "immunogenic peptide" and "MHC binding peptide" are used interchangeably herein and refer to an antigenic determinant in a molecule such as an antigen, i.e., to a part in or fragment of an immunologically active compound that is recognized by the immune system, for example, that is recognized by a T cell, in particular when presented in the context of MHC molecules. An epitope of a protein preferably comprises a continuous or discontinuous portion of said protein and is preferably between 5 and 100, preferably between 5 and 50, more preferably between 8 and 30, most preferably between 10 and 25 amino acids in length, for example, the epitope may be preferably 9, 10, 11, 12, 13, 14, 15, 16, 17, 18, 19, 20, 21, 22, 23, 24, or 25 amino acids in length. According to the teaching an epitope may bind to MHC molecules such as MHC molecules on the surface of a cell and thus, may be a "MHC binding peptide" or "antigen peptide". The term "major histocompatibility complex" and the abbreviation "MHC" include MHC class I and MHC class II molecules and relate to a complex of genes which is present in all vertebrates. MHC proteins or molecules are important for signaling between lymphocytes and antigen presenting cells or diseased cells in immune reactions, wherein the MHC proteins or molecules bind peptides and present them for recognition by T cell receptors. The proteins encoded by the MHC are expressed on the surface of cells, and display both self-antigens (peptide fragments from the cell itself) and non-self-antigens (e.g., fragments of invading microorganisms) to a T cell. Preferred such immunogenic portions bind to an MHC class I or class II molecule. As used herein, an immunogenic portion is said to "bind to" an MHC class I or class II molecule if such binding is detectable using any assay known in the art. The term "MHC binding peptide" relates to a peptide which binds to an MHC class I and/or an MHC class II molecule. In the case of class I MHC/peptide complexes, the binding peptides are typically 8-10 amino acids long although longer or shorter peptides may be effective. In the case of class II MHC/peptide complexes, the binding peptides are typically 10-25 amino acids long and are in particular 13-18 amino acids long, whereas longer and shorter peptides may be effective.

In one embodiment, the protein of interest according to the present teaching comprises an epitope suitable for vaccination of a target organism. A person skilled in the art will know that one of the principles of immunobiology and vaccination is based on the fact that an immunoprotective reaction to a disease is produced by immunizing an organism with an antigen, which is immunologically relevant with respect to the disease to be treated. According to the present teaching, an antigen is selected from the group comprising a self-antigen and non-self-antigen. A non-self-antigen is preferably a bacterial antigen, a virus antigen, a fungus antigen, an allergen or a parasite antigen. It is preferred that the antigen comprises an epitope that is capable of eliciting an immune response in a target organism. For example, the epitope may elicit an immune response against a bacterium, a virus, a fungus, a parasite, an allergen, or a tumor.

In some embodiments the non-self-antigen is a bacterial antigen. In some embodiments, the antigen elicits an immune response against a bacterium which infects animals, including birds, fish and mammals, including domesticated animals. Preferably, the bacterium against which the immune response is elicited is a pathogenic bacterium.

In some embodiments the non-self-antigen is a virus antigen. A virus antigen may for example be a peptide from a virus surface protein, e.g. a capsid polypeptide or a spike polypeptide. In some embodiments, the antigen elicits an immune response against a virus which infects animals, including birds, fish and mammals, including domesticated animals. Preferably, the virus against which the immune response is elicited is a pathogenic virus.

In some embodiments the non-self-antigen is a polypeptide or a protein from a fungus. In some embodiments, the antigen elicits an immune response against a fungus which infects animals, including birds, fish and mammals, including domesticated animals. Preferably, the fungus against which the immune response is elicited is a pathogenic fungus.

In some embodiments the non-self-antigen is a polypeptide or protein from a unicellular eukaryotic parasite. In some embodiments, the antigen elicits an immune response against a unicellular eukaryotic parasite, preferably a pathogenic unicellular eukaryotic parasite. Pathogenic unicellular eukaryotic parasites may be e.g. from the genus *Plasmodium,* e.g. *P. falciparum, P. vivax, P. malariae* or *P. ovale,* from the genus *Leishmania,* or from the genus *Trypanosoma,* e.g. *T. cruzi* or *T. brucei.*

In some embodiments the non-self-antigen is an allergenic polypeptide or an allergenic protein. An allergenic protein or allergenic polypeptide is suitable for allergen immunotherapy, also known as hypo-sensitization.

In some embodiments the antigen is a self-antigen, particularly a tumor antigen. Tumor antigens and their determination are known to the skilled person.

In the context of the present teaching, the term "tumor antigen" or "tumor-associated antigen" relates to proteins that are under normal conditions specifically expressed in a limited number of tissues and/or organs or in specific developmental stages, for example, the tumor antigen may be under normal conditions specifically expressed in stomach tissue, preferably in the gastric mucosa, in reproductive organs, e.g., in testis, in trophoblastic tissue, e.g., in placenta, or in germ line cells, and are expressed or aberrantly expressed in one or more tumor or cancer tissues. In this context, "a limited number" preferably means not more than 3, more preferably not more than 2. The tumor antigens in the context of the present teaching include, for example, differentiation antigens, preferably cell type specific differentiation antigens, i.e., proteins that are under normal conditions specifically expressed in a certain cell type at a certain differentiation stage, cancer/testis antigens, i.e., proteins that are under normal conditions specifically expressed in testis and sometimes in placenta, and germ line specific antigens. In the context of the present teaching, the tumor antigen is preferably associated with the cell surface of a cancer cell and is preferably not or only rarely expressed in normal tissues. Preferably, the tumor antigen or the aberrant expression of the tumor antigen identifies cancer cells. In the context of the present teaching, the tumor antigen that is expressed by a cancer cell in a subject, e.g., a patient suffering from a cancer disease, is preferably a self-protein in said subject. In preferred embodiments, the tumor antigen in the context of the present teaching is expressed under normal conditions specifically in a tissue or organ that is non-essential, i.e., tissues or organs which when damaged by the immune system do not lead to death of the subject, or in organs or structures of the body which are not or only hardly accessible by the immune system. Preferably, the amino acid sequence of the tumor antigen is identical between the tumor antigen which is expressed in normal tissues and the tumor antigen which is expressed in cancer tissues.

Examples for tumor antigens that may be useful in the present teaching are p53, ART-4, BAGE, beta-catenin/m, Bcr-abL CAMEL, CAP-1, CASP-8, CDC27/m, CDK4/m, CEA, the cell surface proteins of the claudin family, such as CLAUDIN-6, CLAUDIN-18.2 and CLAUDIN-12, c-MYC, CT, Cyp-B, DAM, ELF2M, ETV6-AML1, G250, GAGE, GnT-V, Gap100, HAGE, HER-2/neu, HPV-E7, HPV-E6, HAST-2, hTERT (or hTRT), LAGE, LDLR/FUT, MAGE-A, preferably MAGE-A1, MAGE-A2, MAGE-A3, MAGE-A4, MAGE-A5, MAGE-A6, MAGE-A7, MAGE-A8, MAGE-A9, MAGE-A10, MAGE-A11, or MAGE-A12, MAGE-B, MAGE-C, MART-1/Melan-A, MC1R, Myosin/m, MUC1, MUM-1, -2, -3, NA88-A, NF1, NY-ESO-1, NY-BR-1, p190 minor BCR-abL, Pm1/RARa, PRAME, proteinase 3, PSA, PSM, RAGE, RU1 or RU2, SAGE, SART-1 or SART-3, SCGB3A2, SCP1, SCP2, SCP3, SSX, SURVIVIN, TEL/AML1, TPI/m, TRP-1, TRP-2, TRP-2/INT2, TPTE and WT. Particularly preferred tumor antigens include CLAUDIN-18.2 (CLDN18.2) and CLAUDIN-6 (CLDN6).

In some embodiments, it is not required that the pharmaceutically active peptide or protein is an antigen that elicits an immune response. Suitable pharmaceutically active proteins or peptides may be selected from the group consisting of cytokines and immune system proteins such as immunologically active compounds (e.g., interleukins, colony stimulating factor (CSF), granulocyte colony stimulating factor (G-CSF), granulocyte-macrophage colony stimulating factor (GM-CSF), erythropoietin, tumor necrosis factor (TNF), interferons, integrins, addressins, seletins, homing receptors, T cell receptors, chimeric antigen receptors (CARs), immunoglobulins), hormones (insulin, thyroid hormone, catecholamines, gonadotrophines, trophic hormones, prolactin, oxytocin, dopamine, bovine somatotropin, leptins and the like), growth hormones (e.g., human grown hormone), growth factors (e.g., epidermal growth factor, nerve growth factor, insulin-like growth factor and the like), growth factor receptors, enzymes (tissue plasminogen activator, streptokinase, cholesterol biosynthetic or degradative, steriodogenic enzymes, kinases, phosphodiesterases, methylases, de-methylases, dehydrogenases, cellulases, proteases, lipases, phospholipases, aromatases, cytochromes, adenylate or guanylaste cyclases, neuramidases and the like), receptors (steroid hormone receptors, peptide receptors), binding proteins (growth hormone or growth factor binding proteins and the like), transcription and translation factors, tumor growth suppressing proteins (e.g., proteins which inhibit angiogenesis), structural proteins (such as collagen, fibroin, fibrinogen, elastin, tubulin, actin, and myosin), blood proteins (thrombin, serum albumin, Factor VII, Factor VIII, insulin, Factor IX, Factor X, tissue plasminogen activator, protein C, von Wilebrand factor, antithrombin III, glucocerebrosidase, erythropoietin granulocyte colony stimulating factor (GCSF) or modified Factor VIII, anticoagulants and the like. In one embodiment, the pharmaceutically active protein according to the teaching is a cytokine which is involved in regulating lymphoid homeostasis, preferably a cytokine which is involved in and preferably induces or enhances development, priming, expansion, differentiation and/or survival of T cells. In one embodiment, the cytokine is an interleukin, e.g. IL-2, IL-7, IL-12, IL-15, or IL-21.

### Inhibitor of interferon (IFN) signaling

A further suitable protein of interest encoded by an open reading frame is an inhibitor of interferon (IFN) signaling. While it has been reported that viability of cells in which RNA has been introduced for expression can be reduced, in particular, if cells are transfected multiple times with RNA, IFN inhibiting agents were found to enhance the viability of cells in which RNA is to be expressed (WO 2014/071963 A1). Preferably, the inhibitor is an inhibitor of IFN type I signaling. Preventing engagement of IFN receptor by extracellular IFN and inhibiting intracellular IFN signaling in the cells allows stable expression of RNA in the cells. Alternatively or additionally, preventing engagement of IFN receptor by extracellular IFN and inhibiting intracellular IFN signaling enhances survival of the cells, in particular, if cells are transfected repetitively with RNA. Without wishing to be bound by theory, it is envisaged that intracellular IFN signalling can result in inhibition of translation and/or RNA degradation. This can be addressed by inhibiting one or more IFN-inducible antivirally active effector proteins. The IFN-inducible antivirally active effector protein can be selected from the group consisting of RNA-dependent protein kinase (PKR), 2',5'-oligoadenylate synthetase (OAS) and RNaseL. Inhibiting intracellular IFN signalling may comprise inhibiting the PKR-dependent pathway and/or the OAS-dependent pathway. A suitable protein of interest is a protein that is capable of inhibiting the PKR-dependent pathway and/or the OAS-dependent pathway. Inhibiting the PKR-dependent pathway may comprise inhibiting elF2-alpha phosphorylation. Inhibiting PKR may comprise treating the cell with at least one PKR inhibitor. The PKR inhibitor may be a viral inhibitor of PKR. The preferred viral inhibitor of PKR is vaccinia virus E3. If a peptide or protein (e.g. E3, K3) is to inhibit intracellular IFN signaling, intracellular expression of the peptide or protein is preferred. Vaccinia virus E3 is a 25 kDa dsRNA-binding protein (encoded by gene E3L) that binds and sequesters dsRNA to prevent the activation of PKR and OAS. E3 can bind directly to PKR and inhibits its activity, resulting in reduced phosphorylation of elF2-alpha. Other suitable inhibitors of IFN signaling are Herpes simplex virus ICP34.5, Toscana virus NSs, *Bombyx mori* nucleopolyhedrovirus PK2, and HCV NS34A.

### Position of the at least one open reading frame in the RNA replicon

The RNA replicon is suitable for expression of one or more genes encoding a peptide of interest or a protein of interest, optionally under control of a subgenomic promoter. Various embodiments are possible. One or more open reading frames, each encoding a peptide of interest or a protein of interest, can be present on the RNA replicon. The most upstream open reading frame of the RNA replicon is referred to as "first open reading frame". In one embodiment, the first open reading frame encoding a protein of interest is located downstream from the 5' replication recognition sequence and upstream from the IRES (and the open reading frame encoding a functional non-structural protein from a self-replicating virus). In some embodiments, the "first open reading frame" is the only open reading frame of the RNA replicon. Optionally, one or more further open reading frames can be present downstream of the first open reading frame. One or more further open reading frames downstream of the first open reading frame may be referred to as "second open reading frame", "third open reading frame" and so on, in the order (5' to 3') in which they are present downstream of the first open reading frame. In one embodiment, one or more further open reading frames encoding one or more proteins of interest are located downstream from the open reading frame encoding a functional non-structural protein from a self-replicating virus and are preferably controlled by subgenomic promotors. Preferably, each open reading frame comprises a start codon (base triplet), typically AUG (in the RNA molecule), corresponding to ATG (in a respective DNA molecule).

If the replicon comprises a 3' replication recognition sequence, it is preferred that all open reading frames are localized upstream of the 3' replication recognition sequence.

In some embodiments, at least one open reading frame of the replicon is under the control of a subgenomic promoter, preferably an alphavirus subgenomic promoter. The alphavirus subgenomic promoter is very efficient, and is therefore suitable for heterologous gene expression at high levels. Preferably, the subgenomic promoter is a promoter for a subgenomic transcript in an alphavirus. This means that the subgenomic promoter is one which is native to an alphavirus and which preferably controls transcription of the open reading frame encoding one or more structural proteins in said alphavirus. Alternatively, the subgenomic promoter is a variant of a subgenomic promoter of an alphavirus; any variant which functions as promoter for subgenomic RNA transcription in a host cell is suitable. If the replicon comprises a subgenomic promoter, it is preferred that the replicon comprises a conserved sequence element 3 (CSE 3) or a variant thereof.

Preferably, the at least one open reading frame under control of a subgenomic promoter is localized downstream of the subgenomic promoter. Preferably, the subgenomic promoter controls production of subgenomic RNA comprising a transcript of the open reading frame

In some embodiments the first open reading frame is under control of a subgenomic promoter. In one embodiment, when the first open reading frame is under control of the subgenomic promoter, the gene encoded by the first open reading frame can be expressed both from the replicon as well as from a subgenomic transcript thereof (the latter in the presence of functional alphavirus non-structural protein). One or more further open reading frames, each under control of a subgenomic promoter, may be present downstream of the first open reading frame that may be under control of a subgenomic promoter. The genes encoded by the one or more further open reading frames, e.g. by the second open reading frame, may be translated from one or more subgenomic transcripts, each under control of a subgenomic promoter. For example, the RNA replicon may comprise a subgenomic promoter controlling production of a transcript that encodes a second protein of interest.

In other embodiments the first open reading frame is not under control of a subgenomic promoter. In one embodiment, when the first open reading frame is not under control of a subgenomic promoter, the gene encoded by the first open reading frame can be expressed from the replicon. One or more further open reading frames, each under control of a subgenomic promoter, may be present downstream of the first open reading frame. The genes encoded by the one or more further open reading frames may be expressed from subgenomic transcripts.

In a cell which comprises the replicon according to the present teaching, the replicon may be amplified by functional non-structural protein. Additionally, if the replicon comprises one or more open reading frames under control of a subgenomic promoter, one or more subgenomic transcripts are expected to be prepared by functional non-structural protein.

If a replicon comprises more than one open reading frame encoding a protein of interest, it is preferable that each open reading frame encodes a different protein. For example, the protein encoded by the second open reading frame is different from the protein encoded by the first open reading frame.

### Preferred features of RNA molecules

RNA molecules according to the teaching may optionally be characterized by further features, e.g. by a 5'-cap, a 5'-UTR, a 3'-UTR, a poly(A) sequence, and/or adaptation of the codon usage. Details are described in the following.

### Cap

In some embodiments, the replicon according to the present teaching comprises a 5'-cap.

The terms "5'-cap", "cap", "5'-cap structure", "cap structure" are used synonymously to refer to a dinucleotide that is found on the 5' end of some eukaryotic primary transcripts such as precursor messenger RNA. A 5'-cap is a structure wherein a (optionally modified) guanosine is bonded to the first nucleotide of an mRNA molecule via a 5' to 5' triphosphate linkage (or modified triphosphate linkage in the case of certain cap analogs). The terms can refer to a conventional cap or to a cap analog.

"RNA which comprises a 5'-cap" or "RNA which is provided with a 5'-cap" or "RNA which is modified with a 5'-cap" or "capped RNA" refers to RNA which comprises a 5'-cap. For example, providing an RNA with a 5'-cap may be achieved by in vitro transcription of a DNA template in presence of said 5'-cap, wherein said 5'-cap is co-transcriptionally incorporated into the generated RNA strand, or the RNA may be generated, for example, by *in vitro* transcription, and the 5'-cap may be attached to the RNA post-transcriptionally using capping enzymes, for example, capping enzymes of vaccinia virus. In capped RNA, the 3' position of the first base of a (capped) RNA molecule is linked to the 5' position of the subsequent base of the RNA molecule ("second base") via a phosphodiester bond.

In one embodiment, the RNA replicon comprises a 5'-cap. In one embodiment, the RNA replicon does not comprise a 5'-cap.

The term "conventional 5'-cap" refers to a naturally occurring 5'-cap, preferably to the 7-methylguanosine cap. In the 7-methylguanosine cap, the guanosine of the cap is a modified guanosine wherein the modification consists of a methylation at the 7-position.

In the context of the present teaching, the term "5'-cap analog" refers to a molecular structure that resembles a conventional 5'-cap, but is modified to possess the ability to stabilize RNA if attached thereto, preferably *in vivo* and/or in a cell. A cap analog is not a conventional 5'-cap.

For the case of eukaryotic mRNA, the 5'-cap has been generally described to be involved in efficient translation of mRNA: in general, in eukaryotes, translation is initiated only at the 5' end of a messenger RNA (mRNA) molecule, unless an internal ribosomal entry site (IRES) is present. Eukaryotic cells are capable of providing an RNA with a 5'-cap during transcription in the nucleus: newly synthesized mRNAs are usually modified with a 5'-cap structure, e.g. when the transcript reaches a length of 20 to 30 nucleotides. First, the 5' terminal nucleotide pppN (ppp representing triphosphate; N representing any nucleoside) is converted in the cell to 5' GpppN by a capping enzyme having RNA 5'-triphosphatase and guanylyltransferase activities. The GpppN may subsequently be methylated in the cell by a second enzyme with (guanine-7)-methyltransferase activity to form the mono-methylated m⁷GpppN cap. In one embodiment, the 5'-cap used in the present teaching is a natural 5'-cap.

In the present teaching, a natural 5'-cap dinucleotide is typically selected from the group consisting of a non-methylated cap dinucleotide (G(5')ppp(5')N; also termed GpppN) and a methylated cap dinucleotide ((m⁷G(5')ppp(5')N; also termed m⁷GpppN). m⁷GpppN (wherein N is G) is represented by the following formula:

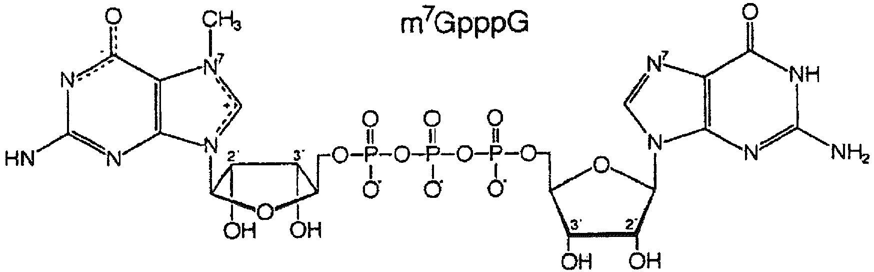

Capped RNA of the present teaching can be prepared *in vitro,* and therefore, does not depend on a capping machinery in a host cell. The most frequently used method to make capped RNAs *in vitro* is to transcribe a DNA template with either a bacterial or bacteriophage RNA polymerase in the presence of all four ribonucleoside triphosphates and a cap dinucleotide such as m⁷G(5')ppp(5')G (also called m⁷GpppG). The RNA polymerase initiates transcription with a nucleophilic attack by the 3'-OH of the guanosine moiety of m⁷GpppG on the α-phosphate of the next templated nucleoside triphosphate (pppN), resulting in the intermediate m⁷GpppGpN (wherein N is the second base of the RNA molecule). The formation of the competing GTP-initiated product pppGpN is suppressed by setting the molar ratio of cap to GTP between 5 and 10 during *in vitro* transcription.

In preferred embodiments of the present teaching, the 5'-cap (if present) is a 5'-cap analog. These embodiments are particularly suitable if the RNA is obtained by *in vitro* transcription, e.g. is an *in vitro* transcribed RNA (IVT-RNA). Cap analogs have been initially described to facilitate large scale synthesis of RNA transcripts by means of *in vitro* transcription.

For messenger RNA, some cap analogs (synthetic caps) have been generally described to date, and they can all be used in the context of the present teaching. Ideally, a cap analog is selected that is associated with higher translation efficiency and/or increased resistance to *in vivo* degradation and/or increased resistance to *in vitro* degradation.

Preferably, a cap analog is used that can only be incorporated into an RNA chain in one orientation. Pasquinelli et al. (1995, RNA J., vol., 1, pp. 957-967) demonstrated that during *in vitro* transcription, bacteriophage RNA polymerases use the 7-methylguanosine unit for initiation of transcription, whereby around 40-50% of the transcripts with cap possess the cap dinucleotide in a reverse orientation (i.e., the initial reaction product is Gpppm⁷GpN). Compared to the RNAs with a correct cap, RNAs with a reverse cap are not functional with respect to translation of a nucleic acid sequence into protein. Thus, it is desirable to incorporate the cap in the correct orientation, i.e., resulting in an RNA with a structure essentially corresponding to m⁷GpppGpN etc. It has been shown that the reverse integration of the cap-dinucleotide is inhibited by the substitution of either the 2'- or the 3'-OH group of the methylated guanosine unit (Stepinski et al., 2001; RNA J., vol. 7, pp. 1486-1495; Peng et al., 2002; Org. Lett., vol. 24, pp. 161-164). RNAs which are synthesized in presence of such "anti reverse cap analogs" are translated more efficiently than RNAs which are *in vitro* transcribed in presence of the conventional 5'-cap m⁷GpppG. To that end, one cap analog in which the 3' OH group of the methylated guanosine unit is replaced by OCH₃ is described e.g. by Holtkamp et al., 2006, Blood, vol. 108, pp. 4009-4017 (7-methyl(3'-O-methyl)GpppG; anti-reverse cap analog (ARCA)). ARCA is a suitable cap dinucleotide according to the present teaching.

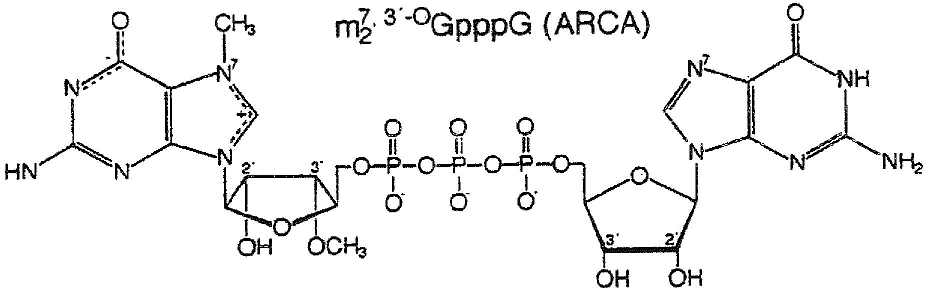

In a preferred embodiment of the present teaching, the RNA of the present teaching is essentially not susceptible to decapping. This is important because, in general, the amount of protein produced from synthetic mRNAs introduced into cultured mammalian cells is limited by the natural degradation of mRNA. One *in vivo* pathway for mRNA degradation begins with the removal of the mRNA cap. This removal is catalyzed by a heterodimeric pyrophosphatase, which contains a regulatory subunit (Dcp1) and a catalytic subunit (Dcp2). The catalytic subunit cleaves between the α and β phosphate groups of the triphosphate bridge. In the present teaching, a cap analog may be selected or present that is not susceptible, or less susceptible, to that type of cleavage. A suitable cap analog for this purpose may be selected from a cap dinucleotide according to formula (I): 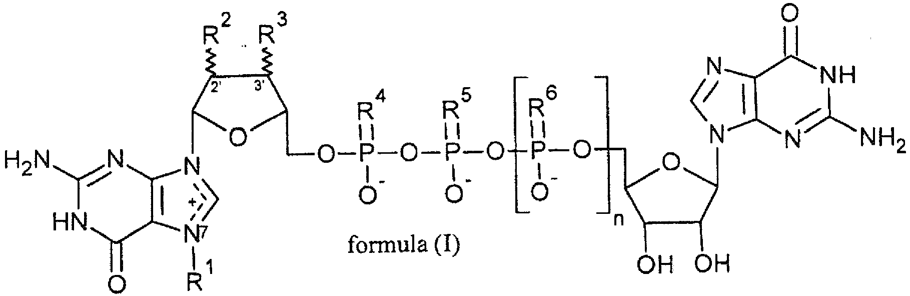
wherein R¹ is selected from the group consisting of optionally substituted alkyl, optionally substituted alkenyl, optionally substituted alkynyl, optionally substituted cycloalkyl, optionally substituted heterocyclyl, optionally substituted aryl, and optionally substituted heteroaryl,
R² and R³ are independently selected from the group consisting of H, halo, OH, and optionally substituted alkoxy, or R² and R³ together form O-X-O, wherein X is selected from the group consisting of optionally substituted CH₂, CH₂CH₂, CH₂CH₂CH₂, CH₂CH(CH₃), and
C(CH₃)₂, or R² is combined with the hydrogen atom at position 4' of the ring to which R² is attached to form -O-CH₂- or -CH₂-O-,
R⁵ is selected from the group consisting of S, Se, and BH₃,
R⁴ and R⁶ are independently selected from the group consisting of O, S, Se, and BH₃. n is 1, 2, or 3.

Preferred embodiments for R¹, R², R3, R⁴, R⁵, R⁶ are disclosed in WO 2011/015347 A1 and may be selected accordingly in the present teaching.

For example, in a preferred embodiment of the present teaching, the RNA of the present teaching comprises a phosphorothioate-cap-analog. Phosphorothioate-cap-analogs are specific cap analogs in which one of the three non-bridging O atoms in the triphosphate chain is replaced with an S atom, i.e. one of R⁴, R⁵ or R⁶ in Formula (I) is S. Phosphorothioate-cap-analogs have been described by J. Kowalska et al., 2008, RNA, vol. 14, pp. 1119-1131, as a solution to the undesired decapping process, and thus to increase the stability of RNA *in vivo.* In particular, the substitution of an oxygen atom for a sulphur atom at the beta-phosphate group of the 5'-cap results in stabilization against Dcp2. In that embodiment, which is preferred in the present teaching, R⁵ in Formula (I) is S; and R⁴ and R⁶ are O.

In a further preferred embodiment of the present teaching, the RNA of the present teaching comprises a phosphorothioate-cap-analog wherein the phosphorothioate modification of the RNA 5'-cap is combined with an "anti-reverse cap analog" (ARCA) modification. Respective ARCA-phosphorothioate-cap-analogs are described in WO 2008/157688 A2, and they can all be used in the RNA of the present teaching. In that embodiment, at least one of R² or R³ in Formula (I) is not OH, preferably one among R² and R³ is methoxy (OCH₃), and the other one among R² and R³ is preferably OH. In a preferred embodiment, an oxygen atom is substituted for a sulphur atom at the beta-phosphate group (so that R⁵ in Formula (I) is S; and R⁴ and R⁶ are O). It is believed that the phosphorothioate modification of the ARCA ensures that the α, β, and γ phosphorothioate groups are precisely positioned within the active sites of cap-binding proteins in both the translational and decapping machinery. At least some of these analogs are essentially resistant to pyrophosphatase Dcp1/Dcp2. Phosphorothioate-modified ARCAs were described to have a much higher affinity for elF4E than the corresponding ARCAs lacking a phosphorothioate group.

A respective cap analog that is particularly preferred in the present teaching, i.e., m_{2'}^{7,2'-O}GppₛpG, is termed beta-S-ARCA (WO 2008/157688 A2; Kuhn et al., Gene Ther., 2010, vol. 17, pp. 961-971). Thus, in one embodiment of the present teaching, the RNA of the present teaching is modified with beta-S-ARCA. beta-S-ARCA is represented by the following structure:

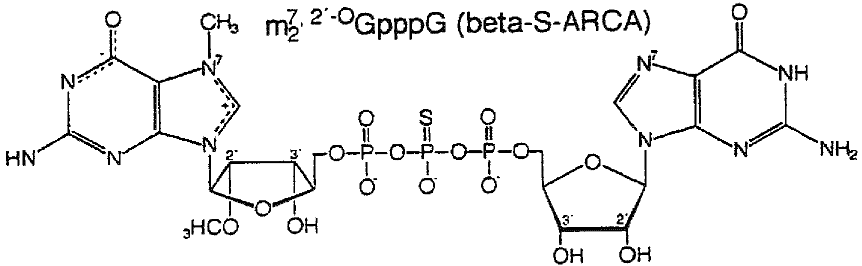

In general, the replacement of an oxygen atom for a sulphur atom at a bridging phosphate results in phosphorothioate diastereomers which are designated D1 and D2, based on their elution pattern in HPLC. Briefly, the D1 diastereomer of beta-S-ARCA" or "beta-S-ARCA(D1)" is the diastereomer of beta-S-ARCA which elutes first on an HPLC column compared to the D2 diastereomer of beta-S-ARCA (beta-S-ARCA(D2)) and thus exhibits a shorter retention time. Determination of the stereochemical configuration by HPLC is described in WO 2011/015347 A1.

In a first particularly preferred embodiment of the present teaching, RNA of the present teaching is modified with the beta-S-ARCA(D2) diastereomer. The two diastereomers of beta-S-ARCA differ in sensitivity against nucleases. It has been shown that RNA carrying the D2 diastereomer of beta-S-ARCA is almost fully resistant against Dcp2 cleavage (only 6% cleavage compared to RNA which has been synthesized in presence of the unmodified ARCA 5'-cap), whereas RNA with the beta-S-ARCA(D1) 5'-cap exhibits an intermediary sensitivity to Dcp2 cleavage (71% cleavage). It has further been shown that the increased stability against Dcp2 cleavage correlates with increased protein expression in mammalian cells. In particular, it has been shown that RNAs carrying the beta-S-ARCA(D2) cap are more efficiently translated in mammalian cells than RNAs carrying the beta-S-ARCA(D1) cap. Therefore, in one embodiment of the present teaching, RNA of the present teaching is modified with a cap analog according to Formula (I), characterized by a stereochemical configuration at the P atom comprising the substituent R⁵ in Formula (I) that corresponds to that at the P_{β} atom of the D2 diastereomer of beta-S-ARCA. In that embodiment, R⁵ in Formula (I) is S; and R⁴ and R⁶ are O. Additionally, at least one of R² or R³ in Formula (I) is preferably not OH, preferably one among R² and R³ is methoxy (OCH3), and the other one among R² and R³ is preferably OH.

In a second particularly preferred embodiment, RNA of the present teaching is modified with the beta-S-ARCA(D1) diastereomer. This embodiment is particularly suitable for transfer of capped RNA into immature antigen presenting cells, such as for vaccination purposes. It has been demonstrated that the beta-S-ARCA(D1) diastereomer, upon transfer of respectively capped RNA into immature antigen presenting cells, is particularly suitable for increasing the stability of the RNA, increasing translation efficiency of the RNA, prolonging translation of the RNA, increasing total protein expression of the RNA, and/or increasing the immune response against an antigen or antigen peptide encoded by said RNA (Kuhn et al., 2010, Gene Ther., vol. 17, pp. 961-971). Therefore, in an alternative embodiment of the present teaching, RNA of the present teaching is modified with a cap analog according to Formula (I), characterized by a stereochemical configuration at the P atom comprising the substituent R⁵ in Formula (I) that corresponds to that at the P_{β} atom of the D1 diastereomer of beta-S-ARCA. Respective cap analogs and embodiments thereof are described in WO 2011/015347 A1 and Kuhn et al., 2010, Gene Ther., vol. 17, pp. 961-971. Any cap analog described in WO 2011/015347 A1, wherein the stereochemical configuration at the P atom comprising the substituent R⁵ corresponds to that at the P_{β} atom of the D1 diastereomer of beta-S-ARCA, may be used in the present teaching. Preferably, R⁵ in Formula (I) is S; and R⁴ and R⁶ are O. Additionally, at least one of R² or R³ in Formula (I) is preferably not OH, preferably one among R² and R³ is methoxy (OCH3), and the other one among R² and R³ is preferably OH.

In one embodiment, RNA of the present teaching is modified with a 5'-cap structure according to Formula (I) wherein any one phosphate group is replaced by a boranophosphate group or a phosphoroselenoate group. Such caps have increased stability both *in vitro* and *in vivo.* Optionally, the respective compound has a 2'-O- or 3'-O-alkyl group (wherein alkyl is preferably methyl); respective cap analogs are termed BH₃-ARCAs or Se-ARCAs. Compounds that are particularly suitable for capping of mRNA include the β-BH₃-ARCAs and β-Se-ARCAs, as described in WO 2009/149253 A2. For these compounds, a stereochemical configuration at the P atom comprising the substituent R⁵ in Formula (I) that corresponds to that at the P_{β} atom of the D1 diastereomer of beta-S-ARCA is preferred.

### UTR

The term "untranslated region" or "UTR" relates to a region in a DNA molecule which is transcribed but is not translated into an amino acid sequence, or to the corresponding region in an RNA molecule, such as an mRNA molecule. An untranslated region (UTR) can be present 5' (upstream) of an open reading frame (5'-UTR) and/or 3' (downstream) of an open reading frame (3'-UTR).

A 3'-UTR, if present, is located at the 3' end of a gene, downstream of the termination codon of a protein-encoding region, but the term "3'-UTR" does preferably not include the poly(A) tail. Thus, the 3'-UTR is upstream of the poly(A) tail (if present), e.g. directly adjacent to the poly(A) tail.

A 5'-UTR, if present, is located at the 5' end of a gene, upstream of the start codon of a protein-encoding region. A 5'-UTR is downstream of the 5'-cap (if present), e.g. directly adjacent to the 5'-cap.

5'- and/or 3'-untranslated regions may, according to the teaching, be functionally linked to an open reading frame, so as for these regions to be associated with the open reading frame in such a way that the stability and/or translation efficiency of the RNA comprising said open reading frame are increased.

In some embodiments, the RNA replicon according to the present teaching comprises a 5'-UTR and/or a 3'-UTR.

UTRs are implicated in stability and translation efficiency of RNA. Both can be improved, besides structural modifications concerning the 5'-cap and/or the 3' poly(A)-tail as described herein, by selecting specific 5' and/or 3' untranslated regions (UTRs). Sequence elements within the UTRs are generally understood to influence translational efficiency (mainly 5'-UTR) and RNA stability (mainly 3'-UTR). It is preferable that a 5'-UTR is present that is active in order to increase the translation efficiency and/or stability of the RNA replicon. Independently or additionally, it is preferable that a 3'-UTR is present that is active in order to increase the translation efficiency and/or stability of the RNA replicon.

The terms "active in order to increase the translation efficiency" and/or "active in order to increase the stability", with reference to a first nucleic acid sequence (e.g. a UTR), means that the first nucleic acid sequence is capable of modifying, in a common transcript with a second nucleic acid sequence, the translation efficiency and/or stability of said second nucleic acid sequence in such a way that said translation efficiency and/or stability is increased in comparison with the translation efficiency and/or stability of said second nucleic acid sequence in the absence of said first nucleic acid sequence..

In one embodiment, the RNA replicon according to the present teaching comprises a 5'-UTR and/or a 3'-UTR which is heterologous or non-native to the alphavirus from which the functional alphavirus non-structural protein is derived. This allows the untranslated regions to be designed according to the desired translation efficiency and RNA stability. Thus, heterologous or non-native UTRs allow for a high degree of flexibility, and this flexibility is advantageous compared to native alphaviral UTRs.

Preferably, the RNA replicon according to the present teaching comprises a 5'-UTR and/or a 3'-UTR that is not of virus origin; particularly not of alphavirus origin. In one embodiment, the RNA replicon comprises a 5'-UTR derived from a eukaryotic 5'-UTR and/or a 3'-UTR derived from a eukaryotic 3'-UTR.

A 5'-UTR according to the present teaching can comprise any combination of more than one nucleic acid sequence, optionally separated by a linker. A 3'-UTR according to the present teaching can comprise any combination of more than one nucleic acid sequence, optionally separated by a linker.

The term "linker" according to the teaching relates to a nucleic acid sequence added between two nucleic acid sequences to connect said two nucleic acid sequences. There is no particular limitation regarding the linker sequence.

A 3'-UTR typically has a length of 200 to 2000 nucleotides, e.g. 500 to 1500 nucleotides. The 3'-untranslated regions of immunoglobulin mRNAs are relatively short (fewer than about 300 nucleotides), while the 3'-untranslated regions of other genes are relatively long. For example, the 3'-untranslated region of tPA is about 800 nucleotides in length, that of factor VIII is about 1800 nucleotides in length and that of erythropoietin is about 560 nucleotides in length. The 3'-untranslated regions of mammalian mRNA typically have a homology region known as the AAUAAA hexanucleotide sequence. This sequence is presumably the poly(A) attachment signal and is frequently located from 10 to 30 bases upstream of the poly(A) attachment site. 3'-untranslated regions may contain one or more inverted repeats which can fold to give stem-loop structures which act as barriers for exoribonucleases or interact with proteins known to increase RNA stability (e.g. RNA-binding proteins).

The human beta-globin 3'-UTR, particularly two consecutive identical copies of the human beta-globin 3'-UTR, contributes to high transcript stability and translational efficiency (Holtkamp et al., 2006, Blood, vol. 108, pp. 4009-4017). Thus, in one embodiment, the RNA replicon according to the present teaching comprises two consecutive identical copies of the human beta-globin 3'-UTR. Thus, it comprises in the 5' → 3' direction: (a) optionally a 5'-UTR; (b) an open reading frame; (c) a 3'-UTR; said 3'-UTR comprising two consecutive identical copies of the human beta-globin 3'-UTR, a fragment thereof, or a variant of the human beta-globin 3'-UTR or fragment thereof.

In one embodiment, the RNA replicon according to the present teaching comprises a 3'-UTR which is active in order to increase translation efficiency and/or stability, but which is not the human beta-globin 3'-UTR, a fragment thereof, or a variant of the human beta-globin 3'-UTR or fragment thereof.

In one embodiment, the RNA replicon according to the present teaching comprises a 5'-UTR which is active in order to increase translation efficiency and/or stability.

### Poly(A) sequence

In some embodiments, the replicon according to the present teaching comprises a 3'-poly(A) sequence. If the replicon comprises conserved sequence element 4 (CSE 4), the 3'-poly(A) sequence of the replicon is preferably present downstream of CSE 4, most preferably directly adjacent to CSE 4.

According to the teaching, in one embodiment, a poly(A) sequence comprises or essentially consists of or consists of at least 20, preferably at least 26, preferably at least 40, preferably at least 80, preferably at least 100 and preferably up to 500, preferably up to 400, preferably up to 300, preferably up to 200, and in particular up to 150, A nucleotides, and in particular about 120 A nucleotides. In this context "essentially consists of" means that most nucleotides in the poly(A) sequence, typically at least 50 %, and preferably at least 75 % by number of nucleotides in the "poly(A) sequence", are A nucleotides (adenylate), but permits that remaining nucleotides are nucleotides other than A nucleotides, such as U nucleotides (uridylate), G nucleotides (guanylate), C nucleotides (cytidylate). In this context "consists of" means that all nucleotides in the poly(A) sequence, i.e. 100 % by number of nucleotides in the poly(A) sequence, are A nucleotides. The term "A nucleotide" or "A" refers to adenylate.

Indeed, it has been demonstrated that a 3' poly(A) sequence of about 120 A nucleotides has a beneficial influence on the levels of RNA in transfected eukaryotic cells, as well as on the levels of protein that is translated from an open reading frame that is present upstream (5') of the 3' poly(A) sequence (Holtkamp et al., 2006, Blood, vol. 108, pp. 4009-4017).

In alphaviruses, a 3' poly(A) sequence of at least 11 consecutive adenylate residues, or at least 25 consecutive adenylate residues, is thought to be important for efficient synthesis of the minus strand. In particular, in alphaviruses, a 3' poly(A) sequence of at least 25 consecutive adenylate residues is understood to function together with conserved sequence element 4 (CSE 4) to promote synthesis of the (-) strand (Hardy & Rice, J. Virol., 2005, vol. 79, pp. 4630-4639).

The present teaching provides for a 3' poly(A) sequence to be attached during RNA transcription, i.e. during preparation of *in vitro* transcribed RNA, based on a DNA template comprising repeated dT nucleotides (deoxythymidylate) in the strand complementary to the coding strand. The DNA sequence encoding a poly(A) sequence (coding strand) is referred to as poly(A) cassette.

In a preferred embodiment of the present teaching, the 3' poly(A) cassette present in the coding strand of DNA essentially consists of dA nucleotides, but is interrupted by a random sequence having an equal distribution of the four nucleotides (dA, dC, dG, dT). Such random sequence may be 5 to 50, preferably 10 to 30, more preferably 10 to 20 nucleotides in length. Such a cassette is disclosed in WO 2016/005004 A1. Any poly(A) cassette disclosed in WO 2016/005004 A1 may be used in the present teaching. A poly(A) cassette that essentially consists of dA nucleotides, but is interrupted by a random sequence having an equal distribution of the four nucleotides (dA, dC, dG, dT) and having a length of e.g. 5 to 50 nucleotides shows, on DNA level, constant propagation of plasmid DNA in *E.coli* and is still associated, on RNA level, with the beneficial properties with respect to supporting RNA stability and translational efficiency.

Consequently, in a preferred embodiment of the present teaching, the 3' poly(A) sequence contained in an RNA molecule described herein essentially consists of A nucleotides, but is interrupted by a random sequence having an equal distribution of the four nucleotides (A, C, G, U). Such random sequence may be 5 to 50, preferably 10 to 30, more preferably 10 to 20 nucleotides in length.

### Codon usage

In general, the degeneracy of the genetic code will allow the substitution of certain codons (base triplets coding for an amino acid) that are present in an RNA sequence by other codons (base triplets), while maintaining the same coding capacity (so that the replacing codon encodes the same amino acid as the replaced codon). In some embodiments of the present teaching, at least one codon of an open reading frame comprised by a RNA molecule differs from the respective codon in the respective open reading frame in the species from which the open reading frame originates. In that embodiment, the coding sequence of the open reading frame is said to be "adapted" or "modified". The coding sequence of an open reading frame comprised by the replicon may be adapted.

For example, when the coding sequence of an open reading frame is adapted, frequently used codons may be selected: WO 2009/024567 A1 describes the adaptation of a coding sequence of a nucleic acid molecule, involving the substitution of rare codons by more frequently used codons. Since the frequency of codon usage depends on the host cell or host organism, that type of adaptation is suitable to fit a nucleic acid sequence to expression in a particular host cell or host organism. Generally, speaking, more frequently used codons are typically translated more efficiently in a host cell or host organism, although adaptation of all codons of an open reading frame is not always required.

For example, when the coding sequence of an open reading frame is adapted, the content of G (guanylate) residues and C (cytidylate) residues may be altered by selecting codons with the highest GC-rich content for each amino acid. RNA molecules with GC-rich open reading frames were reported to have the potential to reduce immune activation and to improve translation and half-life of RNA (Thess et al., 2015, Mol. Ther. 23, 1457-1465).

In particular, the coding sequence for non-structural protein can be adapted as desired. This freedom is possible because the open reading frame encoding non-structural protein does not overlap with the 5' replication recognition sequence of the replicon.

### Safety features of embodiments

The following features are preferred in the present teaching, alone or in any suitable combination:
Preferably, the replicon of the present teaching is not particle-forming. This means that, following inoculation of a host cell by the replicon of the present teaching, the host cell does not produce virus particles, such as next generation virus particles. In one embodiment, the RNA replicon according to the teaching is completely free of genetic information encoding any virus structural protein, e.g., alphavirus structural protein, such as core nucleocapsid protein C, envelope protein P62, and/or envelope protein E1. Preferably, the replicon according to the present teaching does not comprise a virus packaging signal, e.g., an alphavirus packaging signal. For example, the alphavirus packaging signal comprised in the coding region of nsP2 of SFV (White et al. 1998, J. Virol., vol. 72, pp. 4320-4326) may be removed, e.g. by deletion or mutation. A suitable way of removing the alphavirus packaging signal includes adaptation of the codon usage of the coding region of nsP2. The degeneration of the genetic code may allow to delete the function of the packaging signal without affecting the amino acid sequence of the encoded nsP2.

### DNA

The present teaching also provides a DNA comprising a nucleic acid sequence encoding the RNA replicon according to the present teaching.

Preferably, the DNA is double-stranded.

In a preferred embodiment, the DNA is a plasmid. The term "plasmid", as used herein, generally relates to a construct of extrachromosomal genetic material, usually a circular DNA duplex, which can replicate independently of chromosomal DNA.

The DNA of the present teaching may comprise a promoter that can be recognized by a DNA-dependent RNA-polymerase. This allows for transcription of the encoded RNA *in vivo* or *in vitro,* e.g. of the RNA of the present teaching. IVT vectors may be used in a standardized manner as template for *in vitro* transcription. Examples of promoters preferred according to the teaching are promoters for SP6, T3 or T7 polymerase.

In one embodiment, the DNA of the present teaching is an isolated nucleic acid molecule.

### Methods of preparing RNA

The RNA molecule according to the present teaching may be obtainable by in vitro transcription. *In vitro*-transcribed RNA (IVT-RNA) is of particular interest in the present teaching. IVT-RNA is obtainable by transcription from a nucleic acid molecule (particularly a DNA molecule). The DNA molecule(s) of the present teaching are suitable for such purposes, particularly if comprising a promoter that can be recognized by a DNA-dependent RNA-polymerase.

RNA according to the present teaching can be synthesized *in vitro.* This allows to add cap-analogs to the *in vitro* transcription reaction. Typically, the poly(A) tail is encoded by a poly-(dT) sequence on the DNA template. Alternatively, capping and poly(A) tail addition can be achieved enzymatically after transcription.

The *in vitro* transcription methodology is known to the skilled person. For example, as mentioned in WO 2011/015347 A1, a variety of *in vitro* transcription kits is commercially available.

### Kit

The present teaching also provides a kit comprising an RNA replicon according to the teaching.

In one embodiment, the constituents of the kit are present as separate entities. For example, one constituent of the kit may be present in one entity, and another constituent of the kit may be present in a separate entity. For example, an open or closed container is a suitable entity. A closed container is preferred. The container used should preferably be RNAse-free or essentially RNAse-free.

In one embodiment, the kit of the present teaching comprises RNA for inoculation with a cell and/or for administration to a human or animal subject.

The kit according to the present teaching optionally comprises a label or other form of information element, e.g. an electronic data carrier. The label or information element preferably comprises instructions, e.g. printed written instructions or instructions in electronic form that are optionally printable. The instructions may refer to at least one suitable possible use of the kit.

### Pharmaceutical composition

The RNA replicon described herein may be present in the form of a pharmaceutical composition. A pharmaceutical composition according to the teaching may comprise at least one nucleic acid molecule according to the present teaching. A pharmaceutical composition according to the teaching comprises a pharmaceutically acceptable diluent and/or a pharmaceutically acceptable excipient and/or a pharmaceutically acceptable carrier and/or a pharmaceutically acceptable vehicle. The choice of pharmaceutically acceptable carrier, vehicle, excipient or diluent is not particularly limited. Any suitable pharmaceutically acceptable carrier, vehicle, excipient or diluent known in the art may be used.

In one embodiment of the present teaching, a pharmaceutical composition can further comprise a solvent such as an aqueous solvent or any solvent that makes it possible to preserve the integrity of the RNA. In a preferred embodiment, the pharmaceutical composition is an aqueous solution comprising RNA. The aqueous solution may optionally comprise solutes, e.g. salts.

In one embodiment of the present teaching, the pharmaceutical composition is in the form of a freeze-dried composition. A freeze-dried composition is obtainable by freeze-drying a respective aqueous composition.

In one embodiment, the pharmaceutical composition comprises at least one cationic entity. In general, cationic lipids, cationic polymers and other substances with positive charges may form complexes with negatively charged nucleic acids. It is possible to stabilize the RNA according to the teaching by complexation with cationic compounds, preferably polycationic compounds such as for example a cationic or polycationic peptide or protein. In one embodiment, the pharmaceutical composition according to the present teaching comprises at least one cationic molecule selected from the group consisting protamine, polyethylene imine, a poly-L-lysine, a poly-L-arginine, a histone or a cationic lipid.

According to the present teaching, a cationic lipid is a cationic amphiphilic molecule, e.g., a molecule which comprises at least one hydrophilic and lipophilic moiety. The cationic lipid can be monocationic or polycationic. Cationic lipids typically have a lipophilic moiety, such as a sterol, an acyl or diacyl chain, and have an overall net positive charge. The head group of the lipid typically carries the positive charge. The cationic lipid preferably has a positive charge of 1 to 10 valences, more preferably a positive charge of 1 to 3 valences, and more preferably a positive charge of 1 valence. Examples of cationic lipids include, but are not limited to 1,2-di-O-octadecenyl-3-trimethylammonium propane (DOTMA); dimethyldioctadecylammonium (DDAB); 1,2-dioleoyl-3-trimethylammonium-propane (DOTAP); 1,2-dioleoyl-3-dimethylammonium-propane (DODAP); 1,2-diacyloxy-3-dimethylammonium propanes; 1,2-dialkyloxy-3-dimethylammonium propanes; dioctadecyldimethyl ammonium chloride (DODAC), 1,2-dimyristoyloxypropyl-1,3-dimethylhydroxyethyl ammonium (DMRIE), and 2,3-dioleoyloxy-N-[2(spermine carboxamide)ethyl]-N,N-dimethyl-1-propanamium trifluoroacetate (DOSPA). Cationic lipids also include lipids with a tertiary amine group, including 1,2-dilinoleyloxy-N,N-dimethyl-3-aminopropane (DLinDMA). Cationic lipids are suitable for formulating RNA in lipid formulations as described herein, such as liposomes, emulsions and lipoplexes. Typically positive charges are contributed by at least one cationic lipid and negative charges are contributed by the RNA. In one embodiment, the pharmaceutical composition comprises at least one helper lipid, in addition to a cationic lipid. The helper lipid may be a neutral or an anionic lipid. The helper lipid may be a natural lipid, such as a phospholipid, or an analogue of a natural lipid, or a fully synthetic lipid, or lipid-like molecule, with no similarities with natural lipids. In the case where a pharmaceutical composition includes both a cationic lipid and a helper lipid, the molar ratio of the cationic lipid to the neutral lipid can be appropriately determined in view of stability of the formulation and the like.

In one embodiment, the pharmaceutical composition according to the present teaching comprises protamine. According to the teaching, protamine is useful as cationic carrier agent. The term "protamine" refers to any of various strongly basic proteins of relatively low molecular weight that are rich in arginine and are found associated especially with DNA in place of somatic histones in the sperm cells of animals such as fish. In particular, the term "protamine" refers to proteins found in fish sperm that are strongly basic, are soluble in water, are not coagulated by heat, and comprise multiple arginine monomers. According to the teaching, the term "protamine" as used herein is meant to comprise any protamine amino acid sequence obtained or derived from native or biological sources including fragments thereof and multimeric forms of said amino acid sequence or fragment thereof. Furthermore, the term encompasses (synthesized) polypeptides which are artificial and specifically designed for specific purposes and cannot be isolated from native or biological sources.

In some embodiments, the compositions of the teaching may comprise one or more adjuvants. Adjuvants may be added to vaccines to stimulate the immune system's response; adjuvants do not typically provide immunity themselves. Exemplary adjuvants include without limitation the following: Inorganic compounds (e.g. alum, aluminum hydroxide, aluminum phosphate, calcium phosphate hydroxide); mineral oil (e.g. paraffin oil), cytokines (e.g. IL-1, IL-2, IL-12); immunostimulatory polynucleotide (such as RNA or DNA; e.g., CpG-containing oligonucleotides); saponins (e.g. plant saponins from Quillaja, Soybean, *Polygala senega*); oil emulsions or liposomes; polyoxy ethylene ether and poly oxy ethylene ester formulations; polyphosphazene (PCPP); muramyl peptides; imidazoquinolone compounds; thiosemicarbazone compounds; the FIt3 ligand (WO 2010/066418 A1); or any other adjuvant that is known by a person skilled in the art. A preferred adjuvant for administration of RNA according to the present teaching is the FIt3 ligand (WO 2010/066418 A1). When Flt3 ligand is administered together with RNA that codes for an antigen, a strong increase in antigen-specific CD8⁺ T cells may be observed.

The pharmaceutical composition according to the teaching can be buffered, (e.g., with an acetate buffer, a citrate buffer, a succinate buffer, a Tris buffer, a phosphate buffer).

### RNA-containing particles

In some embodiments, owing to the instability of non-protected RNA, it is advantageous to provide the RNA molecules of the present teaching in complexed or encapsulated form. Respective pharmaceutical compositions are provided in the present teaching. In particular, in some embodiments, the pharmaceutical composition of the present teaching comprises nucleic acid-containing particles, preferably RNA-containing particles. Respective pharmaceutical compositions are referred to as particulate formulations. In particulate formulations according to the present teaching, a particle comprises nucleic acid according to the teaching and a pharmaceutically acceptable carrier or a pharmaceutically acceptable vehicle that is suitable for delivery of the nucleic acid. The nucleic acid-containing particles may be, for example, in the form of proteinaceous particles or in the form of lipid-containing particles. Suitable proteins or lipids are referred to as particle forming agents. Proteinaceous particles and lipid-containing particles have been described previously to be suitable for delivery of alphaviral RNA in particulate form (e.g. Strauss & Strauss, Microbiol. Rev., 1994, vol. 58, pp. 491-562). In particular, alphavirus structural proteins (provided e.g. by a helper virus) are a suitable carrier for delivery of RNA in the form of proteinaceous particles.

In one embodiment, the particulate formulation of the present teaching is a nanoparticulate formulation. In that embodiment, the composition according to the present teaching comprises nucleic acid according to the teaching in the form of nanoparticles. Nanoparticulate formulations can be obtained by various protocols and with various complexing compounds. Lipids, polymers, oligomers, or amphipiles are typical constituents of nanoparticulate formulations.

As used herein, the term "nanoparticle" refers to any particle having a diameter making the particle suitable for systemic, in particular parenteral, administration, of, in particular, nucleic acids, typically a diameter of 1000 nanometers (nm) or less. In one embodiment, the nanoparticles have an average diameter in the range of from about 50 nm to about 1000 nm, preferably from about 50 nm to about 400 nm, preferably about 100 nm to about 300 nm such as about 150 nm to about 200 nm. In one embodiment, the nanoparticles have a diameter in the range of about 200 to about 700 nm, about 200 to about 600 nm, preferably about 250 to about 550 nm, in particular about 300 to about 500 nm or about 200 to about 400 nm.

In one embodiment, the polydispersity index (PI) of the nanoparticles described herein, as measured by dynamic light scattering, is 0.5 or less, preferably 0.4 or less or even more preferably 0.3 or less. The "polydispersity index" (PI) is a measurement of homogeneous or heterogeneous size distribution of the individual particles (such as liposomes) in a particle mixture and indicates the breadth of the particle distribution in a mixture. The PI can be determined, for example, as described in WO 2013/143555 A1.

As used herein, the term "nanoparticulate formulation" or similar terms refer to any particulate formulation that contains at least one nanoparticle. In some embodiments, a nanoparticulate composition is a uniform collection of nanoparticles. In some embodiments, a nanoparticulate composition is a lipid-containing pharmaceutical formulation, such as a liposome formulation or an emulsion.

### Lipid-containing pharmaceutical compositions

In one embodiment, the pharmaceutical composition of the present teaching comprises at least one lipid. Preferably, at least one lipid is a cationic lipid. Said lipid-containing pharmaceutical composition comprises nucleic acid according to the present teaching. In one embodiment, the pharmaceutical composition according to the teaching comprises RNA encapsulated in a vesicle, e.g. in a liposome. In one embodiment, the pharmaceutical composition according to the teaching comprises RNA in the form of an emulsion. In one embodiment, the pharmaceutical composition according to the teaching comprises RNA in a complex with a cationic compound, thereby forming e.g. so-called lipoplexes or polyplexes. Encapsulation of RNA within vesicles such as liposomes is distinct from, for instance, lipid/RNA complexes. Lipid/RNA complexes are obtainable e.g. when RNA is e.g. mixed with pre-formed liposomes.

In one embodiment, the pharmaceutical composition according to the teaching comprises RNA encapsulated in a vesicle. Such formulation is a particular particulate formulation according to the teaching. A vesicle is a lipid bilayer rolled up into a spherical shell, enclosing a small space and separating that space from the space outside the vesicle. Typically, the space inside the vesicle is an aqueous space, i.e. comprises water. Typically, the space outside the vesicle is an aqueous space, i.e. comprises water. The lipid bilayer is formed by one or more lipids (vesicle-forming lipids). The membrane enclosing the vesicle is a lamellar phase, similar to that of the plasma membrane. The vesicle according to the present teaching may be a multilamellar vesicle, a unilamellar vesicle, or a mixture thereof. When encapsulated in a vesicle, the RNA is typically separated from any external medium. Thus it is present in protected form, functionally equivalent to the protected form in a natural alphavirus. Suitable vesicles are particles, particularly nanoparticles, as described herein.

For example, RNA may be encapsulated in a liposome. In that embodiment, the pharmaceutical composition is or comprises a liposome formulation. Encapsulation within a liposome will typically protect RNA from RNase digestion. It is possible that the liposomes include some external RNA (e.g. on their surface), but at least half of the RNA (and ideally all of it) is encapsulated within the core of the liposome.

Liposomes are microscopic lipidic vesicles often having one or more bilayers of a vesicle-forming lipid, such as a phospholipid, and are capable of encapsulating a drug, e.g. RNA. Different types of liposomes may be employed in the context of the present teaching, including, without being limited thereto, multilamellar vesicles (MLV), small unilamellar vesicles (SUV), large unilamellar vesicles (LUV), sterically stabilized liposomes (SSL), multivesicular vesicles (MV), and large multivesicular vesicles (LMV) as well as other bilayered forms known in the art. The size and lamellarity of the liposome will depend on the manner of preparation. There are several other forms of supramolecular organization in which lipids may be present in an aqueous medium, comprising lamellar phases, hexagonal and inverse hexagonal phases, cubic phases, micelles, reverse micelles composed of monolayers. These phases may also be obtained in the combination with DNA or RNA, and the interaction with RNA and DNA may substantially affect the phase state. Such phases may be present in nanoparticulate RNA formulations of the present teaching.

Liposomes may be formed using standard methods known to the skilled person. Respective methods include the reverse evaporation method, the ethanol injection method, the dehydration-rehydration method, sonication or other suitable methods. Following liposome formation, the liposomes can be sized to obtain a population of liposomes having a substantially homogeneous size range.

In a preferred embodiment of the present teaching, the RNA is present in a liposome which includes at least one cationic lipid. Respective liposomes can be formed from a single lipid or from a mixture of lipids, provided that at least one cationic lipid is used. Preferred cationic lipids have a nitrogen atom which is capable of being protonated; preferably, such cationic lipids are lipids with a tertiary amine group. A particularly suitable lipid with a tertiary amine group is 1,2-dilinoleyloxy-N,N-dimethyl-3-aminopropane (DLinDMA). In one embodiment, the RNA according to the present teaching is present in a liposome formulation as described in WO 2012/006378 A1: a liposome having a lipid bilayer encapsulating an aqueous core including RNA, wherein the lipid bilayer comprises a lipid with a pKa in the range of 5.0 to 7.6, which preferably has a tertiary amine group. Preferred cationic lipids with a tertiary amine group include DLinDMA (pKa 5.8) and are generally described in WO 2012/031046 A2. According to WO 2012/031046 A2, liposomes comprising a respective compound are particularly suitable for encapsulation of RNA and thus liposomal delivery of RNA. In one embodiment, the RNA according to the present teaching is present in a liposome formulation, wherein the liposome includes at least one cationic lipid whose head group includes at least one nitrogen atom (N) which is capable of being protonated, wherein the liposome and the RNA have a N:P ratio of between 1:1 and 20:1. According to the present teaching, "N:P ratio" refers to the molar ratio of nitrogen atoms (N) in the cationic lipid to phosphate atoms (P) in the RNA comprised in a lipid containing particle (e.g. liposome), as described in WO 2013/006825 A1. The N:P ratio of between 1:1 and 20:1 is implicated in the net charge of the liposome and in efficiency of delivery of RNA to a vertebrate cell.

In one embodiment, the RNA according to the present teaching is present in a liposome formulation that comprises at least one lipid which includes a polyethylene glycol (PEG) moiety, wherein RNA is encapsulated within a PEGylated liposome such that the PEG moiety is present on the liposome's exterior, as described in WO 2012/031043 A1 and WO 2013/033563 A1.

In one embodiment, the RNA according to the present teaching is present in a liposome formulation, wherein the liposome has a diameter in the range of 60-180 nm, as described in WO 2012/030901 A1.

In one embodiment, the RNA according to the present teaching is present in a liposome formulation, wherein the RNA-containing liposomes have a net charge close to zero or negative, as disclosed in WO 2013/143555 A1.

In other embodiments, the RNA according to the present teaching is present in the form of an emulsion. Emulsions have been previously described to be used for delivery of nucleic acid molecules, such as RNA molecules, to cells. Preferred herein are oil-in-water emulsions. The respective emulsion particles comprise an oil core and a cationic lipid. More preferred are cationic oil-in-water emulsions in which the RNA according to the present teaching is complexed to the emulsion particles. The emulsion particles comprise an oil core and a cationic lipid. The cationic lipid can interact with the negatively charged RNA, thereby anchoring the RNA to the emulsion particles. In an oil-in-water emulsion, emulsion particles are dispersed in an aqueous continuous phase. For example, the average diameter of the emulsion particles may typically be from about 80 nm to 180 nm. In one embodiment, the pharmaceutical composition of the present teaching is a cationic oil-in-water emulsion, wherein the emulsion particles comprise an oil core and a cationic lipid, as described in WO 2012/006380 A2. The RNA according to the present teaching may be present in the form of an emulsion comprising a cationic lipid wherein the N:P ratio of the emulsion is at least 4:1, as described in WO 2013/006834 A1. The RNA according to the present teaching may be present in the form of a cationic lipid emulsion, as described in WO 2013/006837 A1. In particular, the composition may comprise RNA complexed with a particle of a cationic oil-in-water emulsion, wherein the ratio of oil/lipid is at least about 8:1 (mole:mole).

In other embodiments, the pharmaceutical composition according to the teaching comprises RNA in the format of a lipoplex. The term, "lipoplex" or "RNA lipoplex" refers to a complex of lipids and nucleic acids such as RNA. Lipoplexes can be formed of cationic (positively charged) liposomes and the anionic (negatively charged) nucleic acid. The cationic liposomes can also include a neutral "helper" lipid. In the simplest case, the lipoplexes form spontaneously by mixing the nucleic acid with the liposomes with a certain mixing protocol, however various other protocols may be applied. It is understood that electrostatic interactions between positively charged liposomes and negatively charged nucleic acid are the driving force for the lipoplex formation (WO 2013/143555 A1). In one embodiment of the present teaching, the net charge of the RNA lipoplex particles is close to zero or negative. It is known that electro-neutral or negatively charged lipoplexes of RNA and liposomes lead to substantial RNA expression in spleen dendritic cells (DCs) after systemic administration and are not associated with the elevated toxicity that has been reported for positively charged liposomes and lipoplexes (cf. WO 2013/143555 A1). Therefore, in one embodiment of the present teaching, the pharmaceutical composition according to the teaching comprises RNA in the format of nanoparticles, preferably lipoplex nanoparticles, in which (i) the number of positive charges in the nanoparticles does not exceed the number of negative charges in the nanoparticles and/or (ii) the nanoparticles have a neutral or net negative charge and/or (iii) the charge ratio of positive charges to negative charges in the nanoparticles is 1.4:1 or less and/or (iv) the zeta potential of the nanoparticles is 0 or less. As described in WO 2013/143555 A1, zeta potential is a scientific term for electrokinetic potential in colloidal systems. In the present teaching, (a) the zeta potential and (b) the charge ratio of the cationic lipid to the RNA in the nanoparticles can both be calculated as disclosed in WO 2013/143555 A1. In summary, pharmaceutical compositions which are nanoparticulate lipoplex formulations with a defined particle size, wherein the net charge of the particles is close to zero or negative, as disclosed in WO 2013/143555 A1, are preferred pharmaceutical compositions in the context of the present teaching.

### Methods for producing a protein

The present teaching also provides a method for producing a protein of interest in a cell comprising the steps of:
(a) obtaining the RNA replicon according to the teaching, which comprises an open reading frame encoding the protein of interest, and
(b) inoculating the RNA replicon into the cell.

In various embodiments of the method, the RNA replicon is as defined above for the RNA replicon of the teaching, as long as the RNA replicon comprises an open reading frame encoding functional non-structural protein and an open reading frame encoding the protein of interest, and can be replicated by the functional non-structural protein.

The cell into which one or more nucleic molecule can be inoculated can be referred to as "host cell". According to the teaching, the term "host cell" refers to any cell which can be transformed or transfected with an exogenous nucleic acid molecule. The term "cell" preferably is an intact cell, i.e. a cell with an intact membrane that has not released its normal intracellular components such as enzymes, organelles, or genetic material. An intact cell preferably is a viable cell, i.e. a living cell capable of carrying out its normal metabolic functions. The term "host cell" comprises, according to the teaching, prokaryotic (e.g. *E.coli*) or eukaryotic cells (e.g. human and animal cells, plant cells, yeast cells and insect cells). Particular preference is given to mammalian cells such as cells from humans, mice, hamsters, pigs, domesticated animals including horses, cows, sheep and goats, as well as primates. The cells may be derived from a multiplicity of tissue types and comprise primary cells and cell lines. Specific examples include keratinocytes, peripheral blood leukocytes, bone marrow stem cells and embryonic stem cells. In other embodiments, the host cell is an antigen-presenting cell, in particular a dendritic cell, a monocyte or a macrophage. A nucleic acid may be present in the host cell in a single or in several copies and, in one embodiment is expressed in the host cell.

The cell may be a prokaryotic cell or a eukaryotic cell. Prokaryotic cells are suitable herein e.g. for propagation of DNA according to the teaching, and eukaryotic cells are suitable herein e.g. for expression of the open reading frame of the replicon.

In the method of the present teaching, any of the RNA replicon according to the teaching, or the kit according to the teaching, or the pharmaceutical composition according to the teaching, can be used. RNA can be used in the form of a pharmaceutical composition, or as naked RNA e.g. for electroporation.

In the method for producing a protein in a cell according to the present teaching, the cell may be an antigen presenting cell, and the method may be used for expressing the RNA encoding the antigen. To this end, the teaching may involve introduction of RNA encoding antigen into antigen presenting cells such as dendritic cells. For transfection of antigen presenting cells such as dendritic cells a pharmaceutical composition comprising RNA encoding the antigen may be used.

In one embodiment, a method for producing a protein in a cell is an *in vitro* method. In one embodiment, a method for production of a protein in a cell does not comprise the removal of a cell from a human or animal subject by surgery or therapy.

In this embodiment, the cell inoculated according to the teaching may be administered to a subject so as to produce the protein in the subject and to provide the subject with the protein. The cell may be autologous, syngenic, allogenic or heterologous with respect to the subject.

In other embodiments, the cell in a method for producing a protein in a cell may be present in a subject, such as a patient. In these embodiments, the method for producing a protein in a cell is an *in vivo* method which comprises administration of RNA molecules to the subject.

In this respect, the teaching also provides a method for producing a protein of interest in a subject comprising the steps of:
(a) obtaining the RNA replicon according to the teaching, which comprises an open reading frame encoding the protein of interest, and
(b) administering the RNA replicon to the subject.

In various embodiments of the method, the RNA replicon is as defined above for the RNA replicon of the teaching, as long as the RNA replicon comprises an open reading frame encoding functional non-structural protein and an open reading frame encoding the protein of interest, and can be replicated by the functional non-structural protein.

Any of the RNA replicon according to the teaching, or the kit according to the teaching, or the pharmaceutical composition according to the teaching can be used in the method for producing a protein in a subject according to the teaching. For example, in the method of the teaching, RNA can be used in the format of a pharmaceutical composition, e.g. as described herein, or as naked RNA.

In view of the capacity to be administered to a subject, each of the RNA replicon according to the teaching, or the kit according to the teaching, or the pharmaceutical composition according to the teaching, may be referred to as "medicament", or the like. The present teaching foresees that the RNA replicon, the kit, the pharmaceutical composition of the present teaching are provided for use as a medicament. The medicament can be used to treat a subject. By "treat" is meant to administer a compound or composition or other entity as described herein to a subject. The term includes methods for treatment of the human or animal body by therapy.

The above described medicament does typically not comprise a DNA, and is thus associated with additional safety features compared to DNA vaccines described in the prior art (e.g. WO 2008/119827 A1).

An alternative medical use according to the present teaching comprises a method for producing a protein in a cell according to the present teaching, wherein the cell may be an antigen presenting cell such as a dendritic cell, followed by the introduction of said cell to a subject. For example, RNA encoding a pharmaceutically active protein, such as an antigen, may be introduced (transfected) into antigen-presenting cells ex *vivo,* e.g. antigen-presenting cells taken from a subject, and the antigen-presenting cells, optionally clonally propagated *ex vivo,* may be reintroduced into the same or a different subject. Transfected cells may be reintroduced into the subject using any means known in the art.

The medicament according to the present teaching may be administered to a subject in need thereof. The medicament of the present teaching can be used in prophylactic as well as in therapeutic methods of treatment of a subject.

The medicament according to the teaching is administered in an effective amount. An "effective amount" concerns an amount that is sufficient, alone or together with other doses, to cause a reaction or a desired effect. In the case of treatment of a certain disease or a certain condition in a subject, the desired effect is the inhibition of disease progression. This includes the deceleration of disease progression, in particular the interruption of disease progression. The desired effect in the treatment of a disease or a condition can also be a delay of disease outbreak or the inhibition of disease outbreak.

The effective amount will depend on the condition being treated, the severity of the disease, the individual parameters of the patient, including age, physiological condition, size and weight, duration of the treatment, type of accompanying therapy (if any), the specific mode of administration and other factors.

### Vaccination

The term "immunization" or "vaccination" generally refers to a process of treating a subject for therapeutic or prophylactic reasons. A treatment, particularly a prophylactic treatment, is or comprises preferably a treatment aiming to induce or enhance an immune response of a subject, e.g. against one or more antigens. If, according to the present teaching, it is desired to induce or enhance an immune response by using RNA as described herein, the immune response may be triggered or enhanced by the RNA. In one embodiment, the teaching provides a prophylactic treatment which is or comprises preferably the vaccination of a subject. An embodiment of the present teaching wherein the replicon encodes, as a protein of interest, a pharmaceutically active peptide or protein which is an immunologically active compound or an antigen is particularly useful for vaccination.

RNA has been previously described for vaccination against foreign agents including pathogens or cancer (reviewed recently by Ulmer et al., 2012, Vaccine, vol. 30, pp. 4414-4418). In contrast to common approaches in the prior art, the replicon according to the present teaching is a particularly suitable element for efficient vaccination because of the ability to be replicated by functional alphavirus non-structural protein as described herein. The vaccination according to the present teaching can be used for example for induction of an immune response to weakly immunogenic proteins. In the case of the RNA vaccines according to the teaching, the protein antigen is never exposed to serum antibodies, but is produced by transfected cells themselves after translation of the RNA. Therefore anaphylaxis should not be a problem. The teaching therefore permits the repeated immunization of a patient without risk of allergic reactions.

In methods involving vaccination according to the present teaching, the medicament of the present teaching is administered to a subject, in particular if treating a subject having a disease involving the antigen or at risk of falling ill with the disease involving the antigen is desired.

In methods involving vaccination according to the present teaching, the protein of interest encoded by the replicon according to the present teaching codes for example for a bacterial antigen, against which an immune response is to be directed, or for a viral antigen, against which an immune response is to be directed, or for a cancer antigen, against which an immune response is to be directed, or for an antigen of a unicellular organism, against which an immune response is to be directed. The efficacy of vaccination can be assessed by known standard methods such as by measurement of antigen-specific IgG antibodies from the organism. In methods involving allergen-specific immunotherapy according to the present teaching, the protein of interest encoded by the replicon according to the present teaching codes for an antigen relevant to an allergy. Allergen-specific immunotherapy (also known as hypo-sensitization) is defined as the administration of preferably increasing doses of an allergen vaccine to an organism with one or more allergies, in order to achieve a state in which the symptoms that are associated with a subsequent exposure to the causative allergen are alleviated. The efficacy of an allergen-specific immunotherapy can be assessed by known standard methods such as by measurement of allergen-specific IgG and IgE antibodies from the organism.

The medicament of the present teaching can be administered to a subject, e.g. for treatment of the subject, including vaccination of the subject.

The term "subject" relates to vertebrates, particularly mammals. For example, mammals in the context of the present teaching are humans, non-human primates, domesticated mammals such as dogs, cats, sheep, cattle, goats, pigs, horses etc., laboratory animals such as mice, rats, rabbits, guinea pigs, etc. as well as animals in captivity such as animals of zoos. The term "subject" also relates to non-mammalian vertebrates such as birds (particularly domesticated birds such as chicken, ducks, geese, turkeys) and to fish (particularly farmed fish, e.g. salmon or catfish). The term "animal" as used herein also includes humans.

The administration to domesticated animals such as dogs, cats, rabbits, guinea pigs, hamsters, sheep, cattle, goats, pigs, horses, chicken, ducks, geese, turkeys, or wild animals, e.g. foxes, is preferred in some embodiments. For example, a prophylactic vaccination according to the present teaching may be suitable to vaccinate an animal population, e.g. in the farming industry, or a wild animal population. Other animal populations in captivity, such as pets, or animals of zoos, may be vaccinated.

When administered to a subject, the replicon used as a medicament does preferably not comprise sequences from a type of virus, e.g., alphavirus, that is infectious to the species or genus to which the treated subject belongs. Preferably, in that case, the replicon does not comprise any nucleotide sequence from an alphavirus that can infect the respective species or genus. This embodiment bears the advantage that no recombination with infectious (e.g. fully functional or wild-type) alphavirus is possible, even if the subject to which the RNA is administered is (e.g. accidentally) affected by infectious alphavirus. As an illustrative example, for treatment of pigs, the replicon used does not comprise any nucleotide sequence from an alphavirus that can infect pigs.

### Mode of administration

The medicament according to the present teaching can be applied to a subject in any suitable route.

For example, the medicament may be administered systemically, for example intravenously (i.v.), subcutaneously (s.c.), intradermally (i.d.) or by inhalation.

In one embodiment, the medicament according to the present teaching is administered to muscle tissue, such as skeletal muscle, or skin, e.g. subcutaneously. It is generally understood that transfer of RNA into the skin or muscles leads to high and sustained local expression, paralleled by a strong induction of humoral and cellular immune responses (Johansson et al. 2012, PLoS. One., 7, e29732; Geall et al., 2012, Proc. Natl. Acad. Sci. U. S. A, vol. 109, pp. 14604-14609).

Alternatives to administration to muscle tissue or skin include, but are not limited to: intradermal, intranasal, intraocular, intraperitoneal, intravenous, interstitial, buccal, transdermal, or sublingual administration. Intradermal and intramuscular administration are two preferred routes.

Administration can be achieved in various ways. In one embodiment, the medicament according to the present teaching is administered by injection. In a preferred embodiment, injection is via a needle. Needle-free injection may be used as an alternative.

The present teaching is described in detail and is illustrated by the figures and examples, which are used only for illustration purposes and are not meant to be limiting. Owing to the description and the examples, further embodiments which are likewise included in the teaching are accessible to the skilled worker.

### Examples

### Material and Methods:

The following materials and methods were used in the examples.

### Plasmids

Vectors systems used herein were engineered from Venezuelan Equine Encephalitis virus (VEEV; accession no. L01442)(Figure 1), but can be done in a comparable way from all other alphaviruses. In a first step, a plasmid encoding a conventional self-replicating RNA (called replicon) was obtained by combining fragments of the VEEV-genome obtained by gene synthesis from a commercial provider. This construct lacks VEEV structural genes, but contains all conserved sequence elements (CSE) of VEEV that serve as replication-recognition sequence (RRS) and control viral replication. The VEEV-sequences were inserted into the pST1 plasmid backbone (Holtkamp et al., 2006, Blood, vol. 108, pp. 4009-4017) under the transcriptional control of a T7 phage RNA-polymerase promoter. A poly(A) cassette consisting of 30 and 70 adenylate residues (polyA30-70), separated by a 10 nucleotide random sequence (WO 2016/005004 A1), was added immediately downstream of the very last nucleotide of the VEEV 3'CSE. A *Sap*I restriction site for plasmid linearization was placed immediately downstream of the poly(A) cassette. The insertion of genes of interest (GOI) into replicons is done downstream of the subgenomic promoter (SGP).

PCR- and recombination-based seamless cloning techniques were used to generate required plasmids. To generate a replicon RNA encoding firefly luciferase, we inserted the open reading frame (ORF) of luciferase as GOI downstream of the SGP. For a replicon RNA expressing a fusion of the enhanced green fluorescence protein (GFP) to the secretable nano-luciferase (SNL) the respective fusion gene (GFP-SNL) was inserted downstream to the SGP. The constructs were named in the following systematic manner: replicon encoding luciferase was named "c-R-sL" (c=cap, R=replicase, s= subgenomic promorer, L= firefly luciferase), a replicon encoding GFP-SNL was named accordingly "c-R-sGFPSNL". The "c" indicates that replicase is translated under the control of the 5'-terminal cap structure.

A derivation of the conventional replicon made use of an exchange of the SGP against the internal ribosomal entry site (IRES) of the Encephalomyocarditis virus (EMCV). Thereby the IRES is upstream of the GOI. A construct expressing luciferase was named "c-R-iL", where "i" indicates the use of the IRES instead of the SGP to express luciferase while the replicase remains translated in a cap-dependent ("c") manner. To generate derivatives of the replicon that put the translation of the replicase from the cap downstream to the EMCV IRES, we exchanged the wildtype (wt) RRS against a modified 5' replication-recognition sequence (RRS) which is lacking the original replicase start-codon as well as all other ATG-triplets internal to the nsP1 sequence overlapping to the RRS (ΔATG-RRS)(WO 2017/162460). Downstream to that we inserted firefly luciferase, followed further downstream be the EMCV IRES and next the replicase ORF. When RNA is transcribed from this plasmid the luciferase is translated in a cap-dependent manner, and the replicase in an IRES-dependent manner. Accordingly, the construct was abbreviated "c-L-iR".

A further variant of this was encoded on a plasmid with a cassette bearing the SGP downstream of the IRES-controlled replicase, and this SGP was followed by the ORF of the GFP-SNL fusion protein. Accordingly, the construct was named "c-L-iR-sGFPSNL".

In additions to constructs encoding the replicase with the wildtype sequence, we generated versions with mutated, transcriptionally inactive replicase nsP4-subunit, which were thus replication-deficient. To this aim, two aspartatic acid residues at positions 2351 and 2352 of the replicase in the amino acid sequence were exchanged by alanins (nsP4-GAA). Replicons with mutated replicase were named "c-R(GAA)-iL", "c-R(GAA)-sL", "c-L-iR(GAA)" or "c-L-iR(GAA)-sGFPSNL".

Plasmid used for in vitro transcription of non-replicable mRNA encoding firefly luciferase (named mRNA-L) was used as reference in some of the experiments.

Plasmid encoding bicistronic trans-replication competent RNA with SNL upstream and firefly luciferase downstream of an IRES was generated from a plasmid backbone equivalent to "Δ5ATG-RRS - bicistronic" (described in WO 2017/162460). Instead of the SGP we inserted either the EMCV IRES or the Israeli acute paralysis virus (IAPV) intergenic region (IGR). The replicative RNAs were named "c-S-EMCV IRES-L" and "c-S-IAPV IGR-L".

Additionally, we cloned an IRES-launched replicon based on Semliki Forest Virus (SFV) sequences (clone SFV4) with the same modular structure as the VEEV replicon launched by the EMCV-IRES. To this aim, we exchanged the wt RRS of SFV against a modified 5'-RRS which is lacking the original replicase start-codon as well as all other ATG-triplets internal to the nsP1 sequence overlapping to the RRS (ΔATG-RRS)(WO 2017/162460), followed by the EMCV IRES, the SFV-replicase, the SFV subgenomic promoter, the GFP-SNL reporter gene and the SFV 3'-UTR and 3'RRS.

To test a variant of VEEV IRES-launched replicon (c-L-iR) with increased RNA replication potential we exchanged nucleotide 4 within the 5'-RRS of from G to A which equals the VEEV vaccine strain TC-83 (ΔATG-RRS-TC83).

Furthermore, IRES-launched replicons made of the VEEV vaccine strain TC-83 (ΔATG-RRS-TC83) were equipped with IRESes from other viruses similarly to the vectors with EMCV-IRES. The hepatitis C virus (HCV) 5'-untranslated region (UT) and the beginning of the polyprotein open reading frame, as well as the 5'-UTR of enterovirus 71 (EV71) harbor IRES activity. We cloned the 5'-terminal 408 nucleotides of HCV (accession number AB016785) and the 5'terminal 743 nucleotides of EV71) (accession number U22521) for use as an IRES in our vectors.

To assess whether interferon inhibitors can increase vector expression we inserted the Vaccinia virus PKR inhibitor E3 or the Toscana virus gene NSs upstream of the EMCV-IRES into IRES-launched VEEV-replicons with ΔATG-RRS-TC83.

### In vitro transcription

*In vitro* transcription from plasmids described above and purification of RNA was performed as previously described with the exception that beta-S-ARCA(D2) cap analog was used instead of ARCA (Holtkamp et al., *supra;* Kuhn et al., 2010, Gene Ther., vol. 17, pp. 961-971). Quality of purified RNA was assessed by spectrophotometry, and capillary electrophoresis (2100 BioAnalyzer, Agilent, Santa Clara, USA). The RNA used in the examples is purified IVT-RNA.

**Cell culture:** All growth media, fetal calf serum (FCS), antibiotics and other supplements were supplied by Life Technologies/Gibco, except when stated otherwise. Human foreskin fibroblasts obtained from System Bioscience (HFF, neonatal) were cultivated in minimum essential media (MEM) containing 15% FCS, 1% non-essential amino acids, 1mM sodium pyruvate at 37°C. Cells were grown at 37°C in humidified atmosphere equilibrated to 5% CO2. BHK21 cells (ATCC; CCL10) were grown in Eagle's Minimum Essential medium supplemented with 10% FCS. C2C12 cells (ATCC; CRL-1772) were grown in Dulbeccos Modified Eagle's medium (DMEM) supplemented with 10% FCS, 1 mM sodium-pyruvate. K-562 cells (ATCC; CCL-243) were grown in RPMI medium supplemented with 10% FCS.

### RNA transfer into cells:

For electroporation, RNA resuspended in a final volume of 62.5 µl/mm cuvette gap size was electroporated into cells at room temperature using a square-wave electroporation device (BTX ECM 830, Harvard Apparatus, Holliston, MA, USA). For used cell types following settings were applied: HFF (500 V/cm, 1 pulse of 24 milliseconds (ms)); BHK-21 (750 V/cm, 1 pulse of 16 ms); C2C12 (600 V/cm, 5 pulses of 5 ms, with intervals of 400 ms); K562 (650 V/cm, 3 pulses of 8 ms and intervals of 400 ms).

RNA lipofections were performed using Lipofectamine MessengerMAX following the manufacturer's instructions (Life Technologies, Darmstadt, Germany). Adherent cells were plated at approximately 20,000 cells/cm² growth area and transfected 24h later with a total amount of 250 ng/cm² RNA and 1 µl/cm² MessengerMAX. RNA species were mixed in RNAse-free Eppendorf tubes.

**Luciferase Assays:** To assess the expression of luciferase in transfected cells, transfected cells were plated in 96-well white microplates (Nunc, Langenselbold, Germany). The detection of firefly luciferase was performed with the Bright-Glo Luciferase Assay System; NanoLuc was detected using the NanoGlo kit (both Promega, Madison, WI, USA) according to the manufacturer's instructions. Bioluminescence was measured using a microplate luminescence reader Infinite M200 (Tecan Group, Männedorf, Switzerland). Data are represented in relative luciferase units [RLU]. Luciferase-negative cells were used to assess the background signal.

**Western Blot:** Cells were lysed using RIPA buffer. Lysates supplemented with 4x Laemmli buffer, were separated by 7.5 % SDS-PAGE and wet-blotted onto nitrocellulose membranes (GE Healthcare LS, Marlborough, MA, USA) using the Trans-Blot Cell (Bio-Rad, Berkeley, CA, USA). Membranes were blocked with 5 % non-fat dry milk in 1xPBS-T and incubated with appropriate dilutions of primary antibodies (anti-myc clone 9E10 (Sigma-Aldrich, #M4439); anti-tubulin clone B-5-1-2 (Sigma-Aldrich, #T5168) and secondary antibodies. Replicase expression was detected using a myc-tag inserted in frame into the variable domain of the nsP3 subunit. To ensure equal loading the concentration of proteins in cell lysates was measured by Pierce BCA Protein Assay (ThermoFisher Scientific, Waltham, MA, USA) and controlled by the detection of α-tubulin. Chemiluminescent signals were developed with Lumi-Light Western Blotting Substrate (Roche) and were recorded using the LAS 4000 system (GE Healthcare LS).

**Quantitative real-time reverse transcriptase PCR (qRT-PCR):** To analyse amplification of exogenous RNA in transfected cells, total cellular RNA was extracted using the RNeasy Mini Kit (Qiagen) according the manufacturer's instructions and reverse transcribed with Superscript IV First-Strand cDNA Synthesis kit (Invitrogen) and random hexamers from PrimerScript Kit (TaKaRa). qRT-PCR were performed with the QuantiTect SYBR Green PCR Kit (Qiagen), the ABI 7300 Real time PCR System, and the companion SDS analysis software (Applied Biosystems), following the manufacturer's instruction. The following specific primers were used: fVEEnsp1 5'-CACGTTGACATCGAGGAAGAC-3'; rVEEnsp1 5'-TCCACCTCCGTTTCGATCAG-3'.

**Example 1 (see** **Figure 1****). RNA constructs.** (1) In conventional alphaviral replicons, the non-structural proteins 1 to 4 (nsP1-4; =replicase enzyme complex) are translated in a cap-dependent manner from the 5'-end of the RNA. The subgenomic transcript that starts within the subgenomic promoter (SGP) and encodes the first gene of interest (GOI 1) is also capped. (2) When an internal ribosome entry site (IRES) substitutes the SGP, the GOI 1 is no longer encoded by a capped subgenomic transcript. Instead, it is encoded by the genomic RNA, and its translation initiates internally (downstream of the nsP1-4) independently from a cap. (3) An IRES upstream of nsP1-4 uncouples the translation of the nsP1-4 from the 5'cap. To conserve the replicative potential of the RNA it is required to keep the unaltered (wildtype; wt) 5' replication-recognition sequence (RRS) which overlaps with the 5'untranslated region (UTR) and the N-terminus of nsP1 (nsP1*). In this IRES-launched replicon the translation of nsP1-4 depends upon the characteristics of the selected IRES, while expression of GOI 1 remains under the control of the SGP. (4) A second GOI (GOI 2) can be fused in frame to the N-terminal part of nsP1* overlapping to the 5'RRS. (5) In a further variant, the SGP and the GOI 1 downstream of it may be removed. (6) To avoid the translation of the N-terminal nsP1* the original start-codon and further putative AUG-codons can be mutated (ΔAUG), thereby enabling cap-dependent translation to start at the AUG of GOI 2. The nsP1-4 remain downstream to the IRES, and GOI 1 downstream to the SGP. (7) A simplified construct having a 5'RRS lacking AUG, and lacking the SGP and GOI 1. (1 - 7) All constructs are 5'-poly-adenylated *in vitro* and *in cellulo.* (3 - 7) All IRES-launched replicons may or may not be capped *in vitro.* In cells, the nsP1-4 will add caps and poly-A to novel RNA copies.

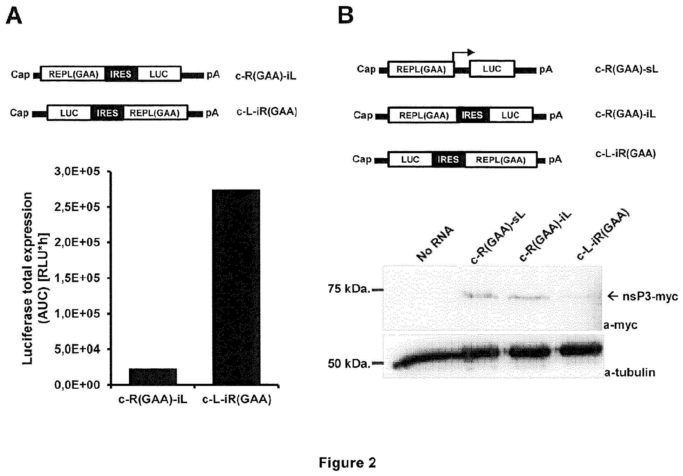

**Example 2 (see** **Figure 2****): IRES-mediated expression is approximately 10 times weaker than cap-dependent translation. (A)** K562 cells were electroporated with 3 µg replicons expressing inactive replicase (nsP4-GAA-mutant) and luciferase as depicted. Either the inactive replicase was placed under the control of the 5' cap and luciferase downstream of an EMCV IRES (c-R(GAA)-iL) or vice versa, the luciferase under the control of the cap and replicase downstream of the IRES (c-L-iR(GAA). 6h to 48h after electroporation luciferase activity was measured and the total expression of luciferase calculated as area under the curve (AUC). Luciferase translation was much lower when placing the ORF downstream of the IRES, which is known from literature (Hennecke et al., 2001, Nucleic Acid Research, Vol 29, No. 16, pp.3327-3334). **(B)** Electroporation of BHK-21 cells with 5 µg of the depicted constructs expressing inactive replicase and luciferase. The cells were lysed after 6h to assess replicase translation by western blot (detection of myc-tagged nsP3). To assess the translation of replicase in the IRES-containing constructs (c-R(GAA)-iL and c-L-iR(GAA) we included a conventional, but also replicase-inactive replicon expressing luciferase downstream of the subgenomic promoter (c-R(GAA)-sL) as a reference. Replicase-expression was inferred from the nsP3-subunit that was myc-tagged within the variable region of nsP3 as described before (Beissert et al., Mol. Ther. 2020; vol 28(1); pp. 119-128).

Replicase translation in the reference was weak, but detectable. The exchange of the subgenomic promoter for an IRES while keeping the replicase under the translational control of the cap did not change replicase expression. However, when replicase is placed downstream to the IRES its expression was much lower, in line to the observation with luciferase downstream to the IRES.

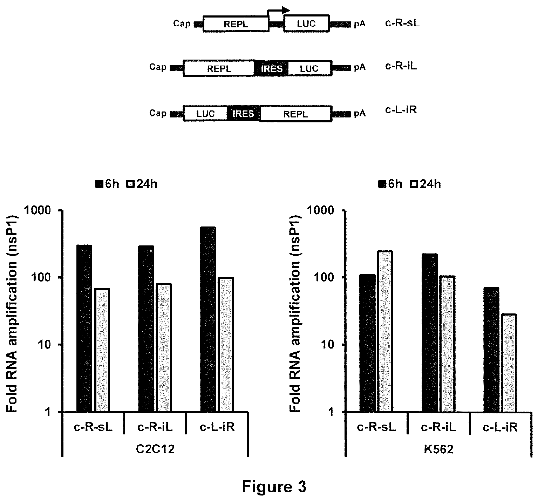

**Example 3 (see** **Figure 3****): IRES-mediated replicase expression enables RNA replication.** C2C12 or K562 cells were electroporated with 3µg of the indicated replicons expressing active replicase. To assess self-amplification of the RNA-constructs, cells were lysed, RNA was extracted and reverse transcribed early after transfer before replication starts (0.5h), or 6h and 24h after electroporation to assess and compare RNA amplification. qPCR primers were binding within replicase (nsP1) to detect and quantify genomic RNA copies. Fold RNA amplification was calculated as the quotient of RNA levels at the indicated time point to the RNA level immediately after electroporation (Fold RNA amplification)

All tested constructs were self-amplified. The very low replicase translation downstream of the IRES that we detected before (example 1) did not impose a barrier to amplification.

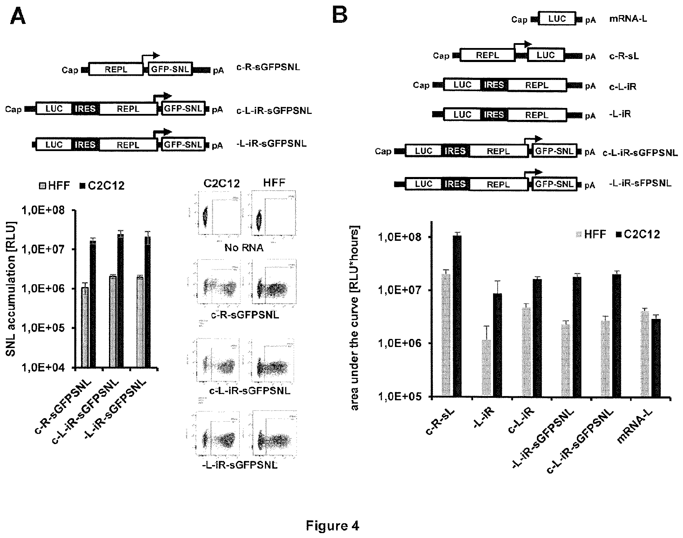

**Example 4 (see** **Figure 4****): IRES-launched replicon show robust expression of both open reading frames. (A)** Equimolar amounts of replicon RNA constructs expressing a fusion protein of the enhanced green fluorescent protein to secretable nano-luciferase (GFP-SNL) were electroporated into human foreskin fibroblasts (HFF) and C2C12 cells. Conventional saRNA served as reference for subgenomic expression of GFP-SNL (c-R-sGFPSNL), and equimolarity was adjusted to 3µg c-R-sGFPSNL. GFP served to monitor transfection rates. Since secreted Nano-Luciferase has a very long half-life in cell culture supernatants (4 days) it allows to assess total protein accumulation with the period between RNA transfer and harvesting the supernatant. The construct expressing replicase downstream of the IRES and upstream of the SGP was used either being 5'-in vitro capped (c-L-iR-sGFPSNL) or being 5'-uncapped (-L-iR-sGFPSNL). Both, GFP expression measured 24h after electroporation, as well as the accumulation of SNL expressed from subgenomic transcripts 48h after electroporation showed that gene expression of all self-amplifying RNA was comparable. This demonstrated that low levels of replicase translation mediated by the IRES does not impose a barrier to transgene expression. **(B)** The construct organization of the novel IRES-launched replicon RNA allows to insert a second gene of interest upstream to the IRES. This upstream gene would be translated under the control of the 5'cap. *In vitro,* this cap may be added during in vitro transcription, or may not be added. Upon transfer to the cells and subsequent replication, *de novo* transcribed RNA will be capped by the replicase nsP1 subunit. Here, we placed firefly luciferase upstream of the IRES into the novel vectors. A 72h time course experiment was done with measurements 6h, 24h, 48h and 72h. Total expression was approximated by calculating the area under the curve. Non-replicating mRNA coding luciferase (mRNA-L) served as reference to assess the strength of cap-dependent translation in the novel saRNA vectors, conventional saRNA served as reference for subgenomic expression (c-R-sL). The data show that the upstream luciferase is well expressed in the cells, at a level between mRNA and subgenomic mediated expression. Thereby, IRES-launched replicons offer the opportunity to co-express two genes independently and at different levels within transfected cells.

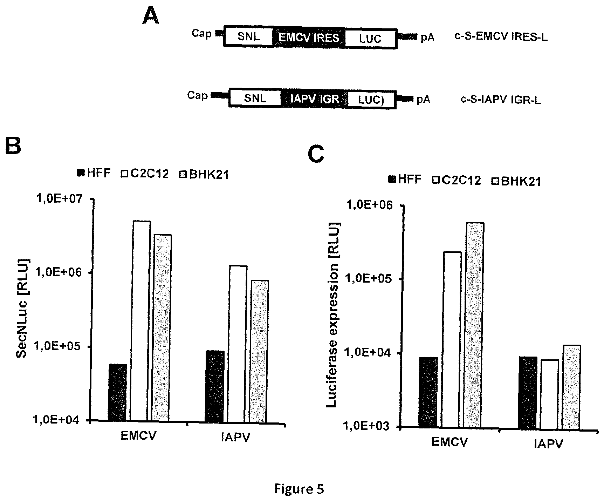

**Example 5 (see** **Figure 5****): Factor independent Dicistrovirus intergenic region compared to EMCV IRES. (A)** Capped bicistronic nano-trans-replicons encode secreted NanoLuciferase (SNL) upstream and firefly luciferase downstream of either the Encephalomyocarditis virus (EMCV) IRES (c-S-EMCV-IRES-L) or the Israeli acute paralysis virus (IAPV) intergenic region (IGR) (c-S-IAPV-IGR-L). The 5'end of these transreplicons was free of AUGs (ΔATG-RRS). Both RNA constructs were lipofected using MessengerMax into primary human foreskin fibroblasts (HFF), C2C12 murine myoblast cells and BHK-21 cells. Transgene expression was measured 24h later. **(B)** Cap dependent SNL translation of both constructs. **(C)** EMCV IRES and IAPV IGR dependent expression of firefly luciferase.

The cap-dependent translation of SNL was much stronger in C2C12 and BHK-21 cells compared to HFF. The level of exogenous gene expression differs in cells in dependency of the innate immune response. HFF mount a strong IFN-response and protein-kinase R activation upon RNA transfection which affects the cap-dependent translation. The EMCV IRES requires the same set of cellular translation initiation factors as cap dependent translation, with the exception of the cap binding protein. Therefore the EMCV IRES is also affected by innate immunity very similarly to the cap-dependent translation. The IAPV IGR, however, does not need cellular factors, this RNA sequence attracts the ribosome directly to the RNA by tRNA-mimicry. Accordingly, the IAPV IGR mediated luciferase expression was comparable in all three cell lines.

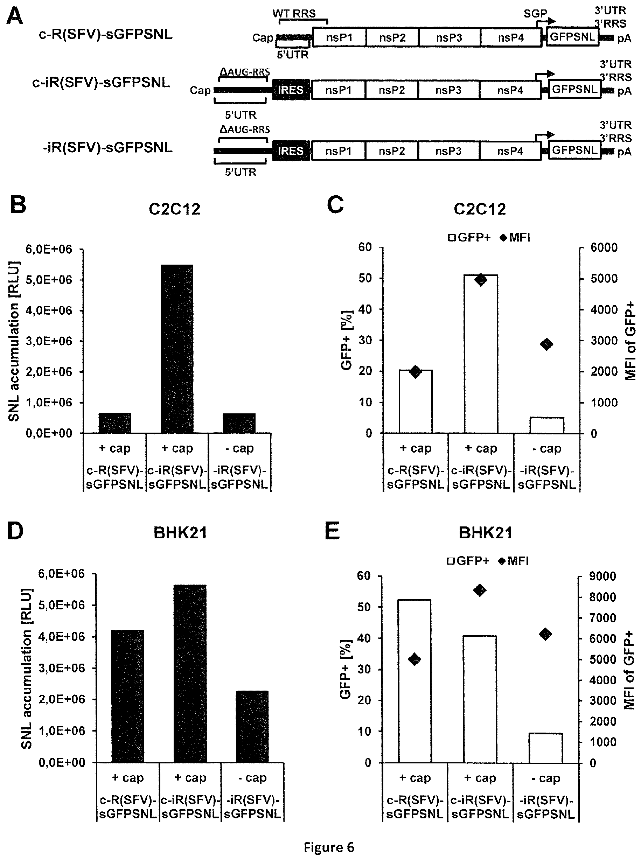

**Example 6 (see** **Figure 6****): IRES-launched replicons derived from the Semliki Forest Virus are replicating with and without cap. (A)** IRES-launched replicons used in this example were constructed from Semliki Forest Virus (SFV) replicon vectors encoding GFP-SNL (c-R(SFV)-sGFPSNL) as described in the material and methods paragraph (c-iR(SFV)-sGFPSNL). The IRES-launched replicon RNA was *in vitro* transcribed with and without a cap analogon, the parentral replicon was capped. **(B,** C) Murine C2C12 cells or (D, E) BHK21 cells were lipofected using MessengerMax. After 24h, cell culture supernatants and the cells were harvested. **(B, D)** Cell supernatants were used to measure the accumulation of SNL (NanoGlo assay) which reflects the total protein secretion within the 24h following transfection. **(C, E)** GFP expression of the cells was assessed by flow cytometry to determine the transfection rates and mean fluorescence intensity (MFI) of GFP+ cells.

As observed before for VEEV-based vectors, the SFV-based IRES-launched replicons were replication competent resulting in robust reporter gene expression in both cells lines. Capping was beneficial, but not prerequisite for vector expression.

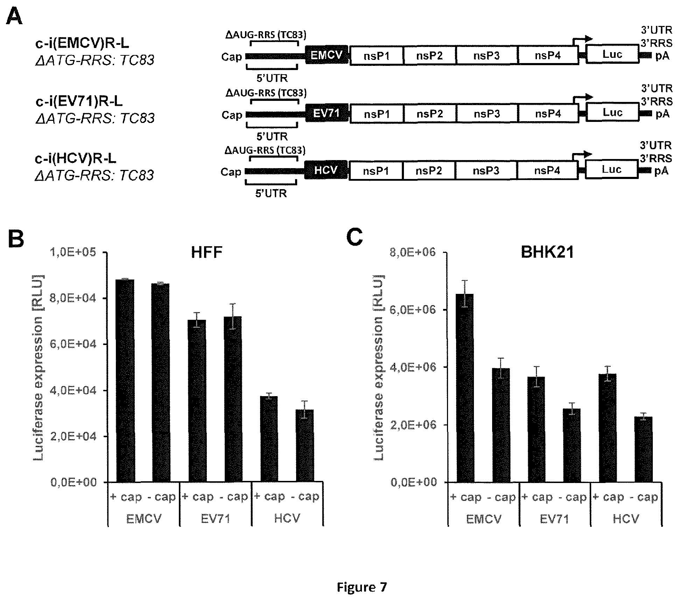

### Example 7 (see Figure 7): The enterovirus 71 IRES and hepatitis C virus IRES controlled replicase expression enables replication

**(A)** IRES-launched replicons expressing firefly luciferase (L) used in this example were constructed from the VEEV vaccine strain TC-83 (AATG-RRS-TC83). The replicon launched by the EMCV-IRES (c-i(EMCV)R-L) was modified and the EMCV IRES was exchanged with the HCV-IRES (c-i(HCV)R-L) or the EV71-IRES (c-i(EV71)R-L) as described in the material and methods. The vector RNA were *in vitro* transcribed with and without a cap analogon. **(B)** Human foreskin fibroblasts (HFF) or **(C)** BHK21 cells were lipofected in quadruplicates using MessengerMax. After 24h, firefly luciferase expression was measured.

Similar to EMCV-IRES launched vectors, EV71- and HCV-IRES launched vectors were replication competent resulting in robust reporter gene expression in both cells lines. Thus, the vector concept is compatible with different classes of IRESes.

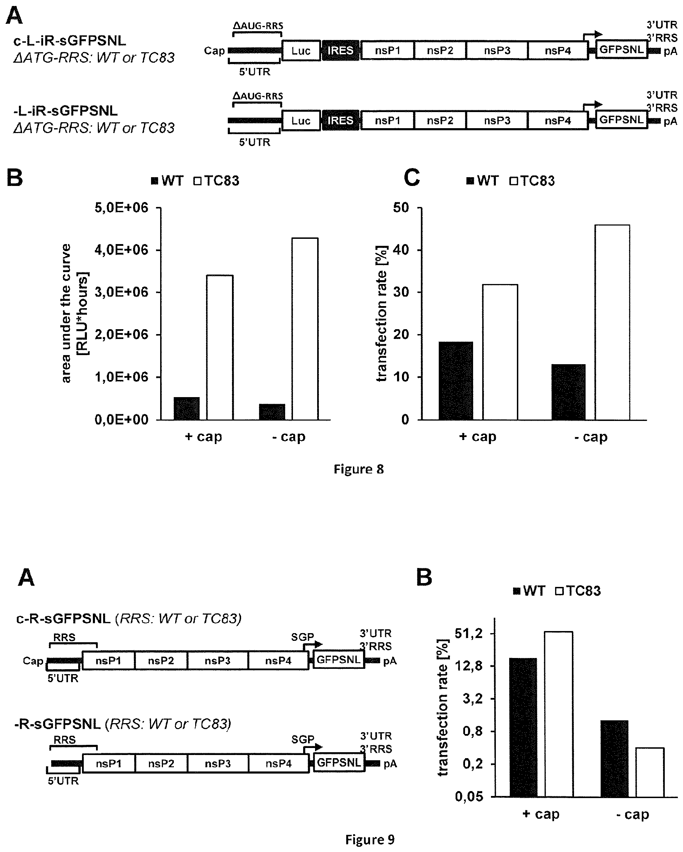

### Example 8 (see Figure 8): IRES-launched replicons derived from the VEEV vaccine strain TC-83 replicate and express more efficiently as WT VEEV.

**(A)** IRES-launched replicons used in this example were expressing both, GFP-SNL under the control of the viral subgenomic promoter, and firefly luciferase (L) from the 5'-ORF upstream of the IRES. The vectors were constructed from the wt VEEV or the VEEV vaccine strain TC-83 (ΔATG-RRS-TC83) as indicated. The vector RNA were *in vitro* transcribed with and without a cap analogon. Human foreskin fibroblasts (HFF) were electroporated. **(B)** Firefly luciferase was measured over a period of 72h after electroporation, and the total expression of luciferase calculated as area under the curve (AUC). **(C)** The transfection rates were assessed 24h after electroporation by quantifying the number of GFP+ cells.

We observed that the expression of TC83-based vectors outperformed vectors based on WT VEEV. The benefit provided by TC83 was more pronounced with respect to the luciferase expression, which is translated from the 5'cap of the genomic replicon-RNA copy. This is in accordance to literature which described that foremost the replication of the genomic RNA of TC83 rather than subgenomic transcription is accelerated by the 5'-nucleotide exchange (Kulasegaran-Shylini et al., 2009, Virology, vol. 387 pp. 211-221) compared to wt VEEV.

### Example 9 (see Figure 9): Uncapped conventional replicon of WT and TC-83 VEEV strains replicate poorly.

**(A)** Conventional replicons constructed from the wt VEEV or the VEEV vaccine strain TC-83 (RRS-TC83) used in this example were expressing GFP-SNL under the control of the viral subgenomic promoter as indicated. The vector RNA were *in vitro* transcribed with and without a cap analogon. Human foreskin fibroblasts (HFF) were electroporated. **(B)** The transfection rates of the cells were assessed 24h after electroporation by quantifying the number of GFP+ cells.

We observed that the expression of conventional replicons depends on capping. Only a minor fraction of the cells was expressing replicon encoded GFP when the RNA was left uncapped.

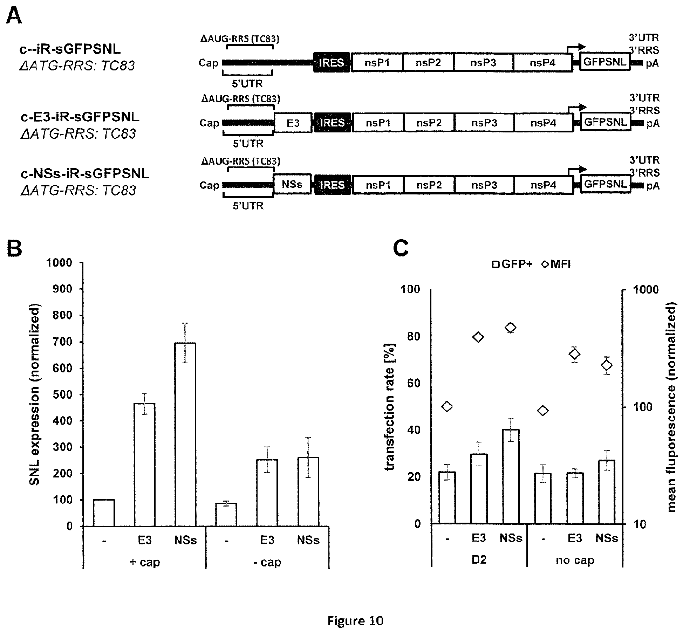

### Example 10 (see Figure 10): Interferon inhibitors expressed upstream of the IRES enhance the expression of IRES-launched replicons.

**(A)** IRES-launched replicons used in this example were expressing GFP-SNL under the control of the viral subgenomic promoter. In addition to that, the vectors expressed upstream of the IRES viral immune evasion genes, either Vaccinia virus E3 (c-E3-iR-sGFPSNL), or Toscana virus NSs (c-NSs-iR-sGFPSNL). Controls did not express any protein upstream of the IRES (c--iR-sGFPSNL). The vectors were constructed from the VEEV vaccine strain TC-83 (ΔATG-RRS-TC83) as indicated. The vector RNA were *in vitro* transcribed with and without a cap analogon. Human foreskin fibroblasts (HFF) were lipofected with these vectors. After 24h, cell culture supernatants and the cells were harvested. **(B)** Cell supernatants were used to measure the accumulation of SNL (NanoGlo assay) which reflects the total protein secretion within the 24h following transfection. **(C)** GFP expression of the cells was assessed by flow cytometry to determine the transfection rates and mean fluorescence intensity (MFI) of GFP+ cells.

We observed that the expression of IRON-launched replicons was enhanced by both interferon inhibitors. The benefit was more pronounced when the RNA was capped. This is in accordance to the fact that the ORF upstream of the IRES cannot be translated from uncapped RNA unless novel RNA copies are capped by the replicase subunit nsP1 during replication. Thus, E3 and NSs are expressed earlier when the RNA is capped *in vitro,* resulting in a more pronounced increase of expression compared to uncapped RNA.

## Claims

1. An RNA replicon comprising an internal ribosome entry site (IRES) and an open reading frame encoding a functional non-structural protein from a self-replicating virus, wherein the IRES controls expression of the functional non-structural protein, wherein the self-replicating virus is an alphavirus and wherein the functional non-structural protein is a functional alphaviral replicase.

2. The RNA replicon according to claim 1, wherein
(a) the IRES is insensitive to cellular stress, and/or interferons, preferably type I interferons, and/or
(b) the IRES is a cellular or viral IRES, preferably a viral IRES, wherein preferably the IRES is derived from viruses selected from the group consisting of picornaviruses, flaviviruses or dicistroviruses, wherein more preferably the IRES is derived from a picornavirus or a dicistrovirus, wherein still more preferably the IRES is derived from a dicistrovirus.

3. The RNA replicon according to claim 1 or 2, wherein the IRES is a type IV IRES and/or wherein expression controlled by the IRES is independent of IRES trans-acting factors, cellular translation initiation factors and/or phosphorylation of eukaryotic initiation factor 2 (eIF2).

4. The RNA replicon according to any one of claims 1 to 3, which does not comprise a 5' cap.

5. The RNA replicon according to any one of claims 1 to 4, which comprises a 5' replication recognition sequence.

6. The RNA replicon according to claim 5, wherein the 5' replication recognition sequence comprises a sequence homologous to an open reading frame of a non- structural protein or a fragment thereof from a self-replicating virus, wherein the sequence homologous to an open reading frame of a non-structural protein or a fragment thereof from a self-replicating virus is **characterized in that** it comprises the removal of at least one initiation codon compared to the native viral sequence,
wherein preferably the sequence homologous to an open reading frame of a non-structural protein or a fragment thereof from a self- replicating virus is **characterized in that** it comprises the removal of at least the native start codon of the open reading frame of a non-structural protein from a self-replicating virus, and/or of at least one initiation codon other than the native start codon of the open reading frame of a non-structural protein from a self-replicating virus,
wherein more preferably the sequence homologous to an open reading frame of a non-structural protein or a fragment thereof from a self-replicating virus is **characterized in that** it is free of initiation codons.

7. The RNA replicon according to claim 6, which comprises at least one nucleotide change compensating for nucleotide pairing disruptions within at least one stem loop introduced by the removal of at least one initiation codon.

8. The RNA replicon according to any one of claims 1 to 7, which does not comprise an open reading frame encoding a truncated non-structural protein from a self-replicating virus.

9. The RNA replicon according to any one of claims 5 to 8, wherein the open reading frame encoding a functional non-structural protein from a self-replicating virus does not overlap with the 5' replication recognition sequence.

10. The RNA replicon according to any one of claims 5 to 9, which comprises an open reading frame encoding a protein of interest downstream from the 5' replication recognition sequence and upstream from the IRES, wherein preferably the protein of interest can be expressed from the RNA replicon as a template.

11. The RNA replicon according to claim 10, wherein the protein of interest is expressed as a fusion protein with a protein or peptide encoded by the sequence homologous to an open reading frame of a non-structural protein or a fragment thereof from a self-replicating virus and expression of the fusion protein is initiated at an initiation codon of the sequence homologous to an open reading frame of a non- structural protein or a fragment thereof from a self-replicating virus, wherein preferably
(a) the initiation codon of the sequence homologous to an open reading frame of a non-structural protein or a fragment thereof from a self-replicating virus is the native start codon of the open reading frame of a non-structural protein from a self-replicating virus, and/or
(b) wherein expression of the protein of interest is initiated at an initiation codon of the open reading frame encoding a protein of interest.

12. The RNA replicon according to any one of claims 1 to 11, which comprises a subgenomic promotor controlling production of subgenomic RNA comprising an open reading frame encoding a protein of interest, wherein preferably
(a) the subgenomic RNA is a transcription product of an RNA-dependent RNA polymerase derived from the functional non-structural protein from a self-replicating virus,
(b) the protein of interest can be expressed from the subgenomic RNA as a template, and/or
(c) the RNA replicon comprises the open reading frame encoding a protein of interest controlled by the subgenomic promotor downstream from the open reading frame encoding a functional non- structural protein from a self-replicating virus, wherein the subgenomic promotor optionally overlaps with the open reading frame encoding a functional non-structural protein from a self-replicating virus.

13. The RNA replicon according to any one of claims 1 to 12, which comprises a 3' replication recognition sequence, wherein preferably the open reading frame encoding a functional non-structural protein from a self-replicating virus, the 5' and/or 3' replication recognition sequences and the subgenomic promotor are derived from a self-replicating virus, preferably the same self-replicating virus species.

14. The RNA replicon according to any one of claims 1 to 13, which can be replicated by an RNA-dependent RNA polymerase derived from the functional non-structural protein from a self-replicating virus.

15. The RNA replicon according to any one of claims 1 to 14, wherein the alphavirus is selected from the group consisting of Venezuelan equine encephalitis complex viruses, Eastern equine encephalitis complex viruses, Western equine encephalitis complex viruses, Semliki Forest virus complex viruses, Barmah Forest virus, Middelburg virus and Ndumu virus, wherein preferably the alphavirus is a Venezuelan equine encephalitis virus or Semliki Forest virus.

16. A non-viral particle comprising the RNA replicon according to any one of claims 1 to 15.

17. A DNA comprising a nucleic acid sequence encoding the RNA replicon according to any one of claims 1 to 15.

18. A composition comprising the RNA replicon according to any one of claims 1 to 15 and a pharmaceutically acceptable carrier.

19. The composition according to claim 18 for use in therapy.

20. A method for producing a protein of interest in a cell comprising the steps of:
(a) obtaining the RNA replicon according to any one of claims 1 to 15, which comprises an open reading frame encoding the protein of interest, and
(b) inoculating the RNA replicon into the cell.

21. The RNA replicon according to any one of claims 1 to 15 for use in a method for treatment, the treatment involving producing a protein of interest in a subject.

22. The RNA replicon for use of claim 21, wherein the protein of interest is an antigenic peptide or protein.

23. The RNA replicon for use of claim 22, wherein the treatment is eliciting an immune response in the subject.

## Patentansprüche

1. Ein RNA-Replikon, umfassend eine interne ribosomale Eintrittsstelle (IRES) und einen offenen Leserahmen, der für ein funktionelles nichtstrukturelles Protein eines selbstreplizierenden Virus kodiert, wobei die IRES die Expression des funktionellen nichtstrukturellen Proteins kontrolliert, wobei das selbstreplizierende Virus ein Alphavirus ist und wobei das funktionelle nichtstrukturelle Protein eine funktionelle alphavirale Replikase ist.

2. Das RNA-Replikon nach Anspruch 1, wobei
(a) die IRES gegenüber zellulärem Stress und/oder Interferonen, vorzugsweise Typ-I-Interferonen, unempfindlich ist, und/oder
(b) die IRES eine zelluläre oder virale IRES ist, vorzugsweise eine virale IRES, wobei die IRES vorzugsweise von Viren ausgewählt aus der Gruppe bestehend aus Picornaviren, Flaviviren oder Dicistroviren abgeleitet ist, wobei die IRES bevorzugterweise von einem Picornavirus oder einem Dicistrovirus abgeleitet ist und wobei die IHRES noch bevorzugterweise von einem Dicistrovirus abgeleitet ist.

3. Das RNA-Replikon nach Anspruch 1 oder 2, wobei die IRES eine Typ-IV-IRES ist und/oder wobei die durch die IRES kontrollierte Expression unabhängig ist von IRES-transagierenden Faktoren, zellulären Translations-Initiationsfaktoren und/oder der Phosphorylierung des eukaryotischen Initiationsfaktors 2 (eIF2).

4. Das RNA-Replikon nach einem der Ansprüche 1 bis 3, das keine 5'-Cap umfaßt.

5. Das RNA-Replikon nach einem der Ansprüche 1 bis 4, das eine 5'-Replikationserkennungssequenz umfasst.

6. Das RNA-Replikon nach Anspruch 5, wobei die 5'-Replikationserkennungssequenz eine Sequenz umfasst, die homolog zu einem offenen Leserahmen eines nichtstrukturellen Proteins oder eines Fragments davon eines selbstreplizierenden Virus ist, wobei die Sequenz, die homolog zu einem offenen Leserahmen eines nichtstrukturellen Proteins oder eines Fragments davon eines selbstreplizierenden Virus ist, **dadurch gekennzeichnet ist, dass** sie im Vergleich zur nativen viralen Sequenz die Entfernung mindestens eines Initiationscodons umfasst, wobei vorzugsweise die Sequenz, die homolog zu einem offenen Leserahmen eines nichtstrukturellen Proteins oder eines Fragments davon eines selbstreplizierenden Virus ist, **dadurch gekennzeichnet ist, dass** sie die Entfernung mindestens des nativen Startcodons des offenen Leserahmens eines nichtstrukturellen Proteins eines selbstreplizierenden Virus und/oder mindestens eines anderen Initiationscodons als des nativen Startcodons des offenen Leserahmens eines nichtstrukturellen Proteins eines selbstreplizierenden Virus umfasst,
wobei die Sequenz bevorzugterweise **dadurch gekennzeichnet ist, dass** sie frei von Initiationscodons ist.

7. Das RNA-Replikon nach Anspruch 6, das mindestens eine Nukleotidänderung umfasst, welche durch die Entfernung mindestens eines Initiationscodons verursachte Störungen der Nukleotidpaarung innerhalb mindestens einer Stem-Loop-Struktur kompensiert.

8. Das RNA-Replikon nach einem der Ansprüche 1 bis 7, das keinen offenen Leserahmen umfasst, der für ein verkürztes nichtstrukturelles Protein eines selbstreplizierenden Virus kodiert.

9. Das RNA-Replikon nach einem der Ansprüche 5 bis 8, wobei der offene Leserahmen, der für ein funktionelles nichtstrukturelles Protein eines selbstreplizierenden Virus kodiert, sich nicht mit der 5'-Replikationserkennungssequenz überlappt.

10. Das RNA-Replikon nach einem der Ansprüche 5 bis 9, das einen offenen Leserahmen umfasst, der für ein Protein von Interesse kodiert, stromabwärts der 5'-Replikationserkennungssequenz und stromaufwärts der IRES, wobei das Protein von Interesse vorzugsweise unter Verwendung des RNA-Replikons als Vorlage exprimiert werden kann.

11. Das RNA-Replikon nach Anspruch 10, wobei das Protein von Interesse als Fusionsprotein mit einem Protein oder Peptid exprimiert wird, das durch die Sequenz kodiert wird, die homolog zu einem offenen Leserahmen eines nichtstrukturellen Proteins oder eines Fragments davon eines selbstreplizierenden Virus ist, und die Expression des Fusionsproteins an einem Initiationscodon der Sequenz initiiert wird, die homolog zu einem offenen Leserahmen eines nichtstrukturellen Proteins oder eines Fragments davon eines selbstreplizierenden Virus ist, wobei vorzugsweise
(a) das Initiationscodon der Sequenz, die homolog zu einem offenen Leserahmen eines nichtstrukturellen Proteins oder eines Fragments davon eines selbstreplizierenden Virus ist, das native Startcodon des offenen Leserahmens eines nichtstrukturellen Proteins eines selbstreplizierenden Virus ist, und/oder
(b) die Expression des Proteins von Interesse an einem Initiationscodon des offenen Leserahmens initiiert wird, der für das Protein von Interesse kodiert.

12. Das RNA-Replikon nach einem der Ansprüche 1 bis 11, das einen subgenomischen Promotor umfasst, der die Produktion einer subgenomischen RNA kontrolliert, welche einen offenen Leserahmen umfasst, der für ein Protein von Interesse kodiert, wobei vorzugsweise
(a) die subgenomische RNA ein Transkriptionsprodukt einer RNA-abhängigen RNA-Polymerase ist, die von dem funktionellen nichtstrukturellen Protein eines selbstreplizierenden Virus abgeleitet ist,
(b) das Protein von Interesse unter Verwendung der subgenomischen RNA als Vorlage exprimiert werden kann, und/oder
(c) das RNA-Replikon den offenen Leserahmen umfasst, der für ein Protein von Interesse kodiert und durch den subgenomischen Promotor stromabwärts des offenen Leserahmens kontrolliert wird, der für ein funktionelles nichtstrukturelles Protein eines selbstreplizierenden Virus kodiert, wobei der subgenomische Promotor optional mit dem offenen Leserahmen überlappt, der für ein funktionelles nichtstrukturelles Protein eines selbstreplizierenden Virus kodiert.

13. Das RNA-Replikon nach einem der Ansprüche 1 bis 12, das eine 3'-Replikationserkennungssequenz umfasst, wobei vorzugsweise der offene Leserahmen, der für ein funktionelles nichtstrukturelles Protein eines selbstreplizierenden Virus kodiert, die 5'- und/oder 3'-Replikationserkennungssequenzen sowie der subgenomische Promotor von einem selbstreplizierenden Virus abgeleitet sind, vorzugsweise von derselben selbstreplizierenden Virusspezies.

14. Das RNA-Replikon nach einem der Ansprüche 1 bis 13, das durch eine RNA-abhängige RNA-Polymerase repliziert werden kann, die von dem funktionellen nichtstrukturellen Protein eines selbstreplizierenden Virus abgeleitet ist.

15. Das RNA-Replikon nach einem der Ansprüche 1 bis 14, wobei das Alphavirus ausgewählt ist aus der Gruppe bestehend aus Viren des Venezuelan-equine-encephalitis-Komplexes, Viren des Eastern-equine-encephalitis-Komplexes, Viren des Western-equine-encephalitis-Komplexes, Viren des Semliki-Forest-Virus-Komplexes, Barmah-Forest-Virus, Middelburg-Virus und Ndumu-Virus, wobei das Alphavirus vorzugsweise ein Venezuelan-equine-encephalitis-Virus oder Semliki-Forest-Virus ist.

16. Ein nichtvirales Partikel, umfassend das RNA-Replikon nach einem der Ansprüche 1 bis 15.

17. Eine DNA, umfassend eine Nukleinsäuresequenz, die für das RNA-Replikon nach einem der Ansprüche 1 bis 15 kodiert.

18. Eine Zusammensetzung, umfassend das RNA-Replikon nach einem der Ansprüche 1 bis 15 und einen pharmazeutisch akzeptablen Träger.

19. Die Zusammensetzung nach Anspruch 18 zur Verwendung in der Therapie.

20. Ein Verfahren zur Herstellung eines Proteins von Interesse in einer Zelle, umfassend die Schritte:
(a) Bereitstellen des RNA-Replikons nach einem der Ansprüche 1 bis 15, das einen offenen Leserahmen umfasst, der für das Protein von Interesse kodiert, und
(b) Einbringen des RNA-Replikons in die Zelle.

21. Das RNA-Replikon nach einem der Ansprüche 1 bis 15 zur Verwendung in einem Behandlungsverfahren, wobei die Behandlung die Herstellung eines Proteins von Interesse in einem Subjekt umfasst.

22. Das RNA-Replikon zur Verwendung nach Anspruch 21, wobei das Protein von Interesse ein antigenes Peptid oder Protein ist.

23. Das RNA-Replikon zur Verwendung nach Anspruch 22, wobei die Behandlung das Auslösen einer Immunantwort in dem Subjekt umfasst.

## Revendications

1. Réplicon d'ARN comprenant un site d'entrée interne du ribosome (IRES) et un cadre de lecture ouvert codant pour une protéine non structurale fonctionnelle provenant d'un virus autoréplicatif, dans lequel l'IRES contrôle l'expression de la protéine non structurale fonctionnelle, dans lequel le virus autoréplicatif est un alphavirus et dans lequel la protéine non structurale fonctionnelle est une réplicase alphavirale fonctionnelle.

2. Réplicon d'ARN selon la revendication 1, dans lequel
(a) l'IRES est insensible au stress cellulaire, et/ou aux interférons, de préférence aux interférons de type I, et/ou
(b) l'IRES est un IRES cellulaire ou viral, de préférence un IRES viral, dans lequel, de préférence, l'IRES est dérivé de virus choisis dans le groupe constitué de picornavirus, de flavivirus ou de dicistrovirus, dans lequel idéalement l'IRES est dérivé d'un picornavirus ou d'un dicistrovirus, dans lequel, idéalement encore, l'IRES est dérivé d'un dicistrovirus.

3. Réplicon d'ARN selon l'une des revendications 1 ou 2, dans lequel l'IRES est un IRES de type IV et/ou dans lequel l'expression contrôlée par l'IRES est indépendante des facteurs agissant en trans sur l'IRES, des facteurs cellulaires d'initiation de la traduction et/ou de la phosphorylation du facteur d'initiation eucaryote 2 (eIF2).

4. Réplicon d'ARN selon l'une quelconque des revendications 1 à 3, qui ne comprend pas de coiffe en 5'.

5. Réplicon d'ARN selon l'une quelconque des revendications 1 à 4, qui comprend une séquence de reconnaissance de réplication en 5'.

6. Réplicon d'ARN selon la revendication 5, dans lequel la séquence de reconnaissance de réplication en 5' comprend une séquence homologue à un cadre de lecture ouvert d'une protéine non structurale ou d'un fragment de celle-ci provenant d'un virus autoréplicatif, dans lequel la séquence homologue à un cadre de lecture ouvert d'une protéine non structurale ou d'un fragment de celle-ci provenant d'un virus autoréplicatif est **caractérisée en ce qu'**elle comprend la suppression d'au moins un codon d'initiation par rapport à la séquence virale native,
dans lequel, de préférence, la séquence homologue à un cadre de lecture ouvert d'une protéine non structurale ou d'un fragment de celle-ci provenant d'un virus autoréplicatif est **caractérisée en ce qu'**elle comprend la suppression d'au moins le codon de départ natif du cadre de lecture ouvert d'une protéine non structurale provenant d'un virus autoréplicatif, et/ou d'au moins un codon d'initiation autre que le codon de départ natif du cadre de lecture ouvert d'une protéine non structurale provenant d'un virus autoréplicatif,
dans lequel, idéalement, la séquence homologue à un cadre de lecture ouvert d'une protéine non structurale ou d'un fragment de celle-ci provenant d'un virus autoréplicatif est **caractérisée en ce qu'**elle est exempte de codons d'initiation.

7. Réplicon d'ARN selon la revendication 6, qui comprend au moins un changement nucléotidique compensant les perturbations d'appariement de nucléotides au sein d'au moins une tige-boucle introduites par la suppression d'au moins un codon d'initiation.

8. Réplicon d'ARN selon l'une quelconque des revendications 1 à 7, qui ne comprend pas de cadre de lecture ouvert codant pour une protéine non structurale tronquée provenant d'un virus autoréplicatif.

9. Réplicon d'ARN selon l'une quelconque des revendications 5 à 8, dans lequel le cadre de lecture ouvert codant pour une protéine non structurale fonctionnelle provenant d'un virus autoréplicatif ne chevauche pas la séquence de reconnaissance de réplication en 5'.

10. Réplicon d'ARN selon l'une quelconque des revendications 5 à 9, qui comprend un cadre de lecture ouvert codant pour une protéine d'intérêt en aval de la séquence de reconnaissance de réplication en 5' et en amont de l'IRES, dans lequel, de préférence, la protéine d'intérêt peut être exprimée à partir du réplicon d'ARN en tant que matrice.

11. Réplicon d'ARN selon la revendication 10, dans lequel la protéine d'intérêt est exprimée sous forme d'une protéine de fusion avec une protéine ou un peptide codé par la séquence homologue à un cadre de lecture ouvert d'une protéine non structurale ou d'un fragment de celle-ci provenant d'un virus autoréplicatif et l'expression de la protéine de fusion est initiée au niveau d'un codon d'initiation de la séquence homologue à un cadre de lecture ouvert d'une protéine non structurale ou d'un fragment de celle-ci provenant d'un virus autoréplicatif, dans lequel, de préférence
(a) le codon d'initiation de la séquence homologue à un cadre de lecture ouvert d'une protéine non structurale ou d'un fragment de celle-ci provenant d'un virus autoréplicatif est le codon de départ natif du cadre de lecture ouvert d'une protéine non structurale provenant d'un virus autoréplicatif, et/ou
(b) dans lequel l'expression de la protéine d'intérêt est initiée au niveau d'un codon d'initiation du cadre de lecture ouvert codant pour une protéine d'intérêt.

12. Réplicon d'ARN selon l'une quelconque des revendications 1 à 11, qui comprend un promoteur sous-génomique contrôlant la production d'ARN sous-génomique comprenant un cadre de lecture ouvert codant pour une protéine d'intérêt, dans lequel, de préférence,
(a) l'ARN sous-génomique est un produit de transcription d'une ARN polymérase ARN-dépendante dérivée de la protéine non structurale fonctionnelle provenant d'un virus autoréplicatif,
(b) la protéine d'intérêt peut être exprimée à partir de l'ARN sous-génomique en tant que matrice, et/ou
(c) le réplicon d'ARN comprend le cadre de lecture ouvert codant pour une protéine d'intérêt contrôlée par le promoteur sous-génomique en aval du cadre de lecture ouvert codant pour une protéine non structurale fonctionnelle provenant d'un virus autoréplicatif, dans lequel le promoteur sous-génomique chevauche éventuellement le cadre de lecture ouvert codant pour une protéine non structurale fonctionnelle provenant d'un virus autoréplicatif.

13. Réplicon d'ARN selon l'une quelconque des revendications 1 à 12, qui comprend une séquence de reconnaissance de réplication en 3', dans lequel de préférence le cadre de lecture ouvert codant pour une protéine non structurale fonctionnelle provenant d'un virus autoréplicatif, les séquences de reconnaissance de réplication en 5' et/ou en 3' et le promoteur sous-génomique sont dérivés d'un virus autoréplicatif, de préférence de la même espèce de virus autoréplicatif.

14. Réplicon d'ARN selon l'une quelconque des revendications 1 à 13, qui peut être répliqué par une ARN polymérase ARN-dépendante dérivée de la protéine non structurale fonctionnelle provenant d'un virus autoréplicatif.

15. Réplicon d'ARN selon l'une quelconque des revendications 1 à 14, dans lequel l'alphavirus est choisi dans le groupe constitué des virus du complexe de l'encéphalite équine vénézuélienne, des virus du complexe de l'encéphalite équine de l'Est, des virus du complexe de l'encéphalite équine de l'Ouest, des virus du complexe du virus de la forêt de Semliki, du virus de la forêt de Barmah, du virus de Middelburg et du virus Ndumu, dans lequel, de préférence, l'alphavirus est un virus de l'encéphalite équine vénézuélienne ou le virus de la forêt de Semliki.

16. Particule non virale comprenant le réplicon d'ARN selon l'une quelconque des revendications 1 à 15.

17. ADN comprenant une séquence d'acide nucléique codant pour le réplicon d'ARN selon l'une quelconque des revendications 1 à 15.

18. Composition comprenant le réplicon d'ARN selon l'une quelconque des revendications 1 à 15 et un support pharmaceutiquement acceptable.

19. Composition selon la revendication 18, destinée à être utilisée en thérapie.

20. Procédé permettant la production d'une protéine d'intérêt dans une cellule comprenant les étapes de :
(a) obtention du réplicon d'ARN selon l'une quelconque des revendications 1 à 15, qui comprend un cadre de lecture ouvert codant pour la protéine d'intérêt, et
(b) inoculation du réplicon d'ARN dans la cellule.

21. Réplicon d'ARN selon l'une quelconque des revendications 1 à 15 destiné à être utilisé dans un procédé de traitement, le traitement impliquant la production d'une protéine d'intérêt chez un sujet.

22. Réplicon d'ARN destiné à être utilisé selon la revendication 21, dans lequel la protéine d'intérêt est un peptide ou une protéine antigénique.

23. Réplicon d'ARN destiné à être utilisé selon la revendication 22, dans lequel le traitement consiste à provoquer une réponse immunitaire chez le sujet.
